# EUROPEAN PATENT APPLICATION

(11) **EP 4 067 483 A1**
(43) Date of publication of application: **05.10.2022**
(21) Application number: 21204332.7
(22) Date of filing: 22.10.2021
(51) Int. Cl.: C12N 9/02, C12Q 1/54, C12Q 1/32, G01N 33/66, C07K 14/195

(54) **MUTANT GLUCOSE DEHYDROGENASE HAVING IMPROVED THERMAL STABILITY, AND USE THEREOF**

(30) Priority: 29.03.2021 US 202117215476
(71) Applicant: The University of North Carolina at Chapel Hill, Chapel Hill, North Carolina 27516 (US); ARKRAY, Inc., Kyoto-shi, Kyoto 601-8045 (JP)
(72) Inventor: SODE, Koji, Chapel Hill, 27516, (US); SHIMAZAKI, Junko, Chapel Hill, 27516, (US); KOJIMA, Katsuhiro, Tokyo, 130-0012 (JP); TSUKADA, Masashi, Kyoto, 602-0008 (JP)
(74) Representative: Dehns

(57) **Abstract**

The present invention provides a mutant FAD-dependent glucose dehydrogenase comprising: a catalytic subunit; an electron transfer subunit; and a hitchhiker subunit; wherein each of the amino acid sequence of the catalytic subunit and the amino acid sequence of the electron transfer subunit comprises a cysteine residue introduced therein, the catalytic subunit and the electron transfer subunit being bound to each other through a disulfide bond between the cysteine residues to achieve improved thermal stability of the FAD-dependent glucose dehydrogenase.

## Description

### TECHNICAL FIELD

The present invention relates to biotechnology, and to electrochemical measurement of glucose utilizing a mutant glucose dehydrogenase.

### BACKGROUND ART

For improvement of the thermal stability of proteins, a number of methods have been developed. Known examples of artificial approaches therefor include glycosylation, chemical cross-linking and binding to polymers. These methods are widely applicable to arbitrary target proteins since the methods comprehensively protect the target proteins to achieve stabilization of the proteins. However, in the case of enzymes, these methods have a non-negligible effect on the activity of the enzyme, low-reproducibility of the results, leading to practical problems such the requirement for an additional step for purification and modification.

US 9074239, and US 8945359 disclose techniques for improvement of the thermal stability of FAD-dependent glucose dehydrogenase (FAD-GDH) by site-directed mutagenesis. However, those documents do not suggest stabilization of binding between subunits by mutagenesis in a catalytic subunit. US 8945359 describes that the specific activity of the mutant at 50°C is only 50% relative to the specific activity at 30°C, and that the mutant is deactivated at a high temperature condition of 60°C.

Biotechnol Lett 1774-8 (2015) reports that a mutant enzyme containing cysteine residues introduced at two specific sites of a catalytic subunit showed improved thermal resistance while maintaining activity and specificity.

### SUMMARY OF THE INVENTION

Glucose dehydrogenase is an enzyme often used for glucose sensors.

Glucose sensors for daily use in self-blood glucose measurement by diabetic patients are required to show accurate measured values in any situation or environment. Therefore, the enzyme, which is a key factor of the performance, is required to have high stability so as to avoid heat inactivation.

Based on information on the crystal structure of FAD-GDH, which is a heterotrimer comprising an electron transfer subunit, the present inventors introduced cysteine residues to specific sites on an intersubunit interface to form a disulfide bond on the interface, to thereby succeed in stabilization of the higher-order structure of the heterotrimer and remarkable improvement of the thermal resistance. Further, since the electron transfer activity of the mutant enzyme could be remarkably increased by introduction of cysteine residues to positions which are thought to be in the intramolecular electron transfer pathway of the enzyme, the present inventors found that the cysteine residues play an important role not only in cross-linking between the subunits, but also in assisting the electron transfer.

In one mode of the present invention, a mutant glucose dehydrogenase having improved thermal stability is provided without deterioration of the enzyme activity.

According to one aspect of the present invention, a mutant FAD-dependent glucose dehydrogenase comprising:
a catalytic subunit;
an electron transfer subunit; and
a hitchhiker subunit;
wherein each of the amino acid sequence of the catalytic subunit and the amino acid sequence of the electron transfer subunit contains a cysteine residue introduced therein, the catalytic subunit and the electron transfer subunit being bound to each other through a disulfide bond between the cysteine residues, is provided.

According to another aspect of the present invention, the mutant FAD-dependent glucose dehydrogenase in which the amino acid sequence of the electron transfer subunit comprises another cysteine residue introduced therein, and in which the amino acid sequence of the hitchhiker subunit comprises a cysteine residue introduced therein, the hitchhiker subunit and the electron transfer subunit being bound to each other through a disulfide bond between the cysteine residues, is provided.

According to another aspect of the present invention, the mutant FAD-dependent glucose dehydrogenase in which the amino acid sequence of the catalytic subunit and the amino acid sequence of the electron transfer subunit contains a cysteine residue introduced therein, the catalytic subunit and the electron transfer subunit being bound to each other through a disulfide bond between the cysteine residues, and simultaneously, the amino acid sequence of the electron transfer subunit comprises another cysteine residue introduced therein, and in which the amino acid sequence of the hitchhiker subunit comprises a cysteine residue introduced therein, the hitchhiker subunit and the electron transfer subunit being bound to each other through a disulfide bond between the cysteine residues, is provided.

According to another aspect of the present invention, an enzyme electrode comprising the mutant FAD-dependent glucose dehydrogenase is provided.

According to another aspect of the present invention, a biosensor comprising the enzyme electrode is provided.

### BRIEF DESCRIPTION OF THE DRAWINGS

Fig. 1 is a graph illustrating the enzyme activity of each GDH from *Burkholderia cepacia* (Bc) (wild type or mutants 1 to 3 with a full-length β subunit) at 25°C to 65°C.
Fig. 2 is a graph illustrating the enzyme activity of each GDH from *Burkholderia cepacia* (wild type or mutants 1 to 3 with a truncated β subunit) at 25°C to 65°C.
Fig. 3 is a graph illustrating the enzyme activity of each GDH from *Burkholderia cepacia* (mutants 4 and 5, with a full-length or truncated β subunit) at 25°C to 75°C.
Fig. 4 is a graph illustrating the enzyme activity of each GDH from *Burkholderia cepacia* (wild type or mutants 1 to 5 with a truncated β subunit) at 75°C.
Fig. 5 is a diagram illustrating the result of measurement of the glucose-dependent electric current using an enzyme electrode comprising each GDH (wild type or mutants 3 and 5 with a full-length or truncated β subunit) from *Burkholderia cepacia.*
Fig. 6 is a diagram illustrating the result of continuous measurement of the electric current response using an enzyme electrode comprising a mutant GDH (Mutant 5: QYY α₂₀₅Cβ₃₈₃C-γ₁₅₅Cβ₃₄₉C) or QYY GDH (control) from *Burkholderia cepacia.*
Fig. 7 is a diagram illustrating the result of measurement of the glucose-dependent electric current response on each day using an enzyme electrode comprising a mutant GDH (Mutant 5: QYY α₂₀₅Cβ₃₈₃C-γ₁₅₅Cβ₃₄₉C) or QYY GDH (control) from *Burkholderia cepacia.*
Fig. 8 is a graph illustrating the enzyme activity of GDH from *Ewingella americana* (Ea) (disulfide-formed mutant or wild type) at 65°C.
Fig. 9 shows the sequence alignment of the hitchhiker subunit between *Burkholderia cepacia* and *Ewingella americana.*
Fig. 10 shows the sequence alignment of the catalytic subunit between *Burkholderia cepacia* and *Ewingella americana.*
Fig. 11 shows the sequence alignment of the electron transfer subunit between *Burkholderia cepacia* and *Ewingella americana.*
Fig. 12 is a graph illustrating the enzyme activity of each of GDH from *Silvimonas terrae* (St) (wild type, αP204CβH382C and αP204CβH382C-γA168CβA347C) at 30°C to 70°C.
Fig. 13 is a graph illustrating the enzyme activity of GDH from *Silvimonas terrae* (St) (wild type, αP204CβH382C and αP204CβH382C-γA168CβA347C) at 60°C.
Fig. 14 is a graph illustrating the enzyme activity of each GDH from *Zymobacter palmae* (Zp) (wild type, αP200CβD389C and αP200CβD389C-γR182CβY355C) at 30°C to 70°C.
Fig. 15 is a graph illustrating the enzyme activity of GDH from *Zymobacter palmae* (Zp) (wild type, αP200CβD389C and αP200CβD389C-γR182CβY355C) at 60°C.
Fig. 16 is a graph illustrating the enzyme activity of each GDH from *Cobetia sp.* (Cb) L2A1 strain (wild type, and αP204CβS467C-γQ198CβY433C) at 30°C to 70°C.
Fig. 17 is a graph illustrating the enzyme activity of GDH from *Cobetia sp.* (Cb) L2A1 strain (wild type, and αP204CβS467C-γQ198CβY433C) at 60°C.
Fig. 18 shows the sequence alignment of the catalytic subunit between *Burkholderia cepacia, Ewingella americana, Silvimonas terrae, Zymobacter palmae* and *Cobetia sp..*
Fig. 19 shows the sequence alignment of the electron transfer subunit subunit between *Burkholderia cepacia, Ewingella americana, Silvimonas terrae, Zymobacterpalmae* and *Cobetia sp..*
Fig. 20 shows the sequence alignment of the hitchhiker between *Burkholderia cepacia, Ewingella americana, Silvimonas terrae, Zymobacter pαlmae,* and *Cobetia sp.*

### EMBODIMENTS FOR CARRYING OUT THE INVENTION

The glucose dehydrogenase according to one embodiment of the present invention is an FAD-dependent glucose dehydrogenase comprising:
a catalytic subunit (α subunit);
an electron transfer subunit (β subunit); and
a hitchhiker subunit (γ subunit);
wherein each of at least the catalytic subunit and the electron transfer subunit comprises a cysteine residue introduced therein, the cysteine residues forming a disulfide bond therebetween.

The glucose dehydrogenase is hereinafter also referred to as mutant glucose dehydrogenase (GDH) or mutant FAD-GDH. As referred to herein the enzyme is a mutant by virtue of the introduction of the cysteine residues and is dependent on flavin adenine dinucleotide (FAD) for catalytic activity. As referred to herein introduction of a cysteine residue refers to introduction by substitution of an existing residue in the sequence for a cysteine residue (e.g. at the residues disclosed hereinafter) or by introduction of a cysteine residue into the sequence (e.g. immediately before or immediately after, e.g. adjacent to, the residues disclosed hereinafter). Thus a mutant GDH of the invention may contain a cysteine residue at a residue disclosed hereinafter, or may contain a cysteine residue immediately before or immediately after, e.g. adjacent to, a residue disclosed hereinafter.

Thus, the mutant glucose dehydrogenase is a trimer composed of three subunits. The subunits may be linked to each other through a linker(s) and/or the like.

Known examples of an FAD-GDH which is a trimer composed of the three subunits include those of *Burkholderia cepacia, Burkholderia cenocepacia, Burkholderia thailandensis, Ralstonia pickettii, Ralstonia solanacearum, Burkholderia phytofirmans, Ralstonia sp., Moraxellaceae bacterium, Pseudomonas knackmussii, Yersinia nurmii, Edaphovirga cremea, Silvimonas terrae, Zymobacter palmae, Cobetia sp., Halomonas sp.*and *Ewingella americana* The mutant FAD-GDH according to one embodiment of the present invention can be obtained by introducing mutations thereto.

According to one embodiment of the present invention, in the mutant FAD-GDH, each of the amino acid sequence of the catalytic subunit and the amino acid sequence of the electron transfer subunit comprises a cysteine residue introduced therein, the catalytic subunit and the electron transfer subunit being bound to each other through a disulfide bond between the cysteine residues. The introduction of a cysteine residue includes introduction by amino acid substitution and introduction by insertion.

Each subunit is described below using the FAD-GDH of *Burkholderia cepacia* as an example. However, the mutant GDH is not limited to the following embodiments.

The *Burkholderia cepacia* is not limited. For example, the *Burkholderia cepacia* KS1 strain may be used. The *Burkholderia cepacia* KS 1 strain has been deposited with International Patent Organism Depositary, National Institute of Advanced Industrial Science and Technology (currently International Patent Organism Depositary (IPOD), National Institute of Technology and Evaluation (NITE)) as of September 25, 2000 under the accession number PERM BP-7306 by Koji Sode of c/o The University of North Carolina at Chapel Hill, 109 Church Street, Chapel Hill, North Carolina 27516, USA.

### <Catalytic Subunit>

The catalytic subunit, also called the α subunit, is a polypeptide having the catalytic activity of glucose dehydrogenation dependent on FAD (flavin adenine dinucleotide). The catalytic subunit preferably contains an Fe-S cluster

SEQ ID NO:3 is the amino acid sequence of the FAD-GDH catalytic subunit of the *Burkholderia cepacia* KS 1 strain. The following description is given with reference to this sequence.

The cysteine introduction site in the amino acid sequence of the catalytic subunit is preferably in a region forming the interface with the electron transfer subunit in the spatial structure of the FAD-GDH. For example, cysteine is introduced to a position in the region of amino acid positions 200 to 240 of SEQ ID NO:3. Thus, an amino acid residue in the region of amino acid positions 200 to 240 of SEQ ID NO:3 is substituted with cysteine. This region in the catalytic subunit is a region located on the interface with the electron transfer subunit in the spatial structure of GDH.

Specific examples of the amino acid in this region include the proline residue at position 205, glycine residue at position 208, asparagine residue at position 215, isoleucine residue at position 224, and glutamic acid residue at position 235 in the amino acid sequence of SEQ ID NO:3. Introduction of a cysteine residue includes substitution by cysteine and insertion of cysteine. For example, any of the above-described amino acid residues may be substituted with a cysteine residue. Alternatively, when a cysteine residue is inserted, the cysteine residue may be inserted at a position immediately before or immediately after these residues.

The above-described positions of amino acid substitution are positions in SEQ ID NO:3. However, the amino acid sequence of the catalytic subunit to which the mutation is to be introduced may have a length different from the length of SEQ ID NO:3. For example, in cases where the amino acid sequence of the catalytic subunit to which the mutation is to be introduced is shorter by three amino acids in the N-terminal side compared to SEQ ID NO:3, the proline residue at position 205 of SEQ ID NO:3 corresponds to the proline at position 202 of the amino acid sequence. Such a case is also regarded as substitution of the proline residue corresponding to the "proline residue at position 205", and included in a mode of the present invention. The same applies to other residues.

Among the above-described residues, the proline residue at position 205 is preferably substituted by a cysteine residue. This proline residue exists in the amino acid motif "ARNSRPYD" (SEQ ID NO: 23). In one embodiment, a cysteine residue is introduced to substitute the proline residue in this motif, and thereby the catalytic subunit of the mutant glucose dehydrogenase such as one having an amino acid sequence at least 60, 70, 80, 90, or 95% identical to the amino acid sequence of SEQ ID NO:3, 7, 13, 17 or 21 comprises the amino acid sequence of "ARNSRCYD" (SEQ ID NO: 24).

FAD-GDH is also known in other microorganisms. In the amino acid sequence of their catalytic subunit, for example, in an amino acid sequence having an identity of at least 60%, at least 80%, at least 90%, or at least 95% to SEQ ID NO:3, cysteine may be introduced into the region described above, or to the position of the amino acid residue corresponding to the specific amino acid residue described above.

More specifically, the amino acid sequence of the catalytic subunit to which the mutation is to be introduced may be aligned with the amino acid sequence of SEQ ID NO:3, and an amino acid residue in the region of amino acid positions 200 to 240 of SEQ ID NO:3, for example, the amino acid whose position corresponds to the position of the proline residue at position 205, glycine residue at position 208, asparagine residue at position 215, isoleucine residue at position 224, or glutamic acid residue at position 235, may be substituted with cysteine.

Any polypeptide having some degree of a sequence identity to SEQ ID NO:3 or the other sequence of the catalytic subunit described below can be used to prepare a catalytic subunit of the mutant glucose dehydrogenase as long as it maintains the function of the catalytic subunit, i.e. forming a complex with the electron transfer subunit and the hitchhiker subunit and exhibiting glucose dehydrogenase activity.

Sequence identity (also referred to herein as identity) refers to the percentage of identity between two amino acid (or nucleic acid) sequences and can be determined by visual examination or mathematical calculation. The amino acid sequence identity herein can be defined by aligning two amino acid sequences such that the number of matched amino acids is maximum while inserting a gap(s) when necessary, and calculating the ratio of the number of matched amino acids to the total number of amino acids in the aligned portion (the same applies hereinafter). Similar comments apply to determining sequence identity for nucleic acid sequences.

Examples of the amino acid sequence of the GDH α subunit having an identity of not less than 60% to SEQ ID NO:3 include the sequence of the α subunit of each of the followings: *Burkholderia cenocepacia* J2315 strain (GenBank Accession No. WP_006482972), *Burkholderia thailandensis* TXDOH strain (WP_009900298), *Ralstonia pickettii* 12D strain (WP_012761508), *Ralstonia solanacearum* IPO1609 strain (WP_003265143), *Burkholderia phytofirmans* PsJN strain (WP_012428610), *Ralstonia sp.*(WP_048931828), *Moraxellaceae bacterium* (WP_114899537), *Pseudomonas knackmussii* (WP_043248521), *Yersinia nurmii* (WP_049596850), *Edaphovirga cremea* (WP_114193339), *Silvimonas terrae* (WP_184102398), *Zymobacter palmae,* (WP_027705356), *Cobetia sp.* (WP_158773851), *Halomonas sp.* (WP_107334716) and *Ewingella americana* (WP_034788281).

As described above, the region and residues to which the cysteine residue is introduced in these catalytic subunits can be determined by aligning the sequence of each catalytic subunit with that of B. cepacia (SEQ ID NO: 3).

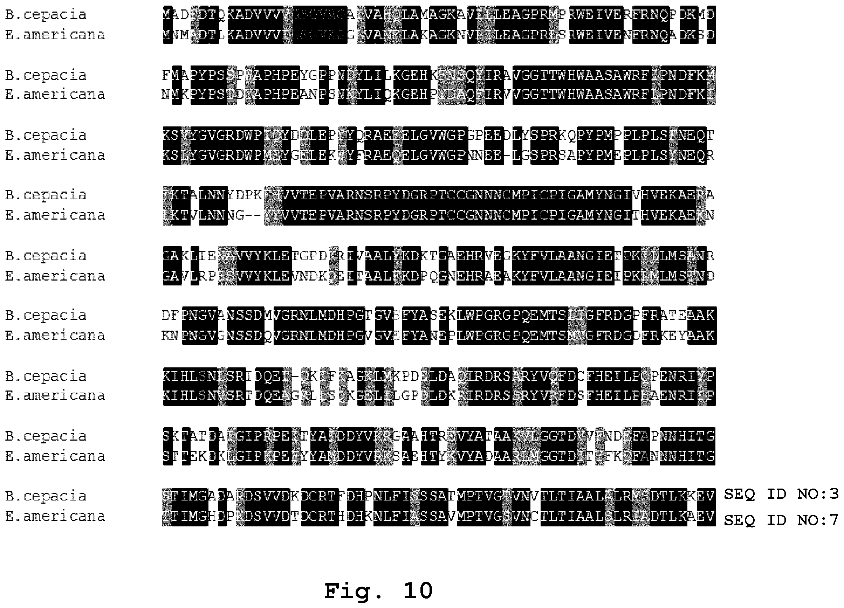

For example, in the case of the catalytic subunit of *E*. *americana* GDH (SEQ ID NO: 7), as shown in Fig. 10, the region 200-240 in SEQ ID NO: 3 is located in the region 197-237 in SEQ ID NO: 7, and the proline residue at position 205, glycine residue at position 208, asparagine residue at position 215, isoleucine residue at position 224, and glutamic acid residue at position 235 respectively correspond to the proline residue at position 202, glycine residue at position 205, asparagine residue at position 212, isoleucine residue at position 221, and glutamic acid residue at position 232 in SEQ ID NO: 7.

Another embodiment of the catalytic subunit includes a catalytic subunit comprising an amino acid sequence having at least 60%, 80%, 90% or 95% identity to SEQ ID NO: 7 wherein cysteine is introduced at the position corresponding to the proline residue at position 202, glycine residue at position 205, asparagine residue at position 212, isoleucine residue at position 221, or glutamic acid residue at position 232 in SEQ ID NO: 7.

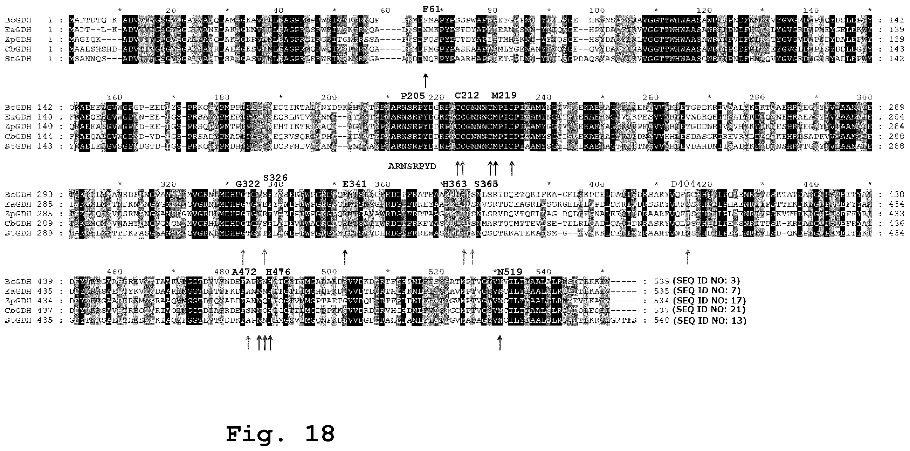

For example, in the case of the catalytic subunit of *Silvimonas terrae* GDH (SEQ ID NO: 13), as shown in Fig. 18, the proline residue at position 205 in SEQ ID NO: 3 corresponds to the proline residue at position 204 in SEQ ID NO: 13.

Another embodiment of the catalytic subunit includes a catalytic subunit comprising an amino acid sequence having at least 60%, 80%, 90% or 95% identity to SEQ ID NO: 13 wherein cysteine is introduced at the position corresponding to the proline residue at position 204 in SEQ ID NO: 13.

For example, in the case of the catalytic subunit of *Zymobacter palmae* GDH (SEQ ID NO: 17), as shown in Fig. 18, the proline residue at position 205 in SEQ ID NO: 3 corresponds to the proline residue at position 200 in SEQ ID NO: 17.

Another embodiment of the catalytic subunit includes a catalytic subunit comprising an amino acid sequence having at least 60%, 80%, 90% or 95% identity to SEQ ID NO: 17 wherein cysteine is introduced at the position corresponding to the proline residue at position 200 in SEQ ID NO: 17.

For example, in the case of the catalytic subunit of *Cobetia sp.* GDH (SEQ ID NO: 21), the proline residue at position 205 corresponds to the proline residue at position 204 in SEQ ID NO: 21.

Another embodiment of the catalytic subunit includes a catalytic subunit comprising an amino acid sequence having at least 60%, 80%, 90% or 95% identity to SEQ ID NO: 21 wherein cysteine is introduced at the position corresponding to the proline residue at position 204 in SEQ ID NO: 21.

The amino acid sequence of the catalytic subunit (α subunit) may contain not only the mutation that introduces cysteine, but also an additional mutation. Known examples of mutants comprising mutations in the catalytic subunit of FAD-dependent GDH derived from *Burkholderia cepacia* include a mutant in which the amino acid residues at positions 472 and 475 are substituted (WO 2005/103248), a mutant in which the amino acid residues at positions 326, 365, and 472 are substituted (QYY mutant JP 2012-090563 A), a mutant in which position 365 and position(s) 326, 472, 475, 529, and/or the like are substituted (WO 2006/137283). The catalytic subunit of the mutant GDH according to one embodiment of the present invention may have these mutations.

### <Electron Transfer Subunit>

The electron transfer subunit is also called β subunit, and contains at least one heme-binding domain. The subunit functions to transfer electrons generated by reaction using glucose as a substrate, to an electrode.

The electron transfer subunit comprises a cysteine residue introduced therein, and this cysteine residue forms a disulfide bond with the cysteine residue introduced to the catalytic subunit.

The electron transfer subunit is not limited as long as it functions to transfer electrons generated by glucose dehydrogenation to an electrode. Electron transfer subunits derived from various organisms, including known electron transfer subunits, may be used. The electron transfer subunit may be derived from the same microorganism as the microorganism from which the catalytic subunit is derived. Examples of the electron transfer subunit include the electron transfer subunit of GDH of *Burkholderia cepacia.* Examples of the GDH electron transfer subunit of the *B. cepacia* KS1 strain include a polypeptide comprising the amino acid sequence of SEQ ID NO:4. SEQ ID NO:4 is the amino acid sequence of the GDH β subunit of the *Burkholderia cepacia* KS 1 strain. The following description is given with reference to this sequence.

The cysteine introduction site in the amino acid sequence of the electron transfer subunit is preferably in a region forming the interface with the catalytic subunit. For example, cysteine is introduced to a position in the region of amino acid positions 330 to 400 of the amino acid sequence of SEQ ID NO:4. Thus, an amino acid residue in the region of amino acid positions 330 to 400 of SEQ ID NO:4 is substituted with cysteine. This region in the electron transfer subunit is a region located on the interface with the catalytic subunit in the spatial structure of GDH.

More specific examples of the amino acid in this region include the threonine residue at position 336, glycine residue at position 385, aspartic acid residue at position 383, and tyrosine residue at position 391 in the amino acid sequence of SEQ ID NO:4. Any of these may be substituted with a cysteine residue. When a cysteine residue is inserted, the cysteine residue may be inserted at a position immediately before or immediately after these residues.

Among the above-described residues, the aspartic acid residue at position 383 is preferably substituted by a cysteine residue. This amino acid residue exists at a position three amino acids before the amino acid motif "MP(A/G)F" (SEQ ID NO: 25). In one embodiment, a cysteine residue is introduced to substitute the amino acid residue three amino acid before this motif, and thereby the electron transfer subunit of the mutant glucose dehydrogenase such as one having an amino acid sequence at least 60, 70, 80, 90, or 95% identical to the amino acid sequence of SEQ ID NO: 4, 8, 14, 18 or 22 comprises the amino acid sequence of

### "CXXMP(A/G)F" (SEQ ID NO: 26).

Examples of a preferred combination of the cysteine introduction site in the region in the catalytic subunit (the region of amino acid positions 200 to 240 of SEQ ID NO:3) and the cysteine introduction site in the region in the electron transfer subunit (the region of amino acid positions 330 to 400 of SEQ ID NO:4) include the combination of the proline at position 205 of the catalytic subunit and the aspartic acid at position 383 of the electron transfer subunit, and the combination of the asparagine at position 215 of the catalytic subunit and the threonine at position 336 of electron transfer subunit. These combinations of residues are combinations of residues having a distance of about 5 Å from each other in the spatial structure of FAD-GDH, and capable of more efficient formation of a disulfide bond.

The mutant glucose dehydrogenase may also be a mutant glucose dehydrogenase in which the amino acid sequence of the electron transfer subunit contains another introduced cysteine residue, which cysteine residue forms a disulfide bond with a cysteine residue introduced in the amino acid sequence of the hitchhiker subunit, to bind the hitchhiker subunit and the electron transfer subunit to each other.

The another cysteine residue is preferably introduced into a region of the electron transfer subunit which is on the interface with the hitchhiker subunit. Examples of such a region include the region of amino acid positions 341 to 350 in the amino acid sequence of SEQ ID NO:4. Thus, an amino acid residue in the region of amino acid positions 341 to 350 of SEQ ID NO:4 may be substituted with cysteine. This region in the electron transfer subunit is a region located on the interface with the hitchhiker subunit in the spatial structure of GDH.

More specific examples of the amino acid in this region include the threonine residue at position 345, proline residue at position 346, and tyrosine residue at position 349 in the amino acid sequence of the region of SEQ ID NO:4. Introduction of a cysteine residue includes substitution by cysteine and insertion of cysteine. For example, any of the above-described amino acid residues may be substituted with a cysteine residue. Alternatively, when a cysteine residue is inserted, the cysteine residue may be inserted at a position immediately before or immediately after these residues.

Among the above-described residues, the tyrosine residue at position 349 is preferably substituted by a cysteine residue. This amino acid residue exists at a position one amino acid before the amino acid motif "YPS(L/M)" (SEQ ID NO: 27). In one embodiment, a cysteine residue is introduced to substitute the amino acid residue one amino acid before this motif, and thereby the electron transfer subunit of the mutant glucose dehydrogenase such as one having an amino acid sequence at least 60, 70, 80, 90, or 95% identical to the amino acid sequence of SEQ ID NO: 4, 8, 14, 18 or 22 comprises the amino acid sequence of "CYPS(L/M)" (SEQ ID NO: 28).

The above-described positions of amino acid substitution mutation are positions in SEQ ID NO:4. However, the amino acid sequence of the electron transfer subunit to which the mutation is to be introduced may have a length different from the length of SEQ ID NO:4. For example, in cases where the amino acid sequence of the electron transfer subunit to which the mutation is to be introduced is shorter by five amino acids in the N-terminal side compared to SEQ ID NO:4, the threonine residue at position 336 of SEQ ID NO:4 corresponds to the threonine at position 331 of the amino acid sequence. Such a case is also regarded as substitution of the threonine residue corresponding to the "threonine residue at position 336", and included in a mode of the present invention. The same applies to other residues.

The electron transfer subunit may also comprise another mutation in addition to the mutation that introduces cysteine.

The electron transfer subunit contains three heme-binding domains. The electron transfer subunit may be a mutant (truncated) electron transfer subunit in which the first heme-binding domain and the second heme-binding domain, that is, the first and second domains as counted from the N-terminal side, respectively, are deleted. Such a mutant electron transfer subunit is disclosed in US 2019-0010215.

Thus, in the electron transfer subunit having three heme-binding domains (CXXCH (SEQ ID NO: 31) motifs), the first and second heme-binding domains as counted from the N-terminal side, or a region(s) including these, may be deleted. The following description uses the electron transfer subunit of *Burkholderia cepacia* as a representative example. In SEQ ID NO:4, there are the first heme-binding domain (amino acid positions 43 to 47), second heme-binding domain (amino acid positions 191 to 195), and third heme-binding domain (amino acid positions 334 to 338). Among these, the first heme-binding domain and second heme-binding domain may be deleted, or the region including the first and second heme-binding domains (amino acid positions 43 to 195) may be deleted.

Examples of a mutant electron transfer subunit in which the region including the first and second heme-binding domains is deleted include a mutant electron transfer subunit composed of amino acid positions 314 to 425 or 330 to 425 of SEQ ID NO:4.

GDH is known also in other microorganisms. In the amino acid sequence of their electron transfer subunit, for example, in an amino acid sequence having an identity of at least 60%, at least 80%, at least 90%, or at least 95% to SEQ ID NO:4 or to the truncated sequence composed of amino acid positions 314 to 425 or 330 to 425 of SEQ ID NO:4, cysteine may be introduced to a position corresponding to such an amino acid residue as the threonine residue at position 336, glycine residue at position 385, aspartic acid residue at position 383, or tyrosine residue at position 391, and/or the threonine residue at position 345, proline residue at position 346, or tyrosine residue at position 349.

Specifically, the amino acid sequence of the electron transfer subunit to which the mutation is to be introduced may be aligned with the amino acid sequence of SEQ ID NO:4, and the amino acid whose position corresponds to the position of the threonine residue at position 336, glycine residue at position 385, aspartic acid residue at position 383, or tyrosine residue at position 391; or the threonine residue at position 345, proline residue at position 346, or tyrosine residue at position 349; of SEQ ID NO:4 may be substituted with cysteine.

Any polypeptide having some degree of a sequence identity to SEQ ID NO:4 or the other sequence of the electron transfer subunit described below can be used to prepare an electron transfer subunit of the mutant glucose dehydrogenase as long as it maintains the function of the electron transfer subunit, i.e. forming a complex with the catalytic subunit and the hitchhiker subunit and receiving and transferring an electron. The amino acid sequence of the electron transfer subunit preferably maintains three heme-binding domains or in the case of a truncated form, the third three heme-binding domain.

Examples of the amino acid sequence of the GDH electron transfer subunit having an identity of at least 60% to SEQ ID NO:4 include the sequence of the electron transfer subunit of each of the following: *Burkholderia cenocepacia* J2315 strain (WP_006482958), *Burkholderia thailandensis* TXDOH strain (WP_009900297), *Ralstonia pickettii* 12D strain (WP_012761509), *Ralstonia solanacearum* IPO1609 strain (WP_049281214), *Burkholderia phytofirmans* PsJN strain (WP_012428609), *Ralstonia sp.*(WP_048931829) *Moraxellaceae bacterium* (WP_114899536), *Pseudomonas knackmussii* (WP_043248520), *Yersinia nurmii* (WP_049596851), *Edaphovirga cremea* (WP_114193338), *Silvimonas terrae* (WP_184102397), *Zymobacter palmae,* (WP_211245188), *Cobetia sp.* (WP_158773850), *Halomonas sp.* (WP_107336529) and *Ewingella americana* (WP_034788317).

As described above, the region and residues to which the cysteine residue is introduced in these electron transfer subunits can be determined by aligning the sequence of each electron transfer subunit with that of *B. cepacia* (SEQ ID NO: 4).

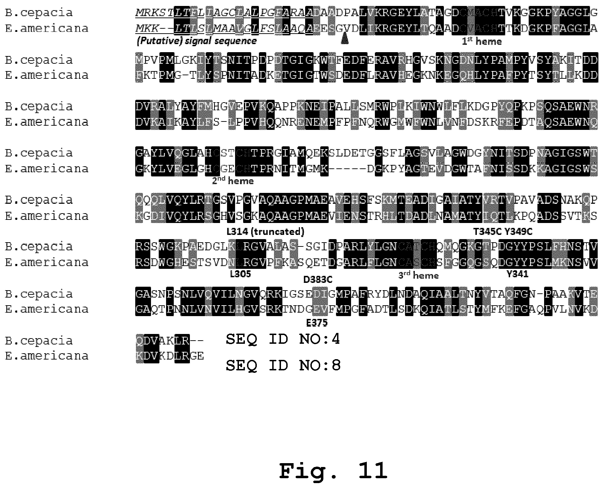

For example, in the case of the electron transfer subunit of *E*. *americana* (SEQ ID NO: 8), as shown in Fig. 11, the region 330 to 400 in SEQ ID NO: 4 is located in the region 322-392 in SEQ ID NO: 8, and the threonine residue at position 336, aspartic acid residue at position 383, glycine residue at position 385 and tyrosine residue at position 391 respectively correspond to serine residue at position 328, glutamic acid residue at position 375, phenylalanine residue at position 377, and aspartic acid residue at position 383 in SEQ ID NO: 8.

Furthermore, in the case of the electron transfer subunit of *E. americana* (SEQ ID NO: 8), as shown in Fig. 11, the region 341 to 350 in SEQ ID NO: 4 is located in the region 333-342 in SEQ ID NO: 8, and the threonine residue at position 345, proline residue at position 346, and tyrosine residue at position 349 respectively correspond to serine residue at position 337, glutamine residue at position 338, and tyrosine residue at position 341 in SEQ ID NO: 8.

In SEQ ID NO: 8, there are the first heme-binding domain (amino acid positions 41 to 45), second heme-binding domain (amino acid positions 187 to 191), and third heme-binding domain (amino acid positions 326 to 330). Among these, the first heme-binding domain and second heme-binding domain may be deleted, or the region including the first and second heme-binding domains (amino acid positions 41 to 191) may be deleted. Examples of a mutant electron transfer subunit in which the region including the first and second heme-binding domains is deleted include a mutant electron transfer subunit composed of amino acid positions 306 to 420 or 322 to 420 of SEQ ID NO: 8.

Another embodiment of the electron transfer subunit includes an electron transfer subunit comprising an amino acid sequence having at least 60%, 80%, 90% or 95% identity to SEQ ID NO: 8 or amino acids 306 to 420 of SEQ ID NO: 8 wherein a first cysteine is introduced at the position corresponding to the serine residue at position 328, glutamic acid residue at position 375, phenylalanine residue at position 377, or aspartic acid residue at position 383, and/or a second cysteine is introduced at the position corresponding to the serine residue at position 337, glutamine residue at position 338, or tyrosine residue at position 341 in SEQ ID NO: 8.

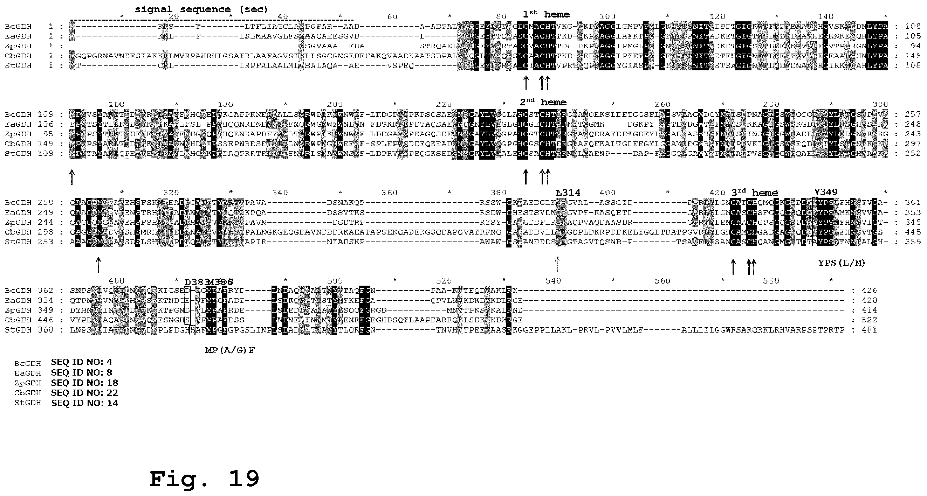

Furthermore, in the case of the electron transfer subunit of *Silvimonas terrae* (SEQ ID NO: 14), as shown in Fig. 19, the aspartic acid residue at position 383 in SEQ ID NO: 4 corresponds to the histidine residue at position 382 in SEQ ID NO: 14. The tyrosine residue at position 349 in SEQ ID NO: 4 corresponds to the alanine residue at position 347 in SEQ ID NO: 14.

In SEQ ID NO: 14, there are the first heme-binding domain (amino acid positions 43 to 47), second heme-binding domain (amino acid positions 191 to 195), and third heme-binding domain (amino acid positions 332 to 336). Among these, the first heme-binding domain and second heme-binding domain may be deleted, or the region including the first and second heme-binding domains (amino acid positions 43 to 195) may be deleted. Examples of a mutant electron transfer subunit in which the region including the first and second heme-binding domains is deleted include a mutant electron transfer subunit composed of amino acid positions 332 to 481 of SEQ ID NO: 14.

Another embodiment of the electron transfer subunit includes an electron transfer subunit comprising an amino acid sequence having at least 60%, 80%, 90% or 95% identity to SEQ ID NO: 14 or amino acids 332 to 481 of SEQ ID NO: 14 wherein a first cysteine is introduced at the position corresponding to the histidine residue at position 382 in SEQ ID NO: 14 and/or a second cysteine is introduced at the position corresponding to the alainine residue at position 347 in SEQ ID NO: 14.

Furthermore, in the case of the electron transfer subunit of *Zymobacter palmae* (SEQ ID NO: 18), as shown in Fig. 19, the aspartic acid residue at position 383 in SEQ ID NO: 4 corresponds to the aspartic acid residue at position 389 in SEQ ID NO: 18. The tyrosine residue at position 349 in SEQ ID NO: 4 corresponds to the tyrosine residue at position 355 in SEQ ID NO: 18.

In SEQ ID NO: 18, there are the first heme-binding domain (amino acid positions 49 to 53), second heme-binding domain (amino acid positions 196 to 200), and third heme-binding domain (amino acid positions 340 to 344). Among these, the first heme-binding domain and second heme-binding domain may be deleted, or the region including the first and second heme-binding domains (amino acid positions 49 to 200) may be deleted. Examples of a mutant electron transfer subunit in which the region including the first and second heme-binding domains is deleted include a mutant electron transfer subunit composed of amino acid positions 340 to 433 of SEQ ID NO: 18.

Another embodiment of the electron transfer subunit includes an electron transfer subunit comprising an amino acid sequence having at least 60%, 80%, 90% or 95% identity to SEQ ID NO: 18 or amino acids 340 to 433 of SEQ ID NO: 18 wherein a first cysteine is introduced at the position corresponding to the aspartic acid residue at position 389 in SEQ ID NO: 18 and/or a second cysteine is introduced at the position corresponding to the tyrosine residue at position 355 in SEQ ID NO: 18.

Furthermore, in the case of the electron transfer subunit of *Cobetia sp.* (SEQ ID NO: 22), as shown in Fig. 19, the aspartic acid residue at position 383 in SEQ ID NO: 4 corresponds to the serine residue at position 467 in SEQ ID NO: 22. The tyrosine residue at position 349 in SEQ ID NO: 4 corresponds to the tyrosine residue at position 433 in SEQ ID NO: 22.

In SEQ ID NO: 22, there are the first heme-binding domain (amino acid positions 84 to 88), second heme-binding domain (amino acid positions 231 to 235), and third heme-binding domain (amino acid positions 418 to 422). Among these, the first heme-binding domain and second heme-binding domain may be deleted, or the region including the first and second heme-binding domains (amino acid positions 84 to 235) may be deleted. Examples of a mutant electron transfer subunit in which the region including the first and second heme-binding domains is deleted include a mutant electron transfer subunit composed of amino acid positions 418 to 522 of SEQ ID NO: 22.

Another embodiment of the electron transfer subunit includes an electron transfer subunit comprising an amino acid sequence having at least 60%, 80%, 90% or 95% identity to SEQ ID NO: 22 or amino acids 418 to 522 of SEQ ID NO: 22 wherein a first cysteine is introduced at the position corresponding to the serine residue at position 467 in SEQ ID NO: 22 and/or a second cysteine is introduced at the position corresponding to the tyrosine residue at position 433 in SEQ ID NO: 22.

### <Hitchhiker Subunit>

The glucose dehydrogenase also includes a hitchhiker subunit. The hitchhiker subunit is also called γ subunit, and has the function of forming a complex with the α subunit and β subunit, and being secreted into periplasm. The hitchhiker subunit is not limited as long as it has the function. γ subunits derived from various organisms, including known γ subunits, may be used. The hitchhiker subunit may be derived from the same microorganism as the microorganism from which the catalytic subunit or electron transfer subunit is derived. Examples of the hitchhiker subunit include the hitchhiker subunit of GDH of *Burkholderia*

### cepacia.

SEQ ID NO:2 is the amino acid sequence of the GDH hitchhiker subunit of the *Burkholderia cepacia* KS1 strain. The following description is given with reference to this sequence.

As described above, the hitchhiker subunit may contain a cysteine residue introduced therein for formation of a disulfide bond with the electron transfer subunit.

The cysteine introduction site in the amino acid sequence of the hitchhiker subunit is preferably in a region forming the interface with the electron transfer subunit. For example, cysteine is introduced to a position in the region of amino acid positions 140 to 160 in the amino acid sequence of SEQ ID NO:2. Thus, an amino acid residue in the region of amino acid positions 140 to 160 of SEQ ID NO:2 is substituted with cysteine. This region in the hitchhiker subunit is a region located on the interface with the electron transfer subunit in the spatial structure of GDH.

Specific examples of the amino acid in this region include the threonine residue at position 145, asparagine residue at position 154, and lysine residue at position 155 in the amino acid sequence of SEQ ID NO:2. Introduction of a cysteine residue includes substitution by cysteine and insertion of cysteine. For example, any of the above-described amino acid residues may be substituted with a cysteine residue. Alternatively, when a cysteine residue is inserted, the cysteine residue may be inserted at a position immediately before or immediately after these residues.

Among the above-described residues, the lysine residue at position 155 is preferably substituted by a cysteine residue. This amino acid residue exists at a position one amino acid before the amino acid motif "PXXWXXXP" (SEQ ID NO: 29). In one embodiment, a cysteine residue is introduced to substitute the amino acid residue one amino acid before this motif, and thereby the hitchhiker subunit of the mutant glucose dehydrogenase such as one having an amino acid sequence at least 60, 70, 80, 90, or 95% identical to the amino acid sequence of SEQ ID NO:2, 6, 12, 16 or 20 comprises the amino acid sequence of "CPXXWXXXP" (SEQ ID NO: 30).

The above-described positions of amino acid substitution mutation are positions in SEQ ID NO:2. However, the amino acid sequence of the hitchhiker subunit to which the mutation is to be introduced may have a length different from the length of SEQ ID NO:2. For example, in cases where the amino acid sequence of the hitchhiker subunit to which the mutation is to be introduced is longer by two amino acids in the N-terminal side compared to SEQ ID NO:2, the threonine residue at position 145 of SEQ ID NO:2 corresponds to the threonine at position 147 of the amino acid sequence. Such a case is also regarded as substitution of the threonine residue corresponding to the "threonine residue at position 145", and included in a mode of the present invention. The same applies to other residues.

Examples of a preferred combination of the cysteine introduction site for disulfide formation with the hitchhiker subunit in the electron transfer subunit (the region of amino acid positions 341 to 350 of SEQ ID NO:4) and the cysteine introduction site in the hitchhiker subunit (the region of amino acid positions 140 to 160 of SEQ ID NO:2) include the combination of the threonine at position 345 of the electron transfer subunit and the asparagine at position 154 of the hitchhiker subunit, and the combination of the tyrosine at position 349 of the electron transfer subunit and the lysine at position 155 of the hitchhiker subunit. These combinations of residues are combinations of residues having a distance of about 5 Å from each other in the spatial structure of FAD-GDH, and capable of more efficient formation of a disulfide bond.

The hitchhiker subunit may also comprise another mutation in addition to the mutation that introduces cysteine.

GDH is known also in other microorganisms. In the amino acid sequence of their hitchhiker subunit, for example, in an amino acid sequence having an identity of at least 60%, at least 80%, at least 90%, or at least 95% to SEQ ID NO:2, cysteine may be introduced into the region described above, or to the position of the amino acid residue corresponding to the specific amino acid residue described above.

Specifically, the amino acid sequence of the hitchhiker subunit to which the mutation is to be introduced may be aligned with the amino acid sequence of SEQ ID NO:2, and the amino acid whose position corresponds to the position of the threonine residue at position 145, asparagine residue at position 154, or lysine residue at position 155 of SEQ ID NO:2 may be substituted with cysteine.

Any polypeptide having some degree of a sequence identity to SEQ ID NO:2 or the other sequence of the hitchhiker subunit described below can be used to prepare a hitchhiker subunit of the mutant glucose dehydrogenase as long as it maintains the function of the hitchhiker subunit, i.e. forming a complex with the catalytic subunit and the electron transfer subunit and being secreted into periplasm.

Examples of the amino acid sequence of the GDH hitchhiker subunit having an identity of at least 60% to SEQ ID NO:2 include the sequence of the γ subunit of each of the following: *Burkholderia cenocepacia* J2315 strain (WP_006482974), *Burkholderia thailandensis* TXDOH strain (WP_009900299), *Ralstonia pickettii* 12D strain (WP_012761507), *Ralstonia solanacearum* IPO1609 strain (WP_003265142), *Burkholderia phytofirmans* PsJN strain (WP_012428611), *Ralstonia sp.* (WP_048931990), *Moraxellaceae bacterium* (WP_114899538), *Pseudomonas knackmussii* (WP_043248523), *Yersinia nurmii* (WP_049596849), *Edaphovirga cremea* (WP_114193340), *Silvimonas terrae* (WP-184102399), *Zymobacter palmae,* (BBG29837), *Cobetia sp.* (WP_158773852), *Halomonas sp.* (WP_107334715) and *Ewingella americana* (WP_034788279).

As described above, the region and residues to which the cysteine residue is introduced in these hitchhiker subunits can be determined by aligning the sequence of each hitchhiker subunit with that of B. cepacia (SEQ ID NO: 2).

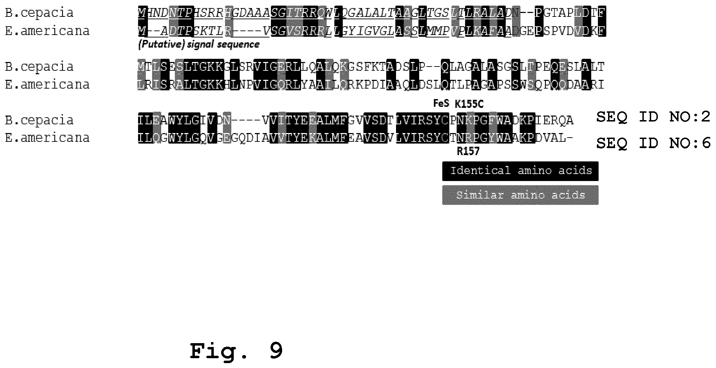

For example, in the case of the hitchhiker subunit of *E*. *americana* (SEQ ID NO: 6), as shown in Fig. 9, the region 140 to 160 in SEQ ID NO: 2 is located in the region 142-162 in SEQ ID NO: 6, and the threonine residue at position 145, asparagine residue at position 154, and lysine residue at position 155 respectively correspond to valine residue at position 147, asparagine residue at position 156, and arginine residue at position 157 in SEQ ID NO: 6.

Another embodiment of the hitchhiker subunit includes a hitchhiker subunit comprising an amino acid sequence having at least 60%, 80%, 90% or 95% identity to SEQ ID NO: 6 wherein cysteine is introduced at the position corresponding to the valine residue at position 147, asparagine residue at position 156, or arginine residue at position 157 in SEQ ID NO: 6.

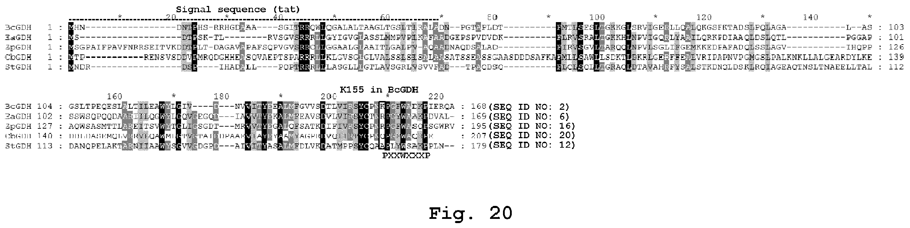

For example, in the case of the hitchhiker subunit of *Silvimonas terrae* (SEQ ID NO: 12), as shown in Fig. 20, the lysine residue at position 155 corresponds to the alanine residue at position 168 in SEQ ID NO: 12.

Another embodiment of the hitchhiker subunit includes a hitchhiker subunit comprising an amino acid sequence having at least 60%, 80%, 90% or 95% identity to SEQ ID NO: 12 wherein cysteine is introduced at the position corresponding to the alanine residue at position 168 in SEQ ID NO: 12.

For example, in the case of the hitchhiker subunit of *Zymobacter palmae* (SEQ ID NO: 16), as shown in Fig. 20, the lysine residue at position 155 corresponds to the arginine residue at position 182 in SEQ ID NO: 16.

Another embodiment of the hitchhiker subunit includes a hitchhiker subunit comprising an amino acid sequence having at least 60%, 80%, 90% or 95% identity to SEQ ID NO: 16 or amino acids 19 to 195 of SEQ ID NO: 16 wherein cysteine is introduced at the position corresponding to the arginine residue at position 182 in SEQ ID NO: 16.

For example, in the case of the hitchhiker subunit of *Cobetia sp.* (SEQ ID NO: 20), as shown in Fig. 20, the lysine residue at position 155 corresponds to the glutamine residue at position 198 in SEQ ID NO: 20.

Another embodiment of the hitchhiker subunit includes a hitchhiker subunit comprising an amino acid sequence having at least 60%, 80%, 90% or 95% identity to SEQ ID NO: 20 or amino acids 14 to 207 of SEQ ID NO: 20 wherein cysteine is introduced at the position corresponding to the glutamine residue at position 198 in SEQ ID NO: 20.

In one embodiment of a mutant FAD-dependent glucose dehydrogenase,
the catalytic subunit comprises an amino acid sequence having at least 60%, 70%, 80%, 90% or 95% identity to SEQ ID NO: 3 wherein cysteine is introduced at the position corresponding to the proline residue at position 205 in SEQ ID NO: 3,
the electron transfer subunit comprises an amino acid sequence having at least 60%, 70%, 80%, 90% or 95% identity to SEQ ID NO: 4 or amino acids 330 to 425 of SEQ ID NO: 4 wherein a first cysteine is introduced at the position corresponding to the aspartic acid residue at position 383 in SEQ ID NO: 4 and a second cysteine is introduced at the position corresponding to the threonine residue at position 345 in SEQ ID NO: 4, and
the hitchhiker subunit comprises an amino acid sequence having at least 60%, 70%, 80%, 90% or 95% identity to SEQ ID NO: 2 wherein cysteine is introduced at the position corresponding to the asparagine residue at position 154 in SEQ ID NO: 2.

In one embodiment of a mutant FAD-dependent glucose dehydrogenase,
the catalytic subunit comprises an amino acid sequence having at least 60%, 70%, 80%, 90% or 95% identity to SEQ ID NO: 3 wherein cysteine is introduced at the position corresponding to the proline residue at position 205 in SEQ ID NO: 3,
the electron transfer subunit comprises an amino acid sequence having at least 60%, 70%, 80%, 90% or 95% identity to SEQ ID NO: 4 or amino acids 330 to 425 of SEQ ID NO: 4 wherein a first cysteine is introduced at the position corresponding to the aspartic acid residue at position 383 in SEQ ID NO: 4 and a second cysteine is introduced at the position corresponding to the tyrosine residue at position 349 in SEQ ID NO: 4, and
the hitchhiker subunit comprises an amino acid sequence having at least 60%, 70%, 80%, 90% or 95% identity to SEQ ID NO: 2 wherein cysteine is introduced at the position corresponding to the lysine residue at position 155 in SEQ ID NO: 2.

In one embodiment of a mutant FAD-dependent glucose dehydrogenase,
the catalytic subunit comprises an amino acid sequence having at least 60%, 70%, 80%, 90% or 95% identity to SEQ ID NO: 7 wherein cysteine is introduced at the position corresponding to the proline residue at position 202 in SEQ ID NO: 7,
the electron transfer subunit comprises an amino acid sequence having at least 60%, 70%, 80%, 90% or 95% identity to SEQ ID NO: 8 or amino acids 326 to 420 of SEQ ID NO: 8 wherein a first cysteine is introduced at the position corresponding to the glutamic acid residue at position 375 in SEQ ID NO: 8 and a second cysteine is introduced at the position corresponding to the tyrosine residue at position 341 in SEQ ID NO: 8, and
the hitchhiker subunit comprises an amino acid sequence having at least 60%, 70%, 80%, 90% or 95% identity to SEQ ID NO: 6 wherein cysteine is introduced at the position corresponding to the arginine residue at position 157 in SEQ ID NO: 6.

In one embodiment of a mutant FAD-dependent glucose dehydrogenase,
the catalytic subunit comprises an amino acid sequence having at least 60%, 70%, 80%, 90% or 95% identity to SEQ ID NO: 13 wherein cysteine is introduced at the position corresponding to the proline residue at position 204 in SEQ ID NO: 13,
the electron transfer subunit comprises an amino acid sequence having at least 60%, 70%, 80%, 90% or 95% identity to SEQ ID NO: 14 or amino acids 332 to 481 of SEQ ID NO: 14 wherein a first cysteine is introduced at the position corresponding to the histidine residue at position 382 in SEQ ID NO: 14 and a second cysteine is introduced at the position corresponding to the alanine residue at position 347 in SEQ ID NO: 14, and
the hitchhiker subunit comprises an amino acid sequence having at least 60%, 70%, 80%, 90% or 95% identity to SEQ ID NO: 12 wherein cysteine is introduced at the position corresponding to the alanine residue at position 168 in SEQ ID NO: 12.

In one embodiment of a mutant FAD-dependent glucose dehydrogenase,
the catalytic subunit comprises an amino acid sequence having at least 60%, 70%, 80%, 90% or 95% identity to SEQ ID NO: 17 wherein cysteine is introduced at the position corresponding to the proline residue at position 200 in SEQ ID NO: 17,
the electron transfer subunit comprises an amino acid sequence having at least 60%, 70%, 80%, 90% or 95% identity to SEQ ID NO: 18 or amino acids 340 to 465 of SEQ ID NO: 18 wherein a first cysteine is introduced at the position corresponding to the aspartic acid residue at position 389 in SEQ ID NO: 18 and a second cysteine is introduced at the position corresponding to the tyrosine residue at position 355 in SEQ ID NO: 18, and
the hitchhiker subunit comprises an amino acid sequence having at least 60%, 70%, 80%, 90% or 95% identity to SEQ ID NO: 16 or amino acids 19 to 195 of SEQ ID NO: 16 wherein cysteine is introduced at the position corresponding to the arginine residue at position 182 in SEQ ID NO: 16.

In one embodiment of a mutant FAD-dependent glucose dehydrogenase,
the catalytic subunit comprises an amino acid sequence having at least 60%, 70%, 80%, 90% or 95% identity to SEQ ID NO: 21 wherein cysteine is introduced at the position corresponding to the proline residue at position 204 in SEQ ID NO: 21,
the electron transfer subunit comprises an amino acid sequence having at least 60%, 70%, 80%, 90% or 95% identity to SEQ ID NO: 22 or amino acids 418 to 522 of SEQ ID NO: 22 wherein a first cysteine is introduced at the position corresponding to the serine residue at position 467 in SEQ ID NO: 22 and a second cysteine is introduced at the position corresponding to the tyrosine residue at position 433 in SEQ ID NO: 22, and
the hitchhiker subunit comprises an amino acid sequence having at least 60%, 70%, 80%, 90% or 95% identity to SEQ ID NO: 20 or amino acids 14 to 207 of SEQ ID NO: 20 wherein cysteine is introduced at the position corresponding to the glutamine residue at position 198 in SEQ ID NO: 20.

The mutant GDH according to one embodiment of the present invention can be obtained by genetic engineering. More specifically, the mutant GDH can be obtained by introducing the mutations of interest by site-directed mutagenesis or the like to the gene encoding GDH, and expressing the resulting mutant GDH gene in a suitable host cell or cell-free translation system. The present invention thus also extends to a method of producing a mutant FAD-dependent glucose dehydrogenase as defined herein comprising introducing a mutation(s) as described herein into a nucleic acid molecule expressing an FAD-dependent glucose dehydrogenase and expressing said mutant FAD-dependent glucose dehydrogenase (e.g. in a translation system as described above). Optionally said dehydrogenase may be isolated or purified.

The gene encoding the GDH catalytic (α) subunit is not limited as long as it has a nucleotide sequence corresponding to the amino acid sequence of the GDH α subunit. Specific examples of the gene include a DNA composed of nucleotide positions 764 to 2380 of SEQ ID NO:1. The α-subunit gene may be a DNA having the nucleotide sequence composed of nucleotide positions 764 to 2380 of the nucleotide sequence of SEQ ID NO:1, or a DNA which hybridizes, under stringent conditions, with a probe that can be prepared from this sequence, which DNA encodes a protein having the GDH activity (or a sequence with an identity of at least 80%, preferably at least 90 or 95%, to nucleotide positions 764 to 2380 of SEQ ID NO: 1).

Specific examples of the gene encoding the GDH electron transfer (β) subunit include a DNA containing the nucleotide sequence composed of nucleotide positions 2386 to 3660 of SEQ ID NO: 1. The β-subunit gene may be a DNA having the nucleotide sequence composed of nucleotide positions 2386 to 3660 of SEQ ID NO: 1, or a DNA which hybridizes, under stringent conditions, with a probe that can be prepared from this sequence, which DNA encodes a protein capable of functioning as the β subunit (or a sequence with an identity of at least 80%, preferably at least 90 or 95%, to nucleotide positions 2386 to 3660 of SEQ ID NO: 1).

Specific examples of the gene encoding the GDH hitchhiker subunit (γ) subunit include a DNA containing the nucleotide sequence composed of nucleotide positions 258 to 761 of SEQ ID NO:1. The γ-subunit gene may be a DNA having the nucleotide sequence composed of nucleotide positions 258 to 761 of SEQ ID NO:1, or a DNA which hybridizes, under stringent conditions, with a probe that can be prepared from this sequence, which DNA encodes a protein capable of functioning as the γ subunit (or a sequence with an identity of at least 80%, preferably at least 90 or 95%, to nucleotide positions 258 to 761 of SEQ ID NO: 1).

Examples of the stringent conditions described above include conditions that allow hybridization of DNAs having an identity of preferably at least 80%, more preferably at least 90%, especially preferably at least 95%, with each other and does not allow hybridization of DNAs having an identity of less than 80%, less than 90% or less than 95%. Specific examples of the stringent conditions include those in which washing is carried out with 0.1 × SSC and 0.1% SDS at 60°C, 65°C, or 68°C.

The α-subunit gene, β-subunit gene, and γ-subunit gene can be obtained by, for example, PCR using chromosomal DNA of the *Burkholderia cepacia* KS1 strain as a template. The PCR primers therefor can be prepared by chemical synthesis based on the above-described nucleotide sequences. Alternatively, the genes can be obtained from chromosomal DNA of the *Burkholderia cepacia* KS 1 strain by hybridization using, as probes, oligonucleotides prepared based on the above-described sequences. Strains other than the KS1 strain, such as the *Burkholderia cenocepacia* J2315 strain, *Burkholderia thailandensis* TXDOH strain, *Ralstonia pickettii* 12D strain, *Ralstonia solanacearum* IPO1609 strain, and *Burkholderia phytofirmans* PsJN strain, may also be used.

Each of the GDH α-subunit, β-subunit, and γ-subunit having a desired mutation(s) can be obtained by introducing a nucleotide mutation(s) corresponding to the desired amino acid mutation(s) (such as the substitution with a cysteine residue) to a DNA encoding the GDH subunit by site-directed mutagenesis, and expressing the resulting mutant DNA using an appropriate expression system. Further, by allowing expression from the DNA encoding the mutant GDH α subunit, and also from the DNA encoding the mutant β subunit and the DNA encoding the mutant γ subunit, a mutant GDH can be obtained. The introduction of the mutation(s) to the DNA encoding each subunit may be carried out using a polycistronic DNA fragment encoding the γ subunit, α subunit, and β subunit in this order.

The polycistronic DNA fragment encoding the γ subunit, α subunit, and β subunit in this order can be obtained by, for example, PCR using chromosomal DNA of the *Burkholderia cepacia* KS1 strain as a template, and oligonucleotides having the nucleotide sequences of SEQ ID NOs:9 and 10 as primers.

The vector used for obtaining the genes of the GDH subunits, introducing the mutations, expressing the genes, and the like include vectors capable of functioning in bacteria belonging to the genus *Escherichia*, such as pTrc99A, pBR322, pUC18, pUC118, pUC19, pUC119, pACYC184, and pBBR122. Examples of the promoter to be used for the gene expression include *lac, trp, tac, trc, PL, tet,* and *PhoA.* By introducing the γ subunit gene, α subunit gene, and β subunit gene to an appropriate site in an expression vector containing a promoter, insertion of the genes into the vector and linking of the promoter can be carried out in the same single process. Examples of such an expression vector include pTrc99A, pBluescript, and pKK223-3.

The γ subunit gene, α subunit gene, and β subunit gene may be incorporated in the chromosomal DNA of the host microorganism in a form which allows the expression. Examples of the method of transformation of the microorganism with the recombinant vector include the competent cell method by calcium treatment, the protoplast method, and the electroporation method.

Examples of the host microorganism include, but are not limited to, bacteria belonging to the genus *Bacillus* such as *Bacillus subtilis*; yeasts such as *Saccharomyces cerevisiae*; and filamentous fungi such as *Aspergillus niger.* Any host microorganism suitable for production of a foreign protein may be used.

The transformant such as a microorganism transformed with the mutant GDH genes may be cultured by an appropriate culture method depending on the type of the transformant. Examples of the conditions for the culture include culturing at 30 to 37°C for 12 to 24 hours.

The mutant GDH may be used also after purification. The mutant GDH may contain an additional sequence such as a tag sequence for purification.

The mutant GDH according to one embodiment of the present invention, or the microorganism which expresses the mutant GDH, may be used as a component of an enzyme electrode of a glucose sensor. Specific examples of the glucose sensor include glucose sensors that use, as a working electrode, an enzyme electrode formed by immobilization of the mutant GDH on a surface of an electrode such as a gold electrode, platinum electrode, or carbon electrode. The sensor means a measurement system for electrochemically measuring the concentration of a test substance of interest, and usually contains three electrodes, which are a working electrode (enzyme electrode), counter electrode (platinum or the like), and reference electrode (Ag/AgCl or the like). The sensor may also be a two-electrode system constituted by a working electrode and a counter electrode, such as those used in conventional, simple blood glucose level systems. The sensor preferably further contains: a constant-temperature cell in which a buffer and a test sample are to be placed; a power source for applying a voltage to the working electrode; an ammeter; a recorder; and/or the like. The sensor may be either a batch-type sensor or flow-type sensor. In particular, the flow-type sensor may be a sensor capable of continuous measurement of the blood glucose level. More specifically, the sensor may be a sensor having a two-electrode system or three-electrode system on which the enzyme of the present invention is immobilized, which electrode system is inserted into a blood sample or dialysis sample that is continuously supplied, or into blood or interstitial fluid, to perform the measurement. The structure of such an enzyme sensor is well known in the art, and described in, for example, Biosensors - Fundamental and Applications - Anthony P. F. Turner, Isao Karube and Geroge S. Wilson, Oxford University Press 1987.

The sensor according to one mode of the present invention may be a direct electron transfer-type sensor containing no electron-transfer mediator.

The method of immobilizing the enzyme (mutant GDH) on the electrode is not limited. Examples of the method include a method in which enzyme molecules are chemically immobilized on the electrode using a cross-linking agent or the like, a method in which enzyme molecules are indirectly immobilized on the electrode using a binder or the like, and a method by physical adsorption of enzyme molecules to the electrode.

The method in which the enzyme is chemically immobilized on the electrode using a cross-linking agent or the like may be a method in which the enzyme is directly immobilized on the electrode. Alternatively, a method such as the one disclosed in US 10563242B2 may be used. More specifically, this method is a method in which a monolayer (SAM)-forming molecule is immobilized on the electrode, and a molecular recognition element containing the enzyme is immobilized through the SAM-forming molecule.

The monolayer-forming molecule is a compound capable of binding to the electrode, and of allowing binding of the enzyme molecule thereto. By binding a plurality of molecules of the compound in the same direction on the electrode surface, a monolayer film can be formed. By using the monolayer-forming molecule, the distance between the electrode and the enzyme molecule can be controlled.

The monolayer-forming molecule preferably contains: a first functional group having affinity to the electrode, a spacer region, and a second functional group reactive with a functional group contained in the enzyme molecule. More preferably, the monolayer-forming molecule has a structure in which the first functional group having affinity to the electrode is bound to a first end of the spacer region, and in which the second functional group reactive with a functional group contained in the enzyme molecule is bound to a second end of the spacer region. Here, examples of the first functional group having affinity to the electrode include a thiol group and dithiol group in cases where the electrode is a metal; and pyrene and porphyrin in cases where the electrode is carbon. On the other hand, examples of the second functional group reactive with a functional group contained in the enzyme molecule include a succinimide group in cases of reaction with an amino group (including a terminal amino group and side-chain amino group) contained in the enzyme molecule; and an oxazoline group in cases of reaction with a carboxyl group (including a terminal carboxyl group and side-chain carboxyl group) contained in the enzyme molecule. Examples of a monolayer-forming molecule containing a thiol group or dithiol group, and succinimide group, include DSH as described later in Examples.

The measurement of the glucose concentration using the glucose sensor of the present invention can be carried out as follows. A buffer is placed in a constant-temperature cell of the sensor, and the temperature of the cell is kept constant. As a working electrode, an enzyme electrode on which the mutant GDH is immobilized is used. As a counter electrode, for example, a platinum electrode is used. As a reference electrode, for example, an Ag/AgCl electrode is used. A constant voltage is applied to the working electrode. After the electric current becomes constant, a sample containing glucose is placed in the constant-temperature cell, and the increase in electric current is measured. According to a calibration curve prepared using glucose solutions having standard concentrations, the glucose concentration in the sample can be calculated.

The mutant GDH according to one embodiment of the present invention can also be used as a component of a glucose assay kit. The glucose assay kit may contain a coloring or luminescence reagent, a dilution buffer, a standard substance, manufacturer's instructions, and/or the like in addition to the mutant GDH.

For example, a glucose sensor and a glucose assay kit using a wild-type GDH of *Burkholderia cepacia* are described in US 2004/0023330 A1. The mutant GDH can be applied in the similar manner.

### EXAMPLES

The present invention is described below more specifically by showing non-limiting Examples.

### Example 1

### Expression of Mutant GDH

As a GDH comprising a cytochrome C-containing subunit, a CyGDH derived from *B*. *cepacia* was used. The CyGDH derived from *B. cepacia* is an oligomer enzyme composed of the γ, α, and β subunits. These three subunits are encoded in the gene having the nucleotide sequence of SEQ ID NO:1. Based on this sequence, pTrc99Aγα(QYY)β, which contains a polynucleotide modified such that the encoded α subunit has a QYY mutation, and pTrc99Aγα(QYY)β330-His, which contains a polynucleotide modified such that the α subunit has the QYY mutation, and such that the encoded β subunit is a deletion-type β subunit composed of amino acid positions 330 to 425, were used (US 2019-0010215).

With these vectors, site-directed mutagenesis was carried out such that the following amino acid substitutions were introduced to the subunits. Mutant 1 is a mutant in which position 154 of the γ subunit is substituted with a cysteine; position 345 of the β subunit is substituted with a cysteine; and a disulfide bond is formed between these cysteines. Mutant 2 is a mutant in which position 155 of the γ subunit is substituted with a cysteine; position 349 of the β subunit is substituted with a cysteine; and a disulfide bond is formed between these cysteines. Mutant 3 is a mutant in which position 205 of the α subunit is substituted with a cysteine; position 383 of the β subunit is substituted with a cysteine; and a disulfide bond is formed between these cysteines. Mutant 4 is a mutant in which position 205 of the α subunit is substituted with a cysteine; position 383 of the β subunit is substituted with a cysteine; a disulfide bond (α-β disulfide bond) is formed between these cysteines; position 345 of the β subunit is substituted with a cysteine; position 154 of the γ subunit is substituted with a cysteine; and a disulfide bond (β-γ disulfide bond) is formed between these cysteines. Mutant 5 is a mutant in which position 205 of the α subunit is substituted with a cysteine; position 383 of the β subunit is substituted with a cysteine; a disulfide bond (α-β disulfide bond) is formed between these cysteines; position 349 of the β subunit is substituted with a cysteine; position 155 of the γ subunit is substituted with a cysteine; and a disulfide bond (β-γ disulfide bond) is formed between these cysteines.

| | α | | β | | γ |
|---|---|---|---|---|---|
| Mutant 1 | | | 345 | - | 154 |
| Mutant 2 | | | 349 | - | 155 |
| Mutant 3 | 205 | - | 383 | | |
| Mutant 4 | 205 | - | 383 & 345 | - | 154 |
| Mutant 5 | 205 | - | 383 & 349 | - | 155 |

*E. coli* was transformed with each vector obtained, and the resulting transformant was cultured to allow expression of each mutant GDH.

The culture and purification of glucose dehydrogenase were carried out by the method described in US 2019-0010215.

### Measurement of Enzyme Activity

Each mutant GDH was heat-treated at each temperature for 15 minutes, and then the enzyme activity was measured according to the method described in US 2019-0010215 with a system using PMS/DCIP.

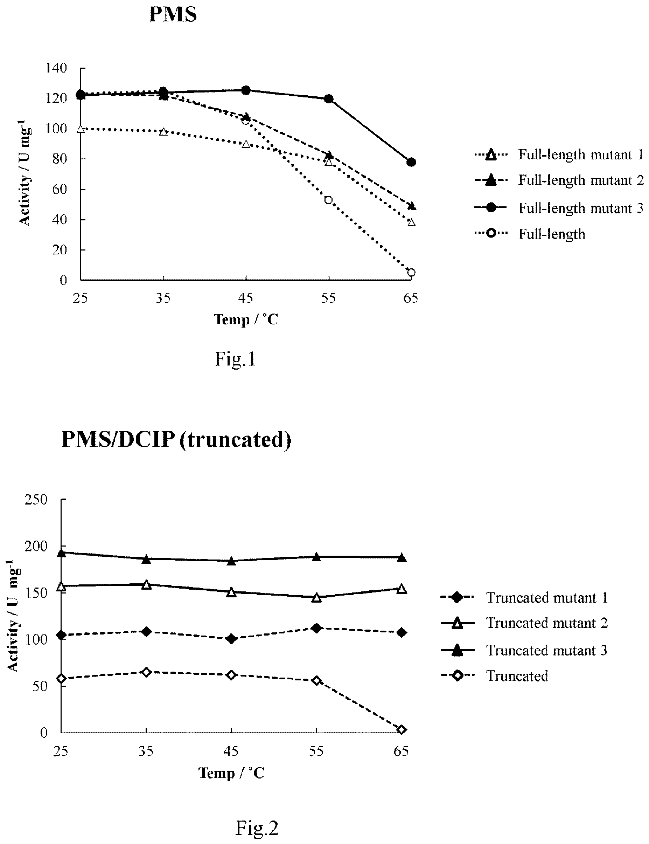

The results are shown in Fig. 1. As a result, QYY with no introduction of a cysteine residue showed a sharp decrease in activity from 55°C, while activity was maintained even at 55°C and 65°C in the cases of Mutant 1, Mutant 2, and Mutant 3.

As shown in Fig. 2, in the cases where the deletion-type β subunit was used, and cysteine was introduced thereto, a decrease in activity was hardly observed.

Subsequently, the thermal stability was evaluated for Mutants 4 and 5, in which cysteine was introduced not only to the α subunit, but also to the γ subunit, to form the α-β and β-γ disulfide bonds.

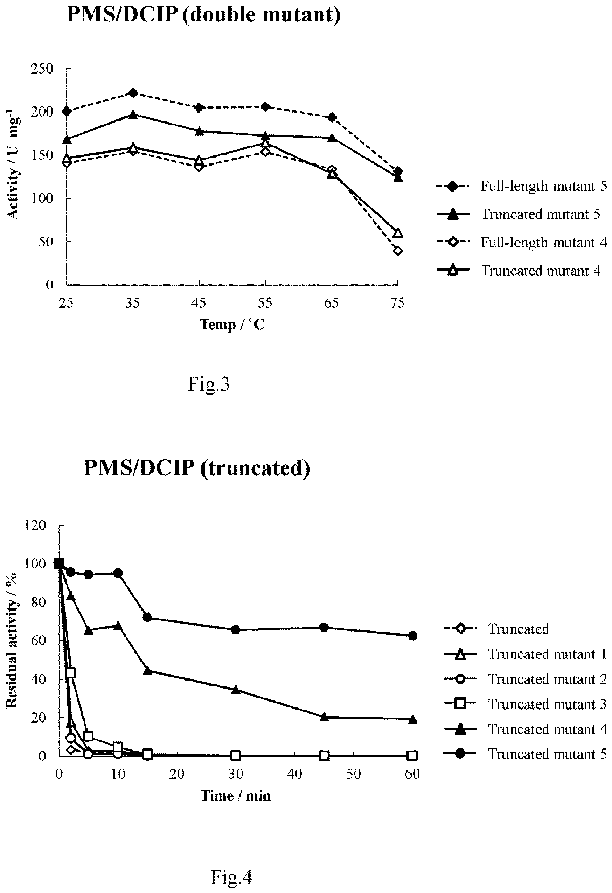

As a result, as shown in Fig. 3, Mutant 4 showed a decrease in activity to about 1/3 relative to the original activity at 75°C. In contrast, Mutant 5, with either the normal-type β subunit or the deletion-type subunit, showed maintenance of about 70% of the original activity at 75°C, showing that its enzyme performance is less likely to decrease even at high temperature.

Subsequently, the activity was investigated after performing heat treatment at 75°C for different lengths of time. As a result, Mutants 4 and 5 showed maintenance of activity even after 60 minutes of the heat treatment. In particular Mutant 5 was found to be an enzyme having high thermal stability with which not less than 60% of the original activity can be maintained even after 60 minutes of the heat treatment.

### Preparation of Enzyme Electrode and Measurement of Glucose

Subsequently, an enzyme electrode was prepared using Mutant 5, and used for measurement of the glucose concentration.

First, an enzyme electrode was prepared by immobilizing the Mutant, on a gold surface through a monolayer-forming molecule. As the monolayer-forming molecule, DSH, shown below, was used.

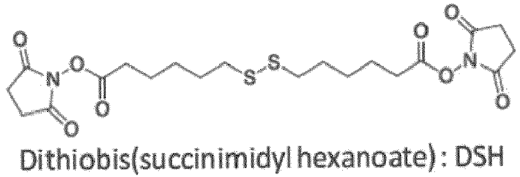

Specifically, a gold wire (diameter, 0.5 mm; length, 6 to 7 cm) was immersed in Piranha solution (200 µl) at room temperature for 2 hours, and then washed with acetone. The wire was then immersed in a solution of DSH in acetone (concentration, 20 µM), and incubated at 25°C for 24 hours to allow binding of the thiol groups of DSH to the gold surface. Subsequently, the wire was washed with acetone, and then immersed in phosphate buffer (300 µl) containing the Mutant GDH (concentration, 0.03 mg/ml), followed by incubation at 4°C overnight to allow binding of the Mutant GDH through a functional group of DSH, thereby obtaining an enzyme electrode in which the Mutant GDH was immobilized on the surface.

Using the enzyme electrode prepared as described above, the response current value for 0 mM (background), 1 mM, 2 mM, 5 mM, 10 mM, 20 mM, or 50 mM aqueous glucose solution was measured by chronoamperometry. The glucose measurement was carried out at 37°C using a counter electrode (Pt wire) and a reference electrode (silver/silver chloride) by application of an electric potential of +0.4 V (vs. silver/silver chloride) to the working electrode.

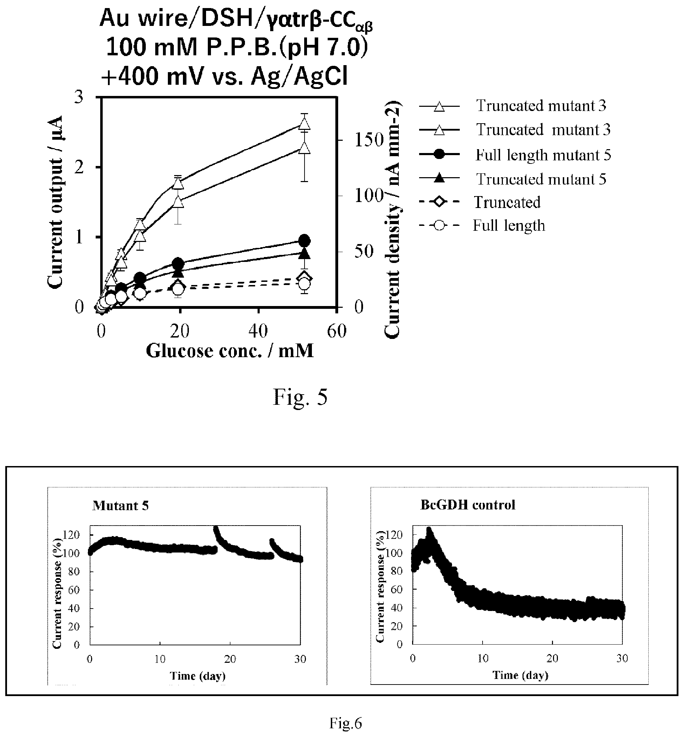

As a result, as shown in Fig. 5, glucose concentration dependency of the response current was found.

Thus, it was shown that the glucose concentration can be measured with a sensor using a GDH in which cysteines are introduced to bind subunits to each other by disulfide bonding. Further, it was surprisingly found that the introduction of the cysteines led to improvement of the electrode properties.

### Continuous Measurement of the Response Current

The enzyme electrode carrying Mutant 5 prepared as described above was immersed in a 20mM glucose solution at room temperature together with a counter electrode (Pt wire) and a reference electrode (silver/silver chloride). The current response was continuously measured for four weeks by application of an electric potential of +0.2 V (vs. silver/silver chloride) to the working electrode and the results were compared to the enzyme electrode carrying QYY GDH with no introduction of a cysteine residue. As a result, as shown in Fig. 6, the current response of the enzyme electrode carrying Mutant 5 was maintained at high level for four weeks whereas the current response of the enzyme electrode carrying QYY GDH decreased to about 40%.

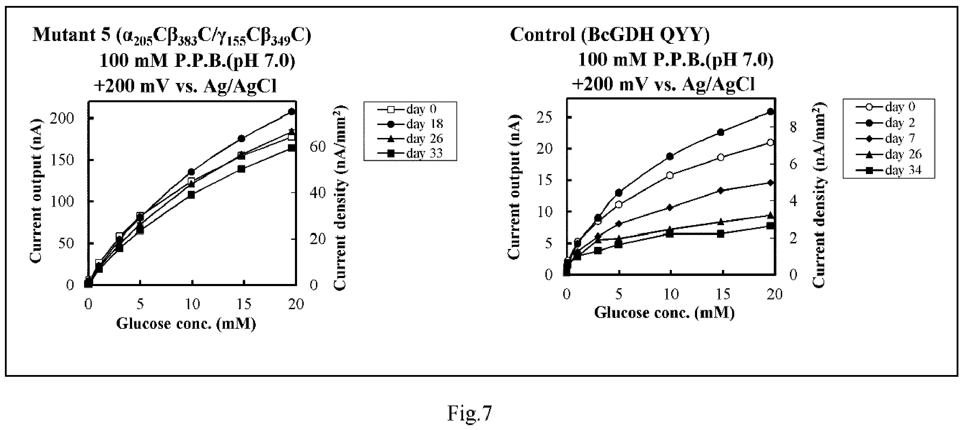

Next, the enzyme electrode carrying Mutant 5 was immersed in a glucose solution (0, 1, 3, 5, 10, 15 and 20mM) at room temperature together with a counter electrode (Pt wire) and a reference electrode (silver/silver chloride). The electric potential of +0.2 V (vs. silver/silver chloride) was continuously applied to the working electrode and the response current was measured over time and plotted and the results were compared to the enzyme electrode carrying QYY GDH with no introduction of a cysteine residue. As a result, as shown in Fig. 7, the glucose concentration-dependency of the current response of the enzyme electrode carrying Mutant 5 was maintained for about five weeks whereas glucose concentration-dependency of the current response of the enzyme electrode carrying QYY GDH was almost lost on Day 26 and Day 34.

These data clearly show that the enzyme having introduced cysteine residues has high stability and can be suitably used for a long-term measurement of glucose.

### Example 2

### Preparation of Mutant GDH from E. americana and Evaluation of Heat Resistance

As a GDH comprising a cytochrome C-containing subunit, a CyGDH derived from *E. americana* was used. The CyGDH derived from *E. americana* is an oligomer enzyme composed of the γ, α, and β subunits. These three subunits are encoded in the gene having the nucleotide sequence of SEQ ID NO:5. The CyGDH gene was inserted into pTrc99A.

With this vector, site-directed mutagenesis was carried out such that the following amino acid substitutions were introduced to the subunits. Mutant E is a mutant in which position 202 of the α subunit is substituted with a cysteine; position 375 of the β subunit is substituted with a cysteine; a disulfide bond (α-β disulfide bond) is formed between these cysteines; position 341 of the β subunit is substituted with a cysteine; position 157 of the γ subunit is substituted with a cysteine; and a disulfide bond (β-γ disulfide bond) is formed between these cysteines.

*E. coli* was transformed with each vector obtained, and the resulting transformant was cultured to allow expression of the mutant GDH.

The culture and purification of glucose dehydrogenase were carried out by the method described in US 2019-0010215.

### Measurement of Enzyme Activity

The mutant GDH was heat-treated at 65°C, and the enzyme activity was measured according to the method described in US 2019-0010215 with a system using PMS/DCIP and the time-dependent change of the enzyme activity was plotted.

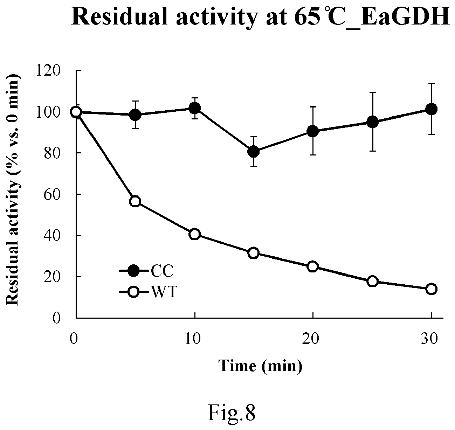

The results are shown in Fig. 8. As a result, the wild-type GDH with no introduction of a cysteine residue showed a sharp decrease in activity at 65°C, while the activity of the Mutant GDH having an α-β disulfide bond and a β-γ disulfide bond was maintained for 30 minutes at 65°C.

These data clearly show that the disulfide bond formation is also effective for enhancing stability of oligomeric GDH enzymes of various origin.

### Example 3

### Preparation of Mutant GDHs from Silvimonas terrae and Evaluation of Heat Resistance

As a GDH comprising a cytochrome C-containing subunit, a CyGDH derived from *Silvimonas terrae* was used. The CyGDH derived from *Silvimonas terrae* is an oligomer enzyme composed of the γ, α, and β subunits. These three subunits are encoded in the gene having the nucleotide sequence of SEQ ID NO: 11. The CyGDH gene was inserted into pTrc99A.

With this vector, site-directed mutagenesis was carried out such that the following amino acid substitutions were introduced to the subunits. Two mutant CyGDHs were prepared: One is a mutant in which proline at position 204 of the α subunit is substituted with a cysteine; histidine at position 382 of the β subunit is substituted with a cysteine; a disulfide bond (α-β disulfide bond) is formed between these cysteines; alanine at position 347 of the β subunit is substituted with a cysteine; alanine at position 168 of the γ subunit is substituted with a cysteine; and a disulfide bond (β-γ disulfide bond) is formed between these cysteines (αP204CβH382C-γA168CβA347C).
The other is a mutant in which proline at position 204 of the α subunit is substituted with a cysteine; histidine at position 382 of the β subunit is substituted with a cysteine; a disulfide bond (α-β disulfide bond) is formed between these cysteines (αP204CβH382C).

*E. coli* was transformed with each vector obtained, and the resulting transformants were cultured to allow expression of the mutant GDHs.

The culture and purification of glucose dehydrogenase were carried out by the method described in US 2019-0010215.

### Measurement of Enzyme Activity

The mutant GDHs as well as the wild type GDH were heat-treated at 60°C, and the enzyme activity was measured according to the method described in US 2019-0010215 with a system using Ru/MTT and the time-dependent change of the enzyme activity was plotted.

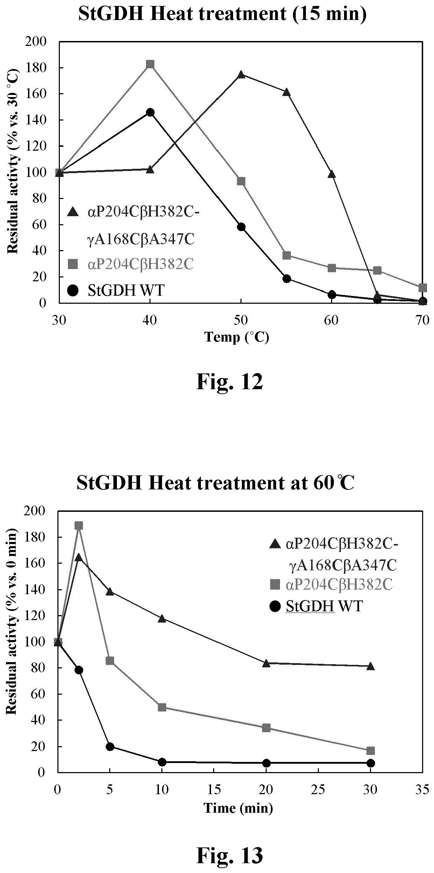

As shown in Fig. 12, the wild-type GDH with no introduction of a cysteine residue showed a sharp decrease in activity as the temperature increased, while the activity of the Mutant GDH having an α-β disulfide bond showed an enhanced thermal stability and the activity of the Mutant GDH having an α-β disulfide bond and a β-γ disulfide bond was maintained up to 60°C. Furthermore, as shown in Fig. 13, the wild-type GDH with no introduction of a cysteine residue showed a sharp decrease in activity at 60°C, while the activity of the Mutant GDH having an α-β disulfide bond showed an enhanced thermal stability and the activity of the Mutant GDH having an α-β disulfide bond and a β-γ disulfide bond was maintained for 30 minutes at 60°C.

### Example 4

### Preparation of Mutant GDHs from Zymobacter palmae and Evaluation of Heat Resistance

As a GDH comprising a cytochrome C-containing subunit, a CyGDH derived from *Zymobacter palmae* was used. The CyGDH derived from *Zymobacter palmae* is an oligomer enzyme composed of the γ, α, and β subunits. These three subunits are encoded in the gene having the nucleotide sequence of SEQ ID NO: 15. A modified CyGDH gene in which the nucleotide "g" at position 55 is replaced with "a" such that Val at position 19 in SEQ ID NO: 16 is changed to Met and the γ subunit is translated from this position was inserted into pTrc99A.

With this vector, site-directed mutagenesis was carried out such that the following amino acid substitutions were introduced to the subunits. Two mutant CyGDHs were prepared: One is a mutant in which proline at position 200 of the α subunit is substituted with a cysteine; aspartic acid at position 389 of the β subunit is substituted with a cysteine; a disulfide bond (α-β disulfide bond) is formed between these cysteines; tyrosine at position 355 of the β subunit is substituted with a cysteine; arginine at position 164 of the γ subunit which corresponds to arginine at position 182 in SEQ ID NO: 16 is substituted with a cysteine; and a disulfide bond (β-γ disulfide bond) is formed between these cysteines (αP200CβD389C-γR182CβY355C). The other is a mutant in which proline at position 200 of the α subunit is substituted with a cysteine; aspartic acid at position 389 of the β subunit is substituted with a cysteine; a disulfide bond (α-β disulfide bond) is formed between these cysteines (αP200CβD389C).

*E. coli* was transformed with each vector obtained, and the resulting transformants were cultured to allow expression of the mutant GDHs.

The culture and purification of glucose dehydrogenase were carried out by the method described in US 2019-0010215.

### Measurement of Enzyme Activity

The mutant GDHs as well as the wild type GDH were heat-treated at 60°C, and the enzyme activity was measured according to the method described in US 2019-0010215 with a system using Ru/MTT and the time-dependent change of the enzyme activity was plotted.

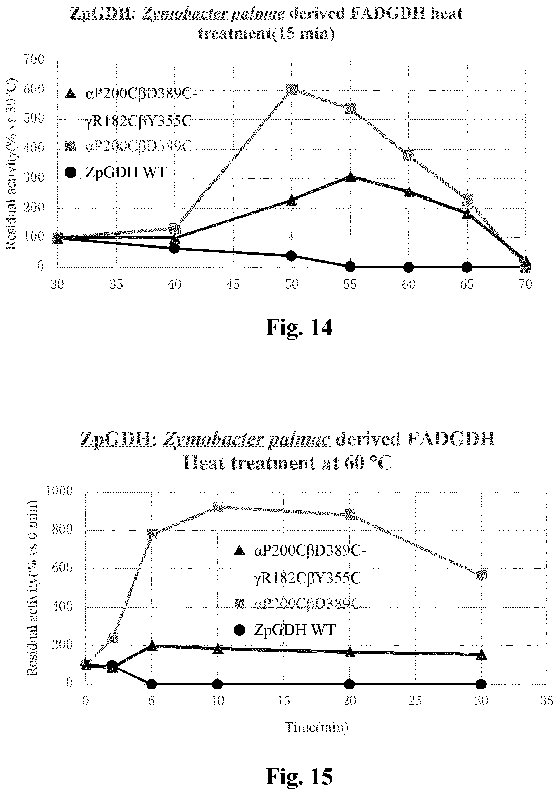

As shown in Fig. 14, the wild-type GDH with no introduction of a cysteine residue showed a decrease in activity as the temperature increased, while the activity of the Mutant GDH having an α-β disulfide bond and the activity of the Mutant GDH having an α-β disulfide bond and a β-γ disulfide bond was maintained at high levels up to 65°C. Furthermore, as shown in Fig. 15, the wild-type GDH with no introduction of a cysteine residue showed a sharp decrease to zero in activity at 60°C, while the activity of the Mutant GDH having an α-β disulfide bond and the activity of the Mutant GDH having an α-β disulfide bond and a β-γ disulfide bond was maintained at high levels for 30 minutes at 60°C.

### Example 5

### Preparation of Mutant GDHs from Cobetia sp. and Evaluation of Heat Resistance

As a GDH comprising a cytochrome C-containing subunit, a CyGDH derived from *Cobetia sp.* was used. The CyGDH derived from *Cobetia sp.* is an oligomer enzyme composed of the γ, α, and β subunits. These three subunits are encoded in the gene having the nucleotide sequence of SEQ ID NO: 19. A CyGDH gene in which the γ subunit (amino acids 14-207 of SEQ ID NO: 20) is translated from the "atg" codon at positions 40-42 in SEQ ID NO: 19 was inserted into pTrc99A.

With this vector, site-directed mutagenesis was carried out such that the following amino acid substitutions were introduced to the subunits. One mutant CyGDH was prepared which is a mutant in which proline at position 204 of the α subunit is substituted with a cysteine; serine at position 467 of the β subunit is substituted with a cysteine; a disulfide bond (α-β disulfide bond) is formed between these cysteines; tyrosine at position 433 of the β subunit is substituted with a cysteine; glutamine at position 185 of the γ subunit which corresponds to glutamine at position 198 in SEQ ID NO: 20 is substituted with a cysteine; and a disulfide bond (β-γ disulfide bond) is formed between these cysteines (αP204CβS467C-γQ198CβY433C).

*E. coli* was transformed with each vector obtained, and the resulting transformants were cultured to allow expression of the mutant GDHs.

The culture and purification of glucose dehydrogenase were carried out by the method described in US 2019-0010215.

### Measurement of Enzyme Activity

The mutant GDH as well as the wild type GDH were heat-treated at 60°C, and the enzyme activity was measured according to the method described in US 2019-0010215 with a system using Ru/MTT and the time-dependent change of the enzyme activity was plotted.

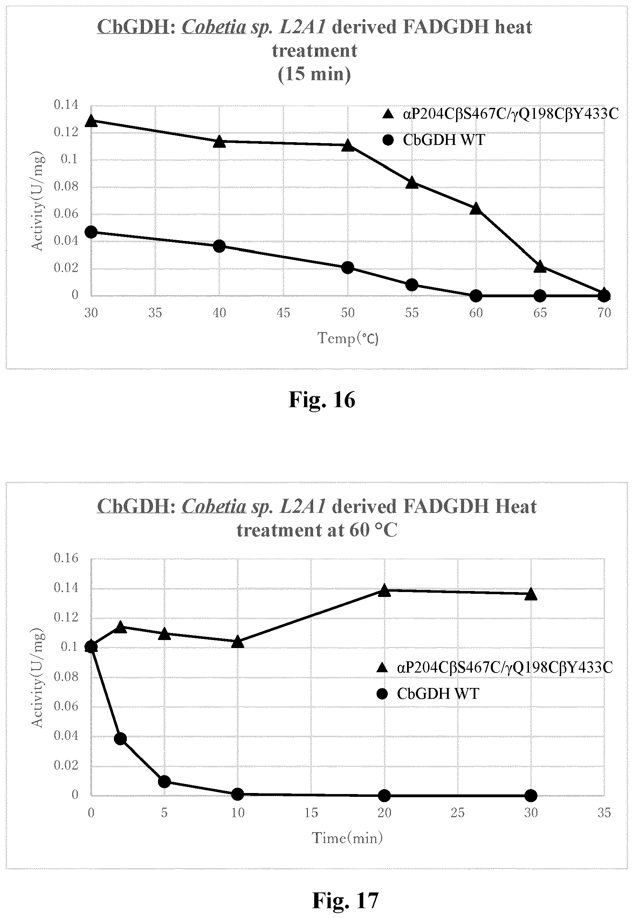

As shown in Fig. 16, the wild-type GDH with no introduction of a cysteine residue showed a decrease in activity as the temperature increased, while the activity of the Mutant GDH having an α-β disulfide bond and a β-γ disulfide bond was maintained at high levels up to 60°C. Furthermore, as shown in Fig. 17, the wild-type GDH with no introduction of a cysteine residue showed a sharp decrease to zero in activity at 60°C, while the activity of the Mutant GDH having an α-β disulfide bond and a β-γ disulfide bond was maintained at high levels for 30 minutes at 60°C.

These data clearly show that the disulfide bond formation is also effective for enhancing stability of oligomeric GDH enzymes of various origin.

## Claims

1. A mutant FAD-dependent glucose dehydrogenase comprising:
a catalytic subunit;
an electron transfer subunit; and
a hitchhiker subunit;
wherein each of the amino acid sequence of the catalytic subunit and the amino acid sequence of the electron transfer subunit comprises a cysteine residue introduced therein, the catalytic subunit and the electron transfer subunit being bound to each other through a disulfide bond between the cysteine residues.

2. The mutant FAD-dependent glucose dehydrogenase according to claim 1, wherein the amino acid sequence of the catalytic subunit comprises a cysteine residue introduced in a region corresponding to the region of amino acid positions 200 to 240 of SEQ ID NO:3.

3. The mutant FAD-dependent glucose dehydrogenase according to claim 2, wherein the amino acid sequence of the catalytic subunit comprises a cysteine residue introduced at an amino acid residue corresponding to the proline residue at position 205, glycine residue at position 208, asparagine residue at position 215, isoleucine residue at position 224, or glutamic acid residue at position 235 of SEQ ID NO:3.

4. The mutant FAD-dependent glucose dehydrogenase according to any one of claims 1 to 3, wherein the amino acid sequence of the electron transfer subunit comprises a cysteine residue introduced in a region corresponding to the region of amino acid positions 330 to 400 of SEQ ID NO:4.

5. The mutant FAD-dependent glucose dehydrogenase according to claim 4, wherein the amino acid sequence of the electron transfer subunit comprises a cysteine residue introduced at an amino acid residue corresponding to the threonine residue at position 336, glycine residue at position 385, aspartic acid residue at position 383, or tyrosine residue at position 391 of SEQ ID NO:4.

6. The mutant FAD-dependent glucose dehydrogenase according to any one of claims 1 to 5, wherein the amino acid sequence of the electron transfer subunit comprises another cysteine residue introduced therein, and the amino acid sequence of the hitchhiker subunit comprises a cysteine residue introduced therein, the hitchhiker subunit and the electron transfer subunit being bound to each other through a disulfide bond between the cysteine residues.

7. The mutant FAD-dependent glucose dehydrogenase according to claim 6, wherein the amino acid sequence of the electron transfer subunit comprises another cysteine residue introduced in a region corresponding to the region of amino acid positions 341 to 350 of SEQ ID NO:4, wherein preferably the amino acid sequence of the electron transfer subunit comprises said another cysteine residue introduced at an amino acid residue corresponding to the threonine residue at position 345, proline residue at position 346, or tyrosine residue at position 349 of SEQ ID NO:4.

8. The mutant FAD-dependent glucose dehydrogenase according to any one of claims 6 to 7, wherein the amino acid sequence of the hitchhiker subunit comprises a cysteine residue introduced in a region corresponding to the region of amino acid positions 140 to 160 of SEQ ID NO:2, wherein preferably the amino acid sequence of the hitchhiker subunit comprises said cysteine residue introduced at an amino acid residue corresponding to the threonine residue at position 145, asparagine residue at position 154, or lysine residue at position 155 of SEQ ID NO:2.

9. The mutant FAD-dependent glucose dehydrogenase according to any one of claims 1 to 8, wherein
the amino acid sequence of the catalytic subunit has an identity of at least 60% to SEQ ID NO:3,
the amino acid sequence of the electron transfer subunit has an identity of at least 60% to
the amino acid sequence of SEQ ID NO:4, or
the amino acid sequence of amino acid positions 314 to 425 of SEQ ID NO:4, and
the amino acid sequence of the hitchhiker subunit has an identity of at least 60% to SEQ ID NO:2.

10. The mutant FAD-dependent glucose dehydrogenase according to any one of claims 1 to 8, wherein the catalytic subunit comprises an amino acid sequence having at least 60% identity to SEQ ID NO: 7 wherein cysteine is introduced at the position corresponding to the proline residue at position 202, glycine residue at position 205, asparagine residue at position 212, isoleucine residue at position 221, or glutamic acid residue at position 232 in SEQ ID NO: 7, the electron transfer subunit comprises an amino acid sequence having at least 60% identity to SEQ ID NO: 8 or amino acids 306 to 420 of SEQ ID NO: 8 wherein a first cysteine is introduced at the position corresponding to the serine residue at position 328, glutamic acid residue at position 375, phenylalanine residue at position 377, or aspartic acid residue at position 383 in SEQ ID NO: 8 and a second cysteine is introduced at the position corresponding to the serine residue at position 337, glutamine residue at position 338, or tyrosine residue at position 341 in SEQ ID NO: 8, and
the hitchhiker subunit comprises an amino acid sequence having at least 60% identity to SEQ ID NO: 6 wherein cysteine is introduced at the position corresponding to the valine residue at position 147, asparagine residue at position 156, or arginine residue at position 157 in SEQ ID NO: 6.

11. The mutant FAD-dependent glucose dehydrogenase according to any one of claims 1 to 8, wherein the catalytic subunit comprises an amino acid sequence having at least 60% identity to SEQ ID NO: 13 wherein cysteine is introduced at the position corresponding to the proline residue at position 204 in SEQ ID NO: 13,
the electron transfer subunit comprises an amino acid sequence having at least 60% identity to SEQ ID NO: 14 or amino acids 332 to 481 of SEQ ID NO: 14 wherein a first cysteine is introduced at the position corresponding to the histidine residue at position 382 in SEQ ID NO: 14 and a second cysteine is introduced at the position corresponding to the alanine residue at position 347 in SEQ ID NO: 14, and
the hitchhiker subunit comprises an amino acid sequence having at least 60% identity to SEQ ID NO: 12 wherein cysteine is introduced at the position corresponding to the alanine residue at position 168 in SEQ ID NO: 12.

12. The mutant FAD-dependent glucose dehydrogenase according to any one of claims 1 to 8, wherein the catalytic subunit comprises an amino acid sequence having at least 60% identity to SEQ ID NO: 17 wherein cysteine is introduced at the position corresponding to the proline residue at position 200 in SEQ ID NO: 17,
the electron transfer subunit comprises an amino acid sequence having at least 60% identity to SEQ ID NO: 18 or amino acids 340 to 465 of SEQ ID NO: 18 wherein a first cysteine is introduced at the position corresponding to the aspartic acid residue at position 389 in SEQ ID NO: 18 and a second cysteine is introduced at the position corresponding to the tyrosine residue at position 355 in SEQ ID NO: 18, and
the hitchhiker subunit comprises an amino acid sequence having at least 60% identity to SEQ ID NO: 16 or amino acids 19 to 195 of SEQ ID NO: 16 wherein cysteine is introduced at the position corresponding to the arginine residue at position 182 in SEQ ID NO: 16.

13. The mutant FAD-dependent glucose dehydrogenase according to any one of claims 1 to 8, wherein the catalytic subunit comprises an amino acid sequence having at least 60% identity to SEQ ID NO: 21 wherein cysteine is introduced at the position corresponding to the proline residue at position 204 in SEQ ID NO: 21,
the electron transfer subunit comprises an amino acid sequence having at least 60% identity to SEQ ID NO: 22 or amino acids 418 to 522 of SEQ ID NO: 22 wherein a first cysteine is introduced at the position corresponding to the serine residue at position 467 in SEQ ID NO: 22 and a second cysteine is introduced at the position corresponding to the tyrosine residue at position 433 in SEQ ID NO: 22, and
the hitchhiker subunit comprises an amino acid sequence having at least 60% identity to SEQ ID NO: 20 or amino acids 14 to 207 of SEQ ID NO: 20 wherein cysteine is introduced at the position corresponding to the glutamine residue at position 198 in SEQ ID NO: 20.

14. An enzyme electrode comprising the FAD-dependent glucose dehydrogenase according to any one of claims 1 to 13.

15. A biosensor comprising the enzyme electrode according to claim 14.


SEQUENCE LISTING

<110>  The University of North Carolina at Chapel Hill
       Arkray, Inc.

<120>  MUTANT GLUCOSE DEHYDROGENASE HAVING IMPROVED THERMAL STABILITY,
       AND USE THEREOF

<130>  42.157409

<150>  US17/215476
<151>  2021-03-29

<160>  31

<170>  PatentIn version 3.5


<210>  1
<211>  3706
<212>  DNA
<213>  Burkholderia cepacia


<220>
<221>  CDS
<222>  (258)..(761)

<220>
<221>  CDS
<222>  (764)..(2380)

<220>
<221>  CDS
<222>  (2386)..(3660)

<400>  1
aagctttctg tttgattgca cgcgattcta accgagcgtc tgtgaggcgg aacgcgacat      60
gcttcgtgtc gcacacgtgt cgcgccgacg acacaaaaat gcagcgaaat ggctgatcgt     120
tacgaatggc tgacacattg aatggactat aaaaccattg tccgttccgg aatgtgcgcg     180
tacatttcag gtccgcgccg atttttgaga aatatcaagc gtggttttcc cgaatccggt     240
gttcgagaga aggaaac atg cac aac gac aac act ccc cac tcg cgt cgc        290
                   Met His Asn Asp Asn Thr Pro His Ser Arg Arg
                   1               5                   10
cac ggc gac gca gcc gca tca ggc atc acg cgg cgt caa tgg ttg caa       338
His Gly Asp Ala Ala Ala Ser Gly Ile Thr Arg Arg Gln Trp Leu Gln
            15                  20                  25
ggc gcg ctg gcg ctg acc gca gcg ggc ctc acg ggt tcg ctg aca ttg       386
Gly Ala Leu Ala Leu Thr Ala Ala Gly Leu Thr Gly Ser Leu Thr Leu
        30                  35                  40
cgg gcg ctt gca gac aac ccc ggc act gcg ccg ctc gat acg ttc atg       434
Arg Ala Leu Ala Asp Asn Pro Gly Thr Ala Pro Leu Asp Thr Phe Met
    45                  50                  55
acg ctt tcc gaa tcg ctg acc ggc aag aaa ggg ctc agc cgc gtg atc       482
Thr Leu Ser Glu Ser Leu Thr Gly Lys Lys Gly Leu Ser Arg Val Ile
60                  65                  70                  75
ggc gag cgc ctg ctg cag gcg ctg cag aag ggc tcg ttc aag acg gcc       530
Gly Glu Arg Leu Leu Gln Ala Leu Gln Lys Gly Ser Phe Lys Thr Ala
                80                  85                  90
gac agc ctg ccg cag ctc gcc ggc gcg ctc gcg tcc ggt tcg ctg acg       578
Asp Ser Leu Pro Gln Leu Ala Gly Ala Leu Ala Ser Gly Ser Leu Thr
            95                  100                 105
cct gaa cag gaa tcg ctc gca ctg acg atc ctc gag gcc tgg tat ctc       626

Pro Glu Gln Glu Ser Leu Ala Leu Thr Ile Leu Glu Ala Trp Tyr Leu
        110                 115                 120
ggc atc gtc gac aac gtc gtg att acg tac gag gaa gca tta atg ttc         674
Gly Ile Val Asp Asn Val Val Ile Thr Tyr Glu Glu Ala Leu Met Phe
    125                 130                 135
ggc gtc gtg tcc gat acg ctc gtg atc cgt tcg tat tgc ccc aac aaa         722
Gly Val Val Ser Asp Thr Leu Val Ile Arg Ser Tyr Cys Pro Asn Lys
140                 145                 150                 155
ccc ggc ttc tgg gcc gac aaa ccg atc gag agg caa gcc tg atg gcc          769
Pro Gly Phe Trp Ala Asp Lys Pro Ile Glu Arg Gln Ala    Met Ala
                160                 165                 170
gat acc gat acg caa aag gcc gac gtc gtc gtc gtt gga tcg ggt gtc         817
Asp Thr Asp Thr Gln Lys Ala Asp Val Val Val Val Gly Ser Gly Val
                175                 180                 185
gcg ggc gcg atc gtc gcg cat cag ctc gcg atg gcg ggc aag gcg gtg         865
Ala Gly Ala Ile Val Ala His Gln Leu Ala Met Ala Gly Lys Ala Val
            190                 195                 200
atc ctg ctc gaa gcg ggc ccg cgc atg ccg cgc tgg gaa atc gtc gag         913
Ile Leu Leu Glu Ala Gly Pro Arg Met Pro Arg Trp Glu Ile Val Glu
        205                 210                 215
cgc ttc cgc aat cag ccc gac aag atg gac ttc atg gcg ccg tac ccg         961
Arg Phe Arg Asn Gln Pro Asp Lys Met Asp Phe Met Ala Pro Tyr Pro
    220                 225                 230
tcg agc ccc tgg gcg ccg cat ccc gag tac ggc ccg ccg aac gac tac         1009
Ser Ser Pro Trp Ala Pro His Pro Glu Tyr Gly Pro Pro Asn Asp Tyr
235                 240                 245                 250
ctg atc ctg aag ggc gag cac aag ttc aac tcg cag tac atc cgc gcg         1057
Leu Ile Leu Lys Gly Glu His Lys Phe Asn Ser Gln Tyr Ile Arg Ala
                255                 260                 265
gtg ggc ggc acg acg tgg cac tgg gcc gcg tcg gcg tgg cgc ttc att         1105
Val Gly Gly Thr Thr Trp His Trp Ala Ala Ser Ala Trp Arg Phe Ile
            270                 275                 280
ccg aac gac ttc aag atg aag agc gtg tac ggc gtc ggc cgc gac tgg         1153
Pro Asn Asp Phe Lys Met Lys Ser Val Tyr Gly Val Gly Arg Asp Trp
        285                 290                 295
ccg atc cag tac gac gat ctc gag ccg tac tat cag cgc gcg gag gaa         1201
Pro Ile Gln Tyr Asp Asp Leu Glu Pro Tyr Tyr Gln Arg Ala Glu Glu
    300                 305                 310
gag ctc ggc gtg tgg ggc ccg ggc ccc gag gaa gat ctg tac tcg ccg         1249
Glu Leu Gly Val Trp Gly Pro Gly Pro Glu Glu Asp Leu Tyr Ser Pro
315                 320                 325                 330
cgc aag cag ccg tat ccg atg ccg ccg ctg ccg ttg tcg ttc aac gag         1297
Arg Lys Gln Pro Tyr Pro Met Pro Pro Leu Pro Leu Ser Phe Asn Glu
                335                 340                 345
cag acc atc aag acg gcg ctg aac aac tac gat ccg aag ttc cat gtc         1345
Gln Thr Ile Lys Thr Ala Leu Asn Asn Tyr Asp Pro Lys Phe His Val
            350                 355                 360
gtg acc gag ccg gtc gcg cgc aac agc cgc ccg tac gac ggc cgc ccg         1393
Val Thr Glu Pro Val Ala Arg Asn Ser Arg Pro Tyr Asp Gly Arg Pro
        365                 370                 375
act tgt tgc ggc aac aac aac tgc atg ccg atc tgc ccg atc ggc gcg         1441
Thr Cys Cys Gly Asn Asn Asn Cys Met Pro Ile Cys Pro Ile Gly Ala
    380                 385                 390
atg tac aac ggc atc gtg cac gtc gag aag gcc gaa cgc gcc ggc gcg         1489
Met Tyr Asn Gly Ile Val His Val Glu Lys Ala Glu Arg Ala Gly Ala
395                 400                 405                 410
aag ctg atc gag aac gcg gtc gtc tac aag ctc gag acg ggc ccg gac         1537
Lys Leu Ile Glu Asn Ala Val Val Tyr Lys Leu Glu Thr Gly Pro Asp
                415                 420                 425
aag cgc atc gtc gcg gcg ctc tac aag gac aag acg ggc gcc gag cat         1585
Lys Arg Ile Val Ala Ala Leu Tyr Lys Asp Lys Thr Gly Ala Glu His
            430                 435                 440
cgc gtc gaa ggc aag tat ttc gtg ctc gcc gcg aac ggc atc gag acg         1633

Arg Val Glu Gly Lys Tyr Phe Val Leu Ala Ala Asn Gly Ile Glu Thr
        445                 450                 455

ccg aag atc ctg ctg atg tcc gcg aac cgc gat ttc ccg aac ggt gtc        1681
Pro Lys Ile Leu Leu Met Ser Ala Asn Arg Asp Phe Pro Asn Gly Val
    460                 465                 470

gcg aac agc tcg gac atg gtc ggc cgc aac ctg atg gac cat ccg ggc        1729
Ala Asn Ser Ser Asp Met Val Gly Arg Asn Leu Met Asp His Pro Gly
475                 480                 485                 490

acc ggc gtg tcg ttc tat gcg agc gag aag ctg tgg ccg ggc cgc ggc        1777
Thr Gly Val Ser Phe Tyr Ala Ser Glu Lys Leu Trp Pro Gly Arg Gly
                495                 500                 505

ccg cag gag atg acg tcg ctg atc ggt ttc cgc gac ggt ccg ttc cgc        1825
Pro Gln Glu Met Thr Ser Leu Ile Gly Phe Arg Asp Gly Pro Phe Arg
            510                 515                 520

gcg acc gaa gcg gcg aag aag atc cac ctg tcg aac ctg tcg cgc atc        1873
Ala Thr Glu Ala Ala Lys Lys Ile His Leu Ser Asn Leu Ser Arg Ile
        525                 530                 535

gac cag gag acg cag aag atc ttc aag gcc ggc aag ctg atg aag ccc        1921
Asp Gln Glu Thr Gln Lys Ile Phe Lys Ala Gly Lys Leu Met Lys Pro
    540                 545                 550

gac gag ctc gac gcg cag atc cgc gac cgt tcc gca cgc tac gtg cag        1969
Asp Glu Leu Asp Ala Gln Ile Arg Asp Arg Ser Ala Arg Tyr Val Gln
555                 560                 565                 570

ttc gac tgc ttc cac gaa atc ctg ccg caa ccc gag aac cgc atc gtg        2017
Phe Asp Cys Phe His Glu Ile Leu Pro Gln Pro Glu Asn Arg Ile Val
                575                 580                 585

ccg agc aag acg gcg acc gat gcg atc ggc att ccg cgc ccc gag atc        2065
Pro Ser Lys Thr Ala Thr Asp Ala Ile Gly Ile Pro Arg Pro Glu Ile
            590                 595                 600

acg tat gcg atc gac gac tac gtg aag cgc ggc gcc gcg cat acg cgc        2113
Thr Tyr Ala Ile Asp Asp Tyr Val Lys Arg Gly Ala Ala His Thr Arg
        605                 610                 615

gag gtc tac gcg acc gcc gcg aag gtg ctc ggc ggc acg gac gtc gtg        2161
Glu Val Tyr Ala Thr Ala Ala Lys Val Leu Gly Gly Thr Asp Val Val
    620                 625                 630

ttc aac gac gaa ttc gcg ccg aac aat cac atc acg ggc tcg acg atc        2209
Phe Asn Asp Glu Phe Ala Pro Asn Asn His Ile Thr Gly Ser Thr Ile
635                 640                 645                 650

atg ggc gcc gat gcg cgc gac tcc gtc gtc gac aag gac tgc cgc acg        2257
Met Gly Ala Asp Ala Arg Asp Ser Val Val Asp Lys Asp Cys Arg Thr
                655                 660                 665

ttc gac cat ccg aac ctg ttc att tcg agc agc gcg acg atg ccg acc        2305
Phe Asp His Pro Asn Leu Phe Ile Ser Ser Ser Ala Thr Met Pro Thr
            670                 675                 680

gtc ggt acc gta aac gtg acg ctg acg atc gcc gcg ctc gcg ctg cgg        2353
Val Gly Thr Val Asn Val Thr Leu Thr Ile Ala Ala Leu Ala Leu Arg
        685                 690                 695

atg tcg gac acg ctg aag aag gaa gtc tgacc gtg cgg aaa tct act ctc        2403
Met Ser Asp Thr Leu Lys Lys Glu Val           Val Arg Lys Ser Thr Leu
    700                 705                           710

act ttc ctc atc gcc ggc tgc ctc gcg ttg ccg ggc ttc gcg cgc gcg        2451
Thr Phe Leu Ile Ala Gly Cys Leu Ala Leu Pro Gly Phe Ala Arg Ala
    715                 720                 725

gcc gat gcg gcc gat ccg gcg ctg gtc aag cgc ggc gaa tac ctc gcg        2499
Ala Asp Ala Ala Asp Pro Ala Leu Val Lys Arg Gly Glu Tyr Leu Ala
730                 735                 740                 745

acc gcc ggc gac tgc atg gcc tgc cac acc gtg aag ggc ggc aag ccg        2547
Thr Ala Gly Asp Cys Met Ala Cys His Thr Val Lys Gly Gly Lys Pro
                750                 755                 760

tac gcg ggc ggc ctt ggc atg ccg gta ccg atg ctc ggc aag atc tac        2595
Tyr Ala Gly Gly Leu Gly Met Pro Val Pro Met Leu Gly Lys Ile Tyr
            765                 770                 775

acg agc aac atc acg ccc gat ccc gat acg ggc atc ggc aaa tgg acg        2643

Thr Ser Asn Ile Thr Pro Asp Pro Asp Thr Gly Ile Gly Lys Trp Thr
        780                 785                 790
ttc gag gac ttc gag cgc gcg gtg cgg cac ggc gtg tcg aag aac ggc 2691
Phe Glu Asp Phe Glu Arg Ala Val Arg His Gly Val Ser Lys Asn Gly
    795                 800                 805
gac aac ctg tat ccg gcg atg ccg tac gtg tcg tac gcg aag atc acg 2739
Asp Asn Leu Tyr Pro Ala Met Pro Tyr Val Ser Tyr Ala Lys Ile Thr
810                 815                 820                 825
gac gac gac gta cgc gcg ctg tac gcc tac ttc atg cac ggc gtc gag 2787
Asp Asp Asp Val Arg Ala Leu Tyr Ala Tyr Phe Met His Gly Val Glu
                830                 835                 840
ccg gtc aag cag gcg ccg ccg aag aac gag att ccc gcg ctg ctc agc 2835
Pro Val Lys Gln Ala Pro Pro Lys Asn Glu Ile Pro Ala Leu Leu Ser
            845                 850                 855
atg cgc tgg ccg ctg aag atc tgg aac tgg ctg ttc ctg aag gac ggc 2883
Met Arg Trp Pro Leu Lys Ile Trp Asn Trp Leu Phe Leu Lys Asp Gly
        860                 865                 870
ccg tac cag ccg aag ccg tcg cag agc gcc gaa tgg aat cgc ggc gcg 2931
Pro Tyr Gln Pro Lys Pro Ser Gln Ser Ala Glu Trp Asn Arg Gly Ala
    875                 880                 885
tat ctg gtg cag ggt ctc gcg cac tgc agc acg tgc cac acg ccg cgc 2979
Tyr Leu Val Gln Gly Leu Ala His Cys Ser Thr Cys His Thr Pro Arg
890                 895                 900                 905
ggc atc gcg atg cag gag aag tcg ctc gac gaa acc ggc ggc agc ttc 3027
Gly Ile Ala Met Gln Glu Lys Ser Leu Asp Glu Thr Gly Gly Ser Phe
                910                 915                 920
ctc gcg ggg tcg gtg ctc gcc ggc tgg gac ggc tac aac atc acg tcg 3075
Leu Ala Gly Ser Val Leu Ala Gly Trp Asp Gly Tyr Asn Ile Thr Ser
            925                 930                 935
gac ccg aat gcg ggg atc ggc agc tgg acg cag cag cag ctc gtg cag 3123
Asp Pro Asn Ala Gly Ile Gly Ser Trp Thr Gln Gln Gln Leu Val Gln
        940                 945                 950
tat ttg cgc acc ggc agc gtg ccg ggc gtc gcg cag gcg gcc ggg ccg 3171
Tyr Leu Arg Thr Gly Ser Val Pro Gly Val Ala Gln Ala Ala Gly Pro
    955                 960                 965
atg gcc gag gcg gtc gag cac agc ttc tcg aag atg acc gaa gcg gac 3219
Met Ala Glu Ala Val Glu His Ser Phe Ser Lys Met Thr Glu Ala Asp
970                 975                 980                 985
atc ggt gcg atc gcc acg tac gtc cgc acg gtg ccg gcc gtt gcc gac 3267
Ile Gly Ala Ile Ala Thr Tyr Val Arg Thr Val Pro Ala Val Ala Asp
                990                 995                 1000
agc aac gcg aag cag ccg cgg tcg tcg tgg ggc aag ccg gcc gag 3312
Ser Asn Ala Lys Gln Pro Arg Ser Ser Trp Gly Lys Pro Ala Glu
            1005                1010                1015
gac ggg ctg aag ctg cgc ggt gtc gcg ctc gcg tcg tcg ggc atc 3357
Asp Gly Leu Lys Leu Arg Gly Val Ala Leu Ala Ser Ser Gly Ile
            1020                1025                1030
gat ccg gcg cgg ctg tat ctc ggc aac tgc gcg acg tgc cac cag 3402
Asp Pro Ala Arg Leu Tyr Leu Gly Asn Cys Ala Thr Cys His Gln
            1035                1040                1045
atg cag ggc aag ggc acg ccg gac ggc tat tac ccg tcg ctg ttc 3447
Met Gln Gly Lys Gly Thr Pro Asp Gly Tyr Tyr Pro Ser Leu Phe
            1050                1055                1060
cac aac tcc acc gtc ggc gcg tcg aat ccg tcg aac ctc gtg cag 3492
His Asn Ser Thr Val Gly Ala Ser Asn Pro Ser Asn Leu Val Gln
            1065                1070                1075
gtg atc ctg aac ggc gtg cag cgc aag atc ggc agc gag gat atc 3537
Val Ile Leu Asn Gly Val Gln Arg Lys Ile Gly Ser Glu Asp Ile
            1080                1085                1090
ggg atg ccc gct ttc cgc tac gat ctg aac gac gcg cag atc gcc 3582
Gly Met Pro Ala Phe Arg Tyr Asp Leu Asn Asp Ala Gln Ile Ala
            1095                1100                1105
gcg ctg acg aac tac gtg acc gcg cag ttc ggc aat ccg gcg gcg 3627

Ala Leu Thr Asn  Tyr Val Thr Ala Gln  Phe Gly Asn Pro Ala  Ala
            1110                1115                1120
aag gtg acg gag  cag gac gtc gcg aag  ctg cgc tgacatagtc            3670
Lys Val Thr Glu  Gln Asp Val Ala Lys  Leu Arg
            1125                1130
gggcgcgccg acacggcgca accgatagga caggag                             3706

<210>  2
<211>  168
<212>  PRT
<213>  Burkholderia cepacia

<400>  2
Met His Asn Asp Asn Thr Pro His Ser Arg Arg His Gly Asp Ala Ala
1               5                   10                  15
Ala Ser Gly Ile Thr Arg Arg Gln Trp Leu Gln Gly Ala Leu Ala Leu
            20                  25                  30
Thr Ala Ala Gly Leu Thr Gly Ser Leu Thr Leu Arg Ala Leu Ala Asp
        35                  40                  45
Asn Pro Gly Thr Ala Pro Leu Asp Thr Phe Met Thr Leu Ser Glu Ser
    50                  55                  60
Leu Thr Gly Lys Lys Gly Leu Ser Arg Val Ile Gly Glu Arg Leu Leu
65                  70                  75                  80
Gln Ala Leu Gln Lys Gly Ser Phe Lys Thr Ala Asp Ser Leu Pro Gln
                85                  90                  95
Leu Ala Gly Ala Leu Ala Ser Gly Ser Leu Thr Pro Glu Gln Glu Ser
            100                 105                 110
Leu Ala Leu Thr Ile Leu Glu Ala Trp Tyr Leu Gly Ile Val Asp Asn
        115                 120                 125
Val Val Ile Thr Tyr Glu Glu Ala Leu Met Phe Gly Val Val Ser Asp
    130                 135                 140
Thr Leu Val Ile Arg Ser Tyr Cys Pro Asn Lys Pro Gly Phe Trp Ala
145                 150                 155                 160
Asp Lys Pro Ile Glu Arg Gln Ala
                165

<210>  3
<211>  539
<212>  PRT
<213>  Burkholderia cepacia

<400>  3
Met Ala Asp Thr Asp Thr Gln Lys Ala Asp Val Val Val Val Gly Ser
1               5                   10                  15
Gly Val Ala Gly Ala Ile Val Ala His Gln Leu Ala Met Ala Gly Lys
            20                  25                  30
Ala Val Ile Leu Leu Glu Ala Gly Pro Arg Met Pro Arg Trp Glu Ile
        35                  40                  45
Val Glu Arg Phe Arg Asn Gln Pro Asp Lys Met Asp Phe Met Ala Pro
    50                  55                  60
Tyr Pro Ser Ser Pro Trp Ala Pro His Pro Glu Tyr Gly Pro Pro Asn
65                  70                  75                  80
Asp Tyr Leu Ile Leu Lys Gly Glu His Lys Phe Asn Ser Gln Tyr Ile
                85                  90                  95
Arg Ala Val Gly Gly Thr Thr Trp His Trp Ala Ala Ser Ala Trp Arg
            100                 105                 110
Phe Ile Pro Asn Asp Phe Lys Met Lys Ser Val Tyr Gly Val Gly Arg
        115                 120                 125
Asp Trp Pro Ile Gln Tyr Asp Asp Leu Glu Pro Tyr Tyr Gln Arg Ala
    130                 135                 140
Glu Glu Glu Leu Gly Val Trp Gly Pro Gly Pro Glu Glu Asp Leu Tyr

145                 150                 155                 160
Ser Pro Arg Lys Gln Pro Tyr Pro Met Pro Pro Leu Pro Leu Ser Phe
                165                 170                 175
Asn Glu Gln Thr Ile Lys Thr Ala Leu Asn Asn Tyr Asp Pro Lys Phe
            180                 185                 190
His Val Val Thr Glu Pro Val Ala Arg Asn Ser Arg Pro Tyr Asp Gly
        195                 200                 205
Arg Pro Thr Cys Cys Gly Asn Asn Asn Cys Met Pro Ile Cys Pro Ile
    210                 215                 220
Gly Ala Met Tyr Asn Gly Ile Val His Val Glu Lys Ala Glu Arg Ala
225                 230                 235                 240
Gly Ala Lys Leu Ile Glu Asn Ala Val Val Tyr Lys Leu Glu Thr Gly
                245                 250                 255
Pro Asp Lys Arg Ile Val Ala Ala Leu Tyr Lys Asp Lys Thr Gly Ala
            260                 265                 270
Glu His Arg Val Glu Gly Lys Tyr Phe Val Leu Ala Ala Asn Gly Ile
        275                 280                 285
Glu Thr Pro Lys Ile Leu Leu Met Ser Ala Asn Arg Asp Phe Pro Asn
    290                 295                 300
Gly Val Ala Asn Ser Ser Asp Met Val Gly Arg Asn Leu Met Asp His
305                 310                 315                 320
Pro Gly Thr Gly Val Ser Phe Tyr Ala Ser Glu Lys Leu Trp Pro Gly
                325                 330                 335
Arg Gly Pro Gln Glu Met Thr Ser Leu Ile Gly Phe Arg Asp Gly Pro
            340                 345                 350
Phe Arg Ala Thr Glu Ala Ala Lys Lys Ile His Leu Ser Asn Leu Ser
        355                 360                 365
Arg Ile Asp Gln Glu Thr Gln Lys Ile Phe Lys Ala Gly Lys Leu Met
    370                 375                 380
Lys Pro Asp Glu Leu Asp Ala Gln Ile Arg Asp Arg Ser Ala Arg Tyr
385                 390                 395                 400
Val Gln Phe Asp Cys Phe His Glu Ile Leu Pro Gln Pro Glu Asn Arg
                405                 410                 415
Ile Val Pro Ser Lys Thr Ala Thr Asp Ala Ile Gly Ile Pro Arg Pro
            420                 425                 430
Glu Ile Thr Tyr Ala Ile Asp Asp Tyr Val Lys Arg Gly Ala Ala His
        435                 440                 445
Thr Arg Glu Val Tyr Ala Thr Ala Ala Lys Val Leu Gly Gly Thr Asp
    450                 455                 460
Val Val Phe Asn Asp Glu Phe Ala Pro Asn Asn His Ile Thr Gly Ser
465                 470                 475                 480
Thr Ile Met Gly Ala Asp Ala Arg Asp Ser Val Val Asp Lys Asp Cys
                485                 490                 495
Arg Thr Phe Asp His Pro Asn Leu Phe Ile Ser Ser Ser Ala Thr Met
            500                 505                 510
Pro Thr Val Gly Thr Val Asn Val Thr Leu Thr Ile Ala Ala Leu Ala
        515                 520                 525
Leu Arg Met Ser Asp Thr Leu Lys Lys Glu Val
    530                 535

<210> 4
<211> 425
<212> PRT
<213> Burkholderia cepacia

<400> 4
Met Arg Lys Ser Thr Leu Thr Phe Leu Ile Ala Gly Cys Leu Ala Leu
1               5                   10                  15
Pro Gly Phe Ala Arg Ala Ala Asp Ala Ala Asp Pro Ala Leu Val Lys
            20                  25                  30
Arg Gly Glu Tyr Leu Ala Thr Ala Gly Asp Cys Met Ala Cys His Thr
        35                  40                  45

Val Lys Gly Gly Lys Pro Tyr Ala Gly Gly Leu Gly Met Pro Val Pro
    50                  55                  60
Met Leu Gly Lys Ile Tyr Thr Ser Asn Ile Thr Pro Asp Pro Asp Thr
65                  70                  75                  80
Gly Ile Gly Lys Trp Thr Phe Glu Asp Phe Glu Arg Ala Val Arg His
                85                  90                  95
Gly Val Ser Lys Asn Gly Asp Asn Leu Tyr Pro Ala Met Pro Tyr Val
            100                 105                 110
Ser Tyr Ala Lys Ile Thr Asp Asp Asp Val Arg Ala Leu Tyr Ala Tyr
        115                 120                 125
Phe Met His Gly Val Glu Pro Val Lys Gln Ala Pro Pro Lys Asn Glu
    130                 135                 140
Ile Pro Ala Leu Leu Ser Met Arg Trp Pro Leu Lys Ile Trp Asn Trp
145                 150                 155                 160
Leu Phe Leu Lys Asp Gly Pro Tyr Gln Pro Lys Pro Ser Gln Ser Ala
                165                 170                 175
Glu Trp Asn Arg Gly Ala Tyr Leu Val Gln Gly Leu Ala His Cys Ser
            180                 185                 190
Thr Cys His Thr Pro Arg Gly Ile Ala Met Gln Glu Lys Ser Leu Asp
        195                 200                 205
Glu Thr Gly Gly Ser Phe Leu Ala Gly Ser Val Leu Ala Gly Trp Asp
    210                 215                 220
Gly Tyr Asn Ile Thr Ser Asp Pro Asn Ala Gly Ile Gly Ser Trp Thr
225                 230                 235                 240
Gln Gln Gln Leu Val Gln Tyr Leu Arg Thr Gly Ser Val Pro Gly Val
                245                 250                 255
Ala Gln Ala Ala Gly Pro Met Ala Glu Ala Val Glu His Ser Phe Ser
            260                 265                 270
Lys Met Thr Glu Ala Asp Ile Gly Ala Ile Ala Thr Tyr Val Arg Thr
        275                 280                 285
Val Pro Ala Val Ala Asp Ser Asn Ala Lys Gln Pro Arg Ser Ser Trp
    290                 295                 300
Gly Lys Pro Ala Glu Asp Gly Leu Lys Leu Arg Gly Val Ala Leu Ala
305                 310                 315                 320
Ser Ser Gly Ile Asp Pro Ala Arg Leu Tyr Leu Gly Asn Cys Ala Thr
                325                 330                 335
Cys His Gln Met Gln Gly Lys Gly Thr Pro Asp Gly Tyr Tyr Pro Ser
            340                 345                 350
Leu Phe His Asn Ser Thr Val Gly Ala Ser Asn Pro Ser Asn Leu Val
        355                 360                 365
Gln Val Ile Leu Asn Gly Val Gln Arg Lys Ile Gly Ser Glu Asp Ile
    370                 375                 380
Gly Met Pro Ala Phe Arg Tyr Asp Leu Asn Asp Ala Gln Ile Ala Ala
385                 390                 395                 400
Leu Thr Asn Tyr Val Thr Ala Gln Phe Gly Asn Pro Ala Ala Lys Val
                405                 410                 415
Thr Glu Gln Asp Val Ala Lys Leu Arg
            420                 425

<210>  5
<211>  3399
<212>  DNA
<213>  Ewingella americana

<220>
<221>  CDS
<222>  (1)..(507)

<220>
<221>  CDS
<222>  (521)..(2131)

<220>
<221>  CDS
<222>  (2137)..(3396)

<400>  5
atg tct gat acc cct tcg aag acg ctt cgc gtc agc ggc gtt tca cgt      48
Met Ser Asp Thr Pro Ser Lys Thr Leu Arg Val Ser Gly Val Ser Arg
1               5                   10                  15
cgt cgc tta ttg ggt tat atc ggc gtc gga ctg gcc agc agc ctg atg      96
Arg Arg Leu Leu Gly Tyr Ile Gly Val Gly Leu Ala Ser Ser Leu Met
            20                  25                  30
atg ccc gtt ccg ctt aaa gcg ttt gcc gcc gac gga gaa cct tcg ccg      144
Met Pro Val Pro Leu Lys Ala Phe Ala Ala Asp Gly Glu Pro Ser Pro
        35                  40                  45
gtt gat gtc gat aaa ttc ttg cgc att tcc cgc gcg cta act ggc aaa      192
Val Asp Val Asp Lys Phe Leu Arg Ile Ser Arg Ala Leu Thr Gly Lys
    50                  55                  60
aag cac ctc aac ccg gtg att ggt caa cgc ctg tat gct gcg att ttg      240
Lys His Leu Asn Pro Val Ile Gly Gln Arg Leu Tyr Ala Ala Ile Leu
65                  70                  75                  80
cag cgc aaa cct gac atc gcc gcg cag ttg gac tca cta caa acc ctg      288
Gln Arg Lys Pro Asp Ile Ala Ala Gln Leu Asp Ser Leu Gln Thr Leu
                85                  90                  95
cct gcc ggt gcg ccg tcg agc tgg tct cag cct cag cag gac gcc gcg      336
Pro Ala Gly Ala Pro Ser Ser Trp Ser Gln Pro Gln Gln Asp Ala Ala
            100                 105                 110
cgc atc atc ctg caa ggc tgg tat ttg ggt cag gtg ggt gag ggg caa      384
Arg Ile Ile Leu Gln Gly Trp Tyr Leu Gly Gln Val Gly Glu Gly Gln
        115                 120                 125
gac att gca gtc gtg act tat gaa aaa gcg ctg atg ttt gag gcg gtc      432
Asp Ile Ala Val Val Thr Tyr Glu Lys Ala Leu Met Phe Glu Ala Val
    130                 135                 140
tcc gat gtg ctg gtg atc cgc tct tac tgt acc aat cgt cct ggc tac      480
Ser Asp Val Leu Val Ile Arg Ser Tyr Cys Thr Asn Arg Pro Gly Tyr
145                 150                 155                 160
tgg gct gcc aag ccc gat gtc gcg ctt taattaagga aaa atg aac atg      529
Trp Ala Ala Lys Pro Asp Val Ala Leu                Met Asn Met
                165                                     170
gct gac aca tta aaa gct gac gtc gtt gtc att ggc tca ggc gtt gcc      577
Ala Asp Thr Leu Lys Ala Asp Val Val Val Ile Gly Ser Gly Val Ala
        175                 180                 185
ggt ggt tta gtg gca aac gaa ctg gcg aag gcg ggg aaa aac gtc tta      625
Gly Gly Leu Val Ala Asn Glu Leu Ala Lys Ala Gly Lys Asn Val Leu
    190                 195                 200
att ttg gaa gca ggc ccg cgc ctg tct cgc tgg gaa atc gtc gag aat      673
Ile Leu Glu Ala Gly Pro Arg Leu Ser Arg Trp Glu Ile Val Glu Asn
205                 210                 215                 220
ttc cgc aat cag gcc gac aag tcg gac aac atg aag ccg tac ccg tct      721
Phe Arg Asn Gln Ala Asp Lys Ser Asp Asn Met Lys Pro Tyr Pro Ser
                225                 230                 235
acc gac tac gcg ccg cat cct gaa gcg aac cca agc aat aac tat ctg      769
Thr Asp Tyr Ala Pro His Pro Glu Ala Asn Pro Ser Asn Asn Tyr Leu
            240                 245                 250
att cag aag ggt gag cac ccg tac gac gcc cag ttt atc cgc gtg gtg      817
Ile Gln Lys Gly Glu His Pro Tyr Asp Ala Gln Phe Ile Arg Val Val
        255                 260                 265
ggt ggc aca acc tgg cac tgg gcg gcc tcc gcg tgg cgc ttc ctg ccg      865
Gly Gly Thr Thr Trp His Trp Ala Ala Ser Ala Trp Arg Phe Leu Pro
    270                 275                 280
aac gac ttc aag att aaa tct ctg tac ggc gta ggc cgc gac tgg ccg      913
Asn Asp Phe Lys Ile Lys Ser Leu Tyr Gly Val Gly Arg Asp Trp Pro
285                 290                 295                 300
atg gag tac ggc gag ctg gag aaa tgg tac ttc cgc gcc gag caa gag      961

Met Glu Tyr Gly Glu Leu Glu Lys Trp Tyr Phe Arg Ala Glu Gln Glu
                305                 310                 315
ctg ggc gtg tgg ggg cca aac aat gaa gag ctg ggt tcg ccg cgc agt      1009
Leu Gly Val Trp Gly Pro Asn Asn Glu Glu Leu Gly Ser Pro Arg Ser
            320                 325                 330
gcg cca tac ccg atg gaa cct ctg ccg ctc tct tat aac gaa cag cgc      1057
Ala Pro Tyr Pro Met Glu Pro Leu Pro Leu Ser Tyr Asn Glu Gln Arg
        335                 340                 345
ctg aaa acc gtg ctc aac aac aac ggt tac tac gtg gtg acc gag ccg      1105
Leu Lys Thr Val Leu Asn Asn Asn Gly Tyr Tyr Val Val Thr Glu Pro
    350                 355                 360
gtg gcg cgt aac agc cgc cct tat gat ggc cgc ccg acg tgc tgt ggt      1153
Val Ala Arg Asn Ser Arg Pro Tyr Asp Gly Arg Pro Thr Cys Cys Gly
365                 370                 375                 380
aac aac aac tgc atg ccg att tgc ccg att ggc gcg atg tac aac ggc      1201
Asn Asn Asn Cys Met Pro Ile Cys Pro Ile Gly Ala Met Tyr Asn Gly
                385                 390                 395
atc acg cac gtt gag aaa gct gag aaa aac ggc gcc gtg ctg cgc cct      1249
Ile Thr His Val Glu Lys Ala Glu Lys Asn Gly Ala Val Leu Arg Pro
            400                 405                 410
gag tcg gtg gtg tac aag ctg gaa gtc aac gac aag cag gag atc acc      1297
Glu Ser Val Val Tyr Lys Leu Glu Val Asn Asp Lys Gln Glu Ile Thr
        415                 420                 425
gcc gcg ctg ttc aaa gac ccg cag ggc aat gag cac cgc gcc gaa gcc      1345
Ala Ala Leu Phe Lys Asp Pro Gln Gly Asn Glu His Arg Ala Glu Ala
    430                 435                 440
aaa tac ttc gtg ctg gcg gcg aac ggc att gaa atc cct aaa ctg atg      1393
Lys Tyr Phe Val Leu Ala Ala Asn Gly Ile Glu Ile Pro Lys Leu Met
445                 450                 455                 460
ctg atg tcc acc aac gac aag aac cct aac ggc gtg ggt aac agc tct      1441
Leu Met Ser Thr Asn Asp Lys Asn Pro Asn Gly Val Gly Asn Ser Ser
                465                 470                 475
gac caa gtg ggc cgt aac ttg atg gac cat ccg ggc gtc ggc gtt gag      1489
Asp Gln Val Gly Arg Asn Leu Met Asp His Pro Gly Val Gly Val Glu
            480                 485                 490
ttc tac gcc aac gag ccg ctg tgg ccg ggg cgc ggg ccg cag gag atg      1537
Phe Tyr Ala Asn Glu Pro Leu Trp Pro Gly Arg Gly Pro Gln Glu Met
        495                 500                 505
acc tct atg gtt ggc ttc cgc gac ggc gat ttc cgt aaa gag tac gcc      1585
Thr Ser Met Val Gly Phe Arg Asp Gly Asp Phe Arg Lys Glu Tyr Ala
    510                 515                 520
gcg aag aaa atc cac ctg tcg aac gtt tcc cgt acc gat cag gaa gca      1633
Ala Lys Lys Ile His Leu Ser Asn Val Ser Arg Thr Asp Gln Glu Ala
525                 530                 535                 540
ggg cgt ctg ctc agc caa aaa ggt gag ttg atc ctt ggg cca gac ctc      1681
Gly Arg Leu Leu Ser Gln Lys Gly Glu Leu Ile Leu Gly Pro Asp Leu
                545                 550                 555
gac aag cgc att cgt gac cgt tcg tcg cgc tac gtg cgt ttt gac agc      1729
Asp Lys Arg Ile Arg Asp Arg Ser Ser Arg Tyr Val Arg Phe Asp Ser
            560                 565                 570
ttc cac gag atc ttg ccg cac gcg gaa aac cgc atc att ccg agt acc      1777
Phe His Glu Ile Leu Pro His Ala Glu Asn Arg Ile Ile Pro Ser Thr
        575                 580                 585
acg gag aaa gac aaa ctg ggc att ccg aag ccg gag ttc tac tac gcg      1825
Thr Glu Lys Asp Lys Leu Gly Ile Pro Lys Pro Glu Phe Tyr Tyr Ala
    590                 595                 600
atg gat gac tac gtg cgt aaa agt gcc gag cac act tac aaa gtc tat      1873
Met Asp Asp Tyr Val Arg Lys Ser Ala Glu His Thr Tyr Lys Val Tyr
605                 610                 615                 620
gcc gat gcg gct cgc ctg atg ggc ggc acc gac atc act tac ttc aaa      1921
Ala Asp Ala Ala Arg Leu Met Gly Gly Thr Asp Ile Thr Tyr Phe Lys
                625                 630                 635
gac ttc gcc aac aac aac cac att acc ggc acc acc atc atg ggc cac      1969

Asp Phe Ala Asn Asn Asn His Ile Thr Gly Thr Thr Ile Met Gly His
        640                 645                 650

gac ccg aaa gac tcg gtg gta gat acc gat tgc cgt act cac gac cac        2017
Asp Pro Lys Asp Ser Val Val Asp Thr Asp Cys Arg Thr His Asp His
        655                 660                 665

aaa aac ctg ttt atc gcc agc agc gcg gtc atg ccg act gtc ggc tcg        2065
Lys Asn Leu Phe Ile Ala Ser Ser Ala Val Met Pro Thr Val Gly Ser
    670                 675                 680

gta aac tgt act ttg act atc gcg gcg ctc tcg ctg cgg att gca gac        2113
Val Asn Cys Thr Leu Thr Ile Ala Ala Leu Ser Leu Arg Ile Ala Asp
685                 690                 695                 700

acg tta aag gcg gag gtg taacc atg aaa aag tta acc ctt agc ctg atg        2163
Thr Leu Lys Ala Glu Val       Met Lys Lys Leu Thr Leu Ser Leu Met
                705                       710                 715

gct gct gtt ggt ctg ttt agc ttg gcg gcg cag gcc gaa gag tct ggc        2211
Ala Ala Val Gly Leu Phe Ser Leu Ala Ala Gln Ala Glu Glu Ser Gly
                720                 725                 730

gtg gac ctg att aag cgc ggt gaa tac ctg acg cag gcc gcg gac tgc        2259
Val Asp Leu Ile Lys Arg Gly Glu Tyr Leu Thr Gln Ala Ala Asp Cys
            735                 740                 745

gtg gcg tgt cac acc act aaa gac ggc aaa cct ttt gcc ggc gga ctg        2307
Val Ala Cys His Thr Thr Lys Asp Gly Lys Pro Phe Ala Gly Gly Leu
        750                 755                 760

gct ttt aag acg cca atg ggc acg ctc tac tcg cca aac atc acg gcg        2355
Ala Phe Lys Thr Pro Met Gly Thr Leu Tyr Ser Pro Asn Ile Thr Ala
    765                 770                 775

gat aaa gag acg ggc att ggc act tgg agc gac gag gac ttc ctg cgc        2403
Asp Lys Glu Thr Gly Ile Gly Thr Trp Ser Asp Glu Asp Phe Leu Arg
780                 785                 790                 795

gcg gtt cac gaa ggt aag aac aaa gag ggc cag cac ctc tat ccg gcc        2451
Ala Val His Glu Gly Lys Asn Lys Glu Gly Gln His Leu Tyr Pro Ala
                800                 805                 810

ttc ccg tat acc tct tac acg ctg ttg aaa gat gac gac gtg aag gct        2499
Phe Pro Tyr Thr Ser Tyr Thr Leu Leu Lys Asp Asp Asp Val Lys Ala
            815                 820                 825

atc aag gct tac ttg ttc agc ctg ccg ccg gtg cat cag caa aat cgt        2547
Ile Lys Ala Tyr Leu Phe Ser Leu Pro Pro Val His Gln Gln Asn Arg
        830                 835                 840

gaa aac gag atg ccg ttc ccg ttc aac cag cgc tgg ggc atg tgg ttc        2595
Glu Asn Glu Met Pro Phe Pro Phe Asn Gln Arg Trp Gly Met Trp Phe
    845                 850                 855

tgg aat ctg gtg aat ttc gac agc aag cgt ttt gag cca gac acc gcg        2643
Trp Asn Leu Val Asn Phe Asp Ser Lys Arg Phe Glu Pro Asp Thr Ala
860                 865                 870                 875

cag tct gcc gag tgg aat cag ggt aaa tat ctg gtc gaa ggc tta ggc        2691
Gln Ser Ala Glu Trp Asn Gln Gly Lys Tyr Leu Val Glu Gly Leu Gly
                880                 885                 890

cac tgc ggc gag tgc cat acc ccg cgt aac atc acg atg ggg atg aaa        2739
His Cys Gly Glu Cys His Thr Pro Arg Asn Ile Thr Met Gly Met Lys
            895                 900                 905

gac ggc aag ccg tac gcc gga acc gag gtt gac ggc tgg acc gcg ttt        2787
Asp Gly Lys Pro Tyr Ala Gly Thr Glu Val Asp Gly Trp Thr Ala Phe
        910                 915                 920

aac atc tct tcc gac aag aaa gcc ggg att ggt agc tgg agc aaa ggc        2835
Asn Ile Ser Ser Asp Lys Lys Ala Gly Ile Gly Ser Trp Ser Lys Gly
    925                 930                 935

gac atc gtg cag tat ctg cgt tct ggt cac gtc agt ggc aaa gcg cag        2883
Asp Ile Val Gln Tyr Leu Arg Ser Gly His Val Ser Gly Lys Ala Gln
940                 945                 950                 955

gcc gct ggc ccg atg gcg gaa gtt atc gag aac agt acc cgc cat ctg        2931
Ala Ala Gly Pro Met Ala Glu Val Ile Glu Asn Ser Thr Arg His Leu
                960                 965                 970

acc gat gcc gac ctg aac gcg atg gcg acc tac atc cag acg ctg aag        2979

Thr Asp Ala Asp Leu Asn Ala Met Ala Thr Tyr Ile Gln Thr Leu Lys
            975                 980                 985
ccg cag gca gat tct tcc gtc acc aag agc cgt tct gat tgg ggc cat      3027
Pro Gln Ala Asp Ser Ser Val Thr Lys Ser Arg Ser Asp Trp Gly His
        990                 995                 1000
gag agc acc agc gta gac aac ctg cgc ggc gtg cca ttc aaa gcc      3072
Glu Ser Thr Ser Val Asp Asn Leu Arg Gly Val Pro Phe Lys Ala
    1005                1010                1015
agc caa gaa acc gac ggc gcg cgt ttg ttc ctc ggc aac tgc gca      3117
Ser Gln Glu Thr Asp Gly Ala Arg Leu Phe Leu Gly Asn Cys Ala
    1020                1025                1030
agc tgc cac agc ttc ggc ggt cag ggc agc caa gac ggt tac tac      3162
Ser Cys His Ser Phe Gly Gly Gln Gly Ser Gln Asp Gly Tyr Tyr
    1035                1040                1045
cca tcg ctg atg aaa aac agc gtg gtg ggt gcc cag aca cca aac      3207
Pro Ser Leu Met Lys Asn Ser Val Val Gly Ala Gln Thr Pro Asn
    1050                1055                1060
aac ttg gtc aac gtt atc ctg cac ggc gta tcg cgc aag acc aac      3252
Asn Leu Val Asn Val Ile Leu His Gly Val Ser Arg Lys Thr Asn
    1065                1070                1075
gac ggc gaa gtg ttc atg ccg ggc ttt gcc gac acc ctg agc gac      3297
Asp Gly Glu Val Phe Met Pro Gly Phe Ala Asp Thr Leu Ser Asp
    1080                1085                1090
aag cag atc gcc acg ctg agc acc tat atg ttt aaa gag ttt ggc      3342
Lys Gln Ile Ala Thr Leu Ser Thr Tyr Met Phe Lys Glu Phe Gly
    1095                1100                1105
gcc cag ccg gtg ttg aat gtg aaa gat aaa gat gtg aag gat ttg      3387
Ala Gln Pro Val Leu Asn Val Lys Asp Lys Asp Val Lys Asp Leu
    1110                1115                1120
cgc ggg gag tga                                                  3399
Arg Gly Glu
    1125

<210>  6
<211>  169
<212>  PRT
<213>  Ewingella americana

<400>  6
Met Ser Asp Thr Pro Ser Lys Thr Leu Arg Val Ser Gly Val Ser Arg
1               5                   10                  15
Arg Arg Leu Leu Gly Tyr Ile Gly Val Gly Leu Ala Ser Ser Leu Met
            20                  25                  30
Met Pro Val Pro Leu Lys Ala Phe Ala Ala Asp Gly Glu Pro Ser Pro
        35                  40                  45
Val Asp Val Asp Lys Phe Leu Arg Ile Ser Arg Ala Leu Thr Gly Lys
    50                  55                  60
Lys His Leu Asn Pro Val Ile Gly Gln Arg Leu Tyr Ala Ala Ile Leu
65                  70                  75                  80
Gln Arg Lys Pro Asp Ile Ala Ala Gln Leu Asp Ser Leu Gln Thr Leu
                85                  90                  95
Pro Ala Gly Ala Pro Ser Ser Trp Ser Gln Pro Gln Gln Asp Ala Ala
            100                 105                 110
Arg Ile Ile Leu Gln Gly Trp Tyr Leu Gly Gln Val Gly Glu Gly Gln
        115                 120                 125
Asp Ile Ala Val Val Thr Tyr Glu Lys Ala Leu Met Phe Glu Ala Val
    130                 135                 140
Ser Asp Val Leu Val Ile Arg Ser Tyr Cys Thr Asn Arg Pro Gly Tyr
145                 150                 155                 160
Trp Ala Ala Lys Pro Asp Val Ala Leu
                165

<210>  7
<211>  537
<212>  PRT
<213>  Ewingella americana

<400>  7
Met Asn Met Ala Asp Thr Leu Lys Ala Asp Val Val Val Ile Gly Ser
1               5                   10                  15
Gly Val Ala Gly Gly Leu Val Ala Asn Glu Leu Ala Lys Ala Gly Lys
            20                  25                  30
Asn Val Leu Ile Leu Glu Ala Gly Pro Arg Leu Ser Arg Trp Glu Ile
        35                  40                  45
Val Glu Asn Phe Arg Asn Gln Ala Asp Lys Ser Asp Asn Met Lys Pro
    50                  55                  60
Tyr Pro Ser Thr Asp Tyr Ala Pro His Pro Glu Ala Asn Pro Ser Asn
65                  70                  75                  80
Asn Tyr Leu Ile Gln Lys Gly Glu His Pro Tyr Asp Ala Gln Phe Ile
                85                  90                  95
Arg Val Val Gly Gly Thr Thr Trp His Trp Ala Ala Ser Ala Trp Arg
            100                 105                 110
Phe Leu Pro Asn Asp Phe Lys Ile Lys Ser Leu Tyr Gly Val Gly Arg
        115                 120                 125
Asp Trp Pro Met Glu Tyr Gly Glu Leu Glu Lys Trp Tyr Phe Arg Ala
    130                 135                 140
Glu Gln Glu Leu Gly Val Trp Gly Pro Asn Asn Glu Glu Leu Gly Ser
145                 150                 155                 160
Pro Arg Ser Ala Pro Tyr Pro Met Glu Pro Leu Pro Leu Ser Tyr Asn
                165                 170                 175
Glu Gln Arg Leu Lys Thr Val Leu Asn Asn Asn Gly Tyr Tyr Val Val
            180                 185                 190
Thr Glu Pro Val Ala Arg Asn Ser Arg Pro Tyr Asp Gly Arg Pro Thr
        195                 200                 205
Cys Cys Gly Asn Asn Asn Cys Met Pro Ile Cys Pro Ile Gly Ala Met
    210                 215                 220
Tyr Asn Gly Ile Thr His Val Glu Lys Ala Glu Lys Asn Gly Ala Val
225                 230                 235                 240
Leu Arg Pro Glu Ser Val Val Tyr Lys Leu Glu Val Asn Asp Lys Gln
                245                 250                 255
Glu Ile Thr Ala Ala Leu Phe Lys Asp Pro Gln Gly Asn Glu His Arg
            260                 265                 270
Ala Glu Ala Lys Tyr Phe Val Leu Ala Ala Asn Gly Ile Glu Ile Pro
        275                 280                 285
Lys Leu Met Leu Met Ser Thr Asn Asp Lys Asn Pro Asn Gly Val Gly
    290                 295                 300
Asn Ser Ser Asp Gln Val Gly Arg Asn Leu Met Asp His Pro Gly Val
305                 310                 315                 320
Gly Val Glu Phe Tyr Ala Asn Glu Pro Leu Trp Pro Gly Arg Gly Pro
                325                 330                 335
Gln Glu Met Thr Ser Met Val Gly Phe Arg Asp Gly Asp Phe Arg Lys
            340                 345                 350
Glu Tyr Ala Ala Lys Lys Ile His Leu Ser Asn Val Ser Arg Thr Asp
        355                 360                 365
Gln Glu Ala Gly Arg Leu Leu Ser Gln Lys Gly Glu Leu Ile Leu Gly
    370                 375                 380
Pro Asp Leu Asp Lys Arg Ile Arg Asp Arg Ser Ser Arg Tyr Val Arg
385                 390                 395                 400
Phe Asp Ser Phe His Glu Ile Leu Pro His Ala Glu Asn Arg Ile Ile
                405                 410                 415
Pro Ser Thr Thr Glu Lys Asp Lys Leu Gly Ile Pro Lys Pro Glu Phe
            420                 425                 430
Tyr Tyr Ala Met Asp Asp Tyr Val Arg Lys Ser Ala Glu His Thr Tyr
        435                 440                 445

Lys Val Tyr Ala Asp Ala Ala Arg Leu Met Gly Gly Thr Asp Ile Thr
    450                 455                 460
Tyr Phe Lys Asp Phe Ala Asn Asn Asn His Ile Thr Gly Thr Thr Ile
465                 470                 475                 480
Met Gly His Asp Pro Lys Asp Ser Val Val Asp Thr Asp Cys Arg Thr
                485                 490                 495
His Asp His Lys Asn Leu Phe Ile Ala Ser Ser Ala Val Met Pro Thr
            500                 505                 510
Val Gly Ser Val Asn Cys Thr Leu Thr Ile Ala Ala Leu Ser Leu Arg
        515                 520                 525
Ile Ala Asp Thr Leu Lys Ala Glu Val
    530                 535

<210>  8
<211>  420
<212>  PRT
<213>  Ewingella americana

<400>  8
Met Lys Lys Leu Thr Leu Ser Leu Met Ala Ala Val Gly Leu Phe Ser
1               5                   10                  15
Leu Ala Ala Gln Ala Glu Glu Ser Gly Val Asp Leu Ile Lys Arg Gly
            20                  25                  30
Glu Tyr Leu Thr Gln Ala Ala Asp Cys Val Ala Cys His Thr Thr Lys
        35                  40                  45
Asp Gly Lys Pro Phe Ala Gly Gly Leu Ala Phe Lys Thr Pro Met Gly
    50                  55                  60
Thr Leu Tyr Ser Pro Asn Ile Thr Ala Asp Lys Glu Thr Gly Ile Gly
65                  70                  75                  80
Thr Trp Ser Asp Glu Asp Phe Leu Arg Ala Val His Glu Gly Lys Asn
                85                  90                  95
Lys Glu Gly Gln His Leu Tyr Pro Ala Phe Pro Tyr Thr Ser Tyr Thr
            100                 105                 110
Leu Leu Lys Asp Asp Asp Val Lys Ala Ile Lys Ala Tyr Leu Phe Ser
        115                 120                 125
Leu Pro Pro Val His Gln Gln Asn Arg Glu Asn Glu Met Pro Phe Pro
    130                 135                 140
Phe Asn Gln Arg Trp Gly Met Trp Phe Trp Asn Leu Val Asn Phe Asp
145                 150                 155                 160
Ser Lys Arg Phe Glu Pro Asp Thr Ala Gln Ser Ala Glu Trp Asn Gln
                165                 170                 175
Gly Lys Tyr Leu Val Glu Gly Leu Gly His Cys Gly Glu Cys His Thr
            180                 185                 190
Pro Arg Asn Ile Thr Met Gly Met Lys Asp Gly Lys Pro Tyr Ala Gly
        195                 200                 205
Thr Glu Val Asp Gly Trp Thr Ala Phe Asn Ile Ser Ser Asp Lys Lys
    210                 215                 220
Ala Gly Ile Gly Ser Trp Ser Lys Gly Asp Ile Val Gln Tyr Leu Arg
225                 230                 235                 240
Ser Gly His Val Ser Gly Lys Ala Gln Ala Ala Gly Pro Met Ala Glu
                245                 250                 255
Val Ile Glu Asn Ser Thr Arg His Leu Thr Asp Ala Asp Leu Asn Ala
            260                 265                 270
Met Ala Thr Tyr Ile Gln Thr Leu Lys Pro Gln Ala Asp Ser Ser Val
        275                 280                 285
Thr Lys Ser Arg Ser Asp Trp Gly His Glu Ser Thr Ser Val Asp Asn
    290                 295                 300
Leu Arg Gly Val Pro Phe Lys Ala Ser Gln Glu Thr Asp Gly Ala Arg
305                 310                 315                 320
Leu Phe Leu Gly Asn Cys Ala Ser Cys His Ser Phe Gly Gly Gln Gly
                325                 330                 335
Ser Gln Asp Gly Tyr Tyr Pro Ser Leu Met Lys Asn Ser Val Val Gly

340                     345                     350
Ala Gln Thr Pro Asn Asn Leu Val Asn Val Ile Leu His Gly Val Ser
        355                     360                     365
Arg Lys Thr Asn Asp Gly Glu Val Phe Met Pro Gly Phe Ala Asp Thr
    370                     375                     380
Leu Ser Asp Lys Gln Ile Ala Thr Leu Ser Thr Tyr Met Phe Lys Glu
385                     390                     395                     400
Phe Gly Ala Gln Pro Val Leu Asn Val Lys Asp Lys Asp Val Lys Asp
                405                     410                     415
Leu Arg Gly Glu
            420

<210>  9
<211>  26
<212>  DNA
<213>  artificial sequence

<220>
<223>  primer

<400>  9
catgccatgg cacacaacga caacac                                            26


<210>  10
<211>  30
<212>  DNA
<213>  artificial sequence

<220>
<223>  primer

<400>  10
gtcgacgatc ttcttccagc cgaacatcac                                        30


<210>  11
<211>  3638
<212>  DNA
<213>  Silvimonas terrae


<220>
<221>  CDS
<222>  (1)..(537)

<220>
<221>  CDS
<222>  (555)..(2174)

<220>
<221>  CDS
<222>  (2193)..(3635)

<400>  11
atg aat gat cgt gat tcg ccc att cac gcc gat gcg cta ctc ccc cag      48
Met Asn Asp Arg Asp Ser Pro Ile His Ala Asp Ala Leu Leu Pro Gln
1               5                   10                  15
ccc acc cgc cgc cgt ctg ttg ctg gca tct ggc ttg ctg ctg ggg acg      96
Pro Thr Arg Arg Arg Leu Leu Leu Ala Ser Gly Leu Leu Leu Gly Thr
            20                  25                  30
ctg gcc gtt tcc ggg cga ctg gtc tcg gtg gtc ttt gcc gat acg cct     144
Leu Ala Val Ser Gly Arg Leu Val Ser Val Val Phe Ala Asp Thr Pro

35                  40                  45
gcg caa gac agc cag ttt ttg cag ctc tcg caa tta ctg acc ggg cgg      192
Ala Gln Asp Ser Gln Phe Leu Gln Leu Ser Gln Leu Leu Thr Gly Arg
    50                  55                  60
gcg caa ctg gat acc gcc gtc gca cac cgt ttt tat tca gcg ctg agc      240
Ala Gln Leu Asp Thr Ala Val Ala His Arg Phe Tyr Ser Ala Leu Ser
65                  70                  75                  80
acc aaa gac aat cag ctg gac agc aag ctg cgg cag att gcg ggc gaa      288
Thr Lys Asp Asn Gln Leu Asp Ser Lys Leu Arg Gln Ile Ala Gly Glu
                85                  90                  95
gcg caa acc aac agc ctg acc aat gcc gaa gaa ttg ctc acc gcg ctg      336
Ala Gln Thr Asn Ser Leu Thr Asn Ala Glu Glu Leu Leu Thr Ala Leu
            100                 105                 110
gat gcc aac cag cca gaa ctg gcc aag acc gcg cgc aac atc att gcc      384
Asp Ala Asn Gln Pro Glu Leu Ala Lys Thr Ala Arg Asn Ile Ile Ala
        115                 120                 125
gcg tgg tat tcc ggt gtg gtg ggt gac ggc ccg gat gcc att gtg att      432
Ala Trp Tyr Ser Gly Val Val Gly Asp Gly Pro Asp Ala Ile Val Ile
    130                 135                 140
act tat gcc agc gcc ttg atg ttc gat ctg gtc aaa gac gcc acc atg      480
Thr Tyr Ala Ser Ala Leu Met Phe Asp Leu Val Lys Asp Ala Thr Met
145                 150                 155                 160
ccg ccc tct tac tgc cag gcc gca ccg ctg tac tgg tcg gcc aaa cca      528
Pro Pro Ser Tyr Cys Gln Ala Ala Pro Leu Tyr Trp Ser Ala Lys Pro
                165                 170                 175
ccg ctg aac tgacccggac cctcttc atg agc gca aac aat caa tct gcg      578
Pro Leu Asn                    Met Ser Ala Asn Asn Gln Ser Ala
                               180                 185
gat gtc att gtc att ggt tca ggg gta gcc ggt gcg ctg gtg gcg cat      626
Asp Val Ile Val Ile Gly Ser Gly Val Ala Gly Ala Leu Val Ala His
        190                 195                 200
gat ctc tct gcc gcc ggg gcg tca gtg ctg atg ctg gaa gcc ggc ccc      674
Asp Leu Ser Ala Ala Gly Ala Ser Val Leu Met Leu Glu Ala Gly Pro
    205                 210                 215
cgg ctg gaa cgc tgg cgc att gtc gaa aac tac cgc aat ggc gcc gcc      722
Arg Leu Glu Arg Trp Arg Ile Val Glu Asn Tyr Arg Asn Gly Ala Ala
220                 225                 230                 235
aaa gat gat aac cag cgc ccc tac ccg gcc gcc aga cac gcg ccg cac      770
Lys Asp Asp Asn Gln Arg Pro Tyr Pro Ala Ala Arg His Ala Pro His
                240                 245                 250
ccg gaa tac ggg ccc gac aac aac tat ctg atc ctc aaa ggc ccg gat      818
Pro Glu Tyr Gly Pro Asp Asn Asn Tyr Leu Ile Leu Lys Gly Pro Asp
            255                 260                 265
gcg cag tct tat gcc tcg caa tat ctg cgg gtc gtt ggc ggc acc acc      866
Ala Gln Ser Tyr Ala Ser Gln Tyr Leu Arg Val Val Gly Gly Thr Thr
        270                 275                 280
tgg cac tgg gcc gcg tcc tgt tgg cgc ttt ctg ccc aat gat ttt cat      914
Trp His Trp Ala Ala Ser Cys Trp Arg Phe Leu Pro Asn Asp Phe His
    285                 290                 295
atg aag cag gtc tac ggc gtg ggc cgg gac tgg ggg atc agc tat gcc      962
Met Lys Gln Val Tyr Gly Val Gly Arg Asp Trp Gly Ile Ser Tyr Ala
300                 305                 310                 315
gat ctg gaa ccc tgg tac tac aag gcc gag ctg gaa ctc ggg gtt tcc     1010
Asp Leu Glu Pro Trp Tyr Tyr Lys Ala Glu Leu Glu Leu Gly Val Ser
                320                 325                 330
ggg ccc aac gac ggc aca gac ctg ggc tca ccg cgc aag cag ccc tac     1058
Gly Pro Asn Asp Gly Thr Asp Leu Gly Ser Pro Arg Lys Gln Pro Tyr
            335                 340                 345
ccg atg gat cac ctg ccg ctg tct tac aac gac cag cgg ttc cac gat     1106
Pro Met Asp His Leu Pro Leu Ser Tyr Asn Asp Gln Arg Phe His Asp
        350                 355                 360
gtc ctc aac gcc aac ggg ttc aat ctg gtg gcc gaa ccg gtg gcc cgc     1154
Val Leu Asn Ala Asn Gly Phe Asn Leu Val Ala Glu Pro Val Ala Arg

365                 370                 375
aac agc cgg ccc tac gac gag cgc ccg acg tgc tgt ggc aac aac aac      1202
Asn Ser Arg Pro Tyr Asp Glu Arg Pro Thr Cys Cys Gly Asn Asn Asn
380                 385                 390                 395
tgc atg ccc atc tgc ccg att gcc gcc atg tac agc ggc atc atc cat      1250
Cys Met Pro Ile Cys Pro Ile Ala Ala Met Tyr Ser Gly Ile Ile His
                400                 405                 410
gtg ctc aaa gcc gaa cgc gcg ggt acg cga ttg ctg acc aat gcg gtg      1298
Val Leu Lys Ala Glu Arg Ala Gly Thr Arg Leu Leu Thr Asn Ala Val
            415                 420                 425
gtg tac cgg ctg gaa gta gac gct cac gac aac atc att gct gcg ctc      1346
Val Tyr Arg Leu Glu Val Asp Ala His Asp Asn Ile Ile Ala Ala Leu
        430                 435                 440
tac aaa gac ccg gac ggc aac agt cat cgg gtc acc ggc aag tat ttc      1394
Tyr Lys Asp Pro Asp Gly Asn Ser His Arg Val Thr Gly Lys Tyr Phe
    445                 450                 455
gtg ctg gcg gcc aat gcc ctg gaa tca gcc aaa atc ctg ttg atg tcg      1442
Val Leu Ala Ala Asn Ala Leu Glu Ser Ala Lys Ile Leu Leu Met Ser
460                 465                 470                 475
acc acc gac aaa ttt gcc agt ggc ctg gcc aac agt tca gac cag gta      1490
Thr Thr Asp Lys Phe Ala Ser Gly Leu Ala Asn Ser Ser Asp Gln Val
                480                 485                 490
ggc cgt aat ctg atg gat cac ccc ggc act ggc ata acc ttt ctg gcc      1538
Gly Arg Asn Leu Met Asp His Pro Gly Thr Gly Ile Thr Phe Leu Ala
            495                 500                 505
aat gag ccg ctc tgg ccg ggc cgc ggg ccg atg gag ctg acc tcg att      1586
Asn Glu Pro Leu Trp Pro Gly Arg Gly Pro Met Glu Leu Thr Ser Ile
        510                 515                 520
gtc gat cac cgc gac ggg gat ttc cgg cgg gag tgg gcc agc aag aag      1634
Val Asp His Arg Asp Gly Asp Phe Arg Arg Glu Trp Ala Ser Lys Lys
    525                 530                 535
ttg cac ctc aat aac cag tcc cag acg cgc aag gcc aca gaa aaa gcc      1682
Leu His Leu Asn Asn Gln Ser Gln Thr Arg Lys Ala Thr Glu Lys Ala
540                 545                 550                 555
ttg tcg atg ggg ctg gtg ggc aag aag ctg gat gaa gaa atc cgc tac      1730
Leu Ser Met Gly Leu Val Gly Lys Lys Leu Asp Glu Glu Ile Arg Tyr
                560                 565                 570
cgg gcc gcg cat acg gtc aac atc aac agt ttt cat gac att ttg ccg      1778
Arg Ala Ala His Thr Val Asn Ile Asn Ser Phe His Asp Ile Leu Pro
            575                 580                 585
gag ccg gac aac cgc ctc gtc ctg tca gac cag aaa gac ccg ctg ggc      1826
Glu Pro Asp Asn Arg Leu Val Leu Ser Asp Gln Lys Asp Pro Leu Gly
        590                 595                 600
ctg ccc cgc atg gaa atc acc tac aag att ggg gat tac acc aag cgc      1874
Leu Pro Arg Met Glu Ile Thr Tyr Lys Ile Gly Asp Tyr Thr Lys Arg
    605                 610                 615
agc gcc gaa cat acg cat gag tcc tac gca aaa att gcc cag ttg ttt      1922
Ser Ala Glu His Thr His Glu Ser Tyr Ala Lys Ile Ala Gln Leu Phe
620                 625                 630                 635
ggc ggg acc gag gtg acg ttt gat gac aaa ttt gcc ccc aac aat cac      1970
Gly Gly Thr Glu Val Thr Phe Asp Asp Lys Phe Ala Pro Asn Asn His
                640                 645                 650
ctg atg ggc tcg gtc atc atg gga caa aac ccc aaa gac tcg gtg gtc      2018
Leu Met Gly Ser Val Ile Met Gly Gln Asn Pro Lys Asp Ser Val Val
            655                 660                 665
gat ggc gat tgt cgc gcg cac gac cac gcc aat ctg tat ctg gct acc      2066
Asp Gly Asp Cys Arg Ala His Asp His Ala Asn Leu Tyr Leu Ala Thr
        670                 675                 680
agc ggg gtg atg gcg tcg gcc ggc agt gtg aac tgc acg ctg acc atc      2114
Ser Gly Val Met Ala Ser Ala Gly Ser Val Asn Cys Thr Leu Thr Ile
    685                 690                 695
gcc gcg ctg tcc ttg cgt att gcc gac acg ctc aag cgc cag ctt ggg      2162
Ala Ala Leu Ser Leu Arg Ile Ala Asp Thr Leu Lys Arg Gln Leu Gly

700                 705                 710                 715
cgc acc acc agt taagacggga gcgctacc atg acc tgc cgt ctt ctt cgc     2213
Arg Thr Thr Ser                     Met Thr Cys Arg Leu Leu Arg
                                    720                 725
ccc ttt gcc ctt gcg gcc ctg atg ctt gtt tct gcg ctg gcg cag gct     2261
Pro Phe Ala Leu Ala Ala Leu Met Leu Val Ser Ala Leu Ala Gln Ala
            730                 735                 740
gct gag gtc agc ccg gaa cag atc aag cgc ggc gag tat ctg gcc cgc     2309
Ala Glu Val Ser Pro Glu Gln Ile Lys Arg Gly Glu Tyr Leu Ala Arg
        745                 750                 755
gcc gcc gac tgt att gcc tgt cat acc gtg ccg cgt acc ggc cag ccg     2357
Ala Ala Asp Cys Ile Ala Cys His Thr Val Pro Arg Thr Gly Gln Pro
    760                 765                 770
ttt gcg ggt ggt tac ggc att gcc tcg ccg ttg ggc act att tat tcg     2405
Phe Ala Gly Gly Tyr Gly Ile Ala Ser Pro Leu Gly Thr Ile Tyr Ser
775                 780                 785                 790
tcc aac atc acg ccg tcc act tcg gcc ggg att ggc aac tac acg cta     2453
Ser Asn Ile Thr Pro Ser Thr Ser Ala Gly Ile Gly Asn Tyr Thr Leu
                795                 800                 805
caa gat ttc gac aac gcc ttg cgc cgg ggc atc cgc aaa gat ggc gcg     2501
Gln Asp Phe Asp Asn Ala Leu Arg Arg Gly Ile Arg Lys Asp Gly Ala
            810                 815                 820
cac ctc tac ccg gcc atg ccc tac acc gcc tac gcc aag ctg cag cca     2549
His Leu Tyr Pro Ala Met Pro Tyr Thr Ala Tyr Ala Lys Leu Gln Pro
        825                 830                 835
gaa gac gtc gag gcg ctc tat gcc tat ctg atg cat ggc gtg aaa ccg     2597
Glu Asp Val Glu Ala Leu Tyr Ala Tyr Leu Met His Gly Val Lys Pro
    840                 845                 850
gta gat gcc cag cca cgt cgc acg cgg ttg ccg ttt ccg ttc aac atc     2645
Val Asp Ala Gln Pro Arg Arg Thr Arg Leu Pro Phe Pro Phe Asn Ile
855                 860                 865                 870
cgg ctc tcc atg gcc gtc tgg aac agc ttg ttt ctg gac cca cgg gtt     2693
Arg Leu Ser Met Ala Val Trp Asn Ser Leu Phe Leu Asp Pro Arg Val
                875                 880                 885
ttc cag cca gaa caa ggc aag agt gag gac ttc aat cgc ggc aag tat     2741
Phe Gln Pro Glu Gln Gly Lys Ser Glu Asp Phe Asn Arg Gly Lys Tyr
            890                 895                 900
ctg gta gaa gca ctg gaa cat tgc agc gcc tgc cac acg ccc cgc aac     2789
Leu Val Glu Ala Leu Glu His Cys Ser Ala Cys His Thr Pro Arg Asn
        905                 910                 915
atg ctg atg gca gaa aac ccg gac gcg cca ttt gcc ggc ggg caa ctg     2837
Met Leu Met Ala Glu Asn Pro Asp Ala Pro Phe Ala Gly Gly Gln Leu
    920                 925                 930
ggt gca tgg tat gcg ccc aat atc acc gcc gac ccc gtc agc ggc gtt     2885
Gly Ala Trp Tyr Ala Pro Asn Ile Thr Ala Asp Pro Val Ser Gly Val
935                 940                 945                 950
ggc ggc tgg acc cag gcg gaa ctg gtg caa tac ctc aaa acc ggc cat     2933
Gly Gly Trp Thr Gln Ala Glu Leu Val Gln Tyr Leu Lys Thr Gly His
                955                 960                 965
gtc gcg ggc aaa gct gcg gcc gcc ggc ccg atg gcc gaa gcg gtc tct     2981
Val Ala Gly Lys Ala Ala Ala Ala Gly Pro Met Ala Glu Ala Val Ser
            970                 975                 980
gac agc tta agc cat ctg acc gac cca gac ctg cag gcc atg gcg acc     3029
Asp Ser Leu Ser His Leu Thr Asp Pro Asp Leu Gln Ala Met Ala Thr
        985                 990                 995
tac ctc  aaa acc atc gcc ccc  atc cgc aat acc gcc  gac agc aaa     3074
Tyr Leu  Lys Thr Ile Ala Pro  Ile Arg Asn Thr Ala  Asp Ser Lys
    1000                1005                1010
ccc gcc  tgg gca tgg ggt agc  ccg gcc aat gac gac  gat agc ttg     3119
Pro Ala  Trp Ala Trp Gly Ser  Pro Ala Asn Asp Asp  Asp Ser Leu
    1015                1020                1025
cgc ggt  acg gcg ggc gtc acc  caa agt aac cgc cca  acc agc gcg     3164
Arg Gly  Thr Ala Gly Val Thr  Gln Ser Asn Arg Pro  Thr Ser Ala

1030                1035                1040
gcc gaa ttg ttc agc gcc aat tgc gcc agt tgc cat caa gcc aat      3209
Ala Glu Leu Phe Ser Ala Asn Cys Ala Ser Cys His Gln Ala Asn
    1045                1050                1055
ggc atg ggc acg acc gac acc gcc tac cca tcg ctc acc aac aac      3254
Gly Met Gly Thr Thr Asp Thr Ala Tyr Pro Ser Leu Thr Asn Asn
    1060                1065                1070
acg gcg ttg gga cac ctt aac ccc agc aac ctg atc gcc gta atc      3299
Thr Ala Leu Gly His Leu Asn Pro Ser Asn Leu Ile Ala Val Ile
    1075                1080                1085
ctg aac ggg gta gac cgg cca ctg ccg gac ggc cac cac gcc ttc      3344
Leu Asn Gly Val Asp Arg Pro Leu Pro Asp Gly His His Ala Phe
    1090                1095                1100
atg cct ggt ttt ggc ccc ggc agc ctg atc aac ccc ttg tcg gat      3389
Met Pro Gly Phe Gly Pro Gly Ser Leu Ile Asn Pro Leu Ser Asp
    1105                1110                1115
gcg gat atc gcc acg ctg gcc aat tac acg ttg caa cgg ttc ggc      3434
Ala Asp Ile Ala Thr Leu Ala Asn Tyr Thr Leu Gln Arg Phe Gly
    1120                1125                1130
aac acc aac gtc cac gtc acg ccc gag gag gtc gct gcc tcg cga      3479
Asn Thr Asn Val His Val Thr Pro Glu Glu Val Ala Ala Ser Arg
    1135                1140                1145
aaa ggc ggt gag ccg cca ctg ctc gcc aaa ttg ccc cgt gta ttg      3524
Lys Gly Gly Glu Pro Pro Leu Leu Ala Lys Leu Pro Arg Val Leu
    1150                1155                1160
ccg gtc gtg ctg atg ctg ttt gcg ctg ttg ctg att ctg ggc ggc      3569
Pro Val Val Leu Met Leu Phe Ala Leu Leu Leu Ile Leu Gly Gly
    1165                1170                1175
tgg cgc agt gcg cga cag cgc aaa ttg cgc cat gta gcg cgc ccc      3614
Trp Arg Ser Ala Arg Gln Arg Lys Leu Arg His Val Ala Arg Pro
    1180                1185                1190
tct ccc acg cca cgc acg cct tga                                  3638
Ser Pro Thr Pro Arg Thr Pro
    1195                1200

<210>  12
<211>  179
<212>  PRT
<213>  Silvimonas terrae

<400>  12
Met Asn Asp Arg Asp Ser Pro Ile His Ala Asp Ala Leu Leu Pro Gln
1               5                   10                  15
Pro Thr Arg Arg Arg Leu Leu Leu Ala Ser Gly Leu Leu Leu Gly Thr
            20                  25                  30
Leu Ala Val Ser Gly Arg Leu Val Ser Val Val Phe Ala Asp Thr Pro
        35                  40                  45
Ala Gln Asp Ser Gln Phe Leu Gln Leu Ser Gln Leu Leu Thr Gly Arg
    50                  55                  60
Ala Gln Leu Asp Thr Ala Val Ala His Arg Phe Tyr Ser Ala Leu Ser
65                  70                  75                  80
Thr Lys Asp Asn Gln Leu Asp Ser Lys Leu Arg Gln Ile Ala Gly Glu
                85                  90                  95
Ala Gln Thr Asn Ser Leu Thr Asn Ala Glu Glu Leu Leu Thr Ala Leu
            100                 105                 110
Asp Ala Asn Gln Pro Glu Leu Ala Lys Thr Ala Arg Asn Ile Ile Ala
        115                 120                 125
Ala Trp Tyr Ser Gly Val Val Gly Asp Gly Pro Asp Ala Ile Val Ile
    130                 135                 140
Thr Tyr Ala Ser Ala Leu Met Phe Asp Leu Val Lys Asp Ala Thr Met
145                 150                 155                 160
Pro Pro Ser Tyr Cys Gln Ala Ala Pro Leu Tyr Trp Ser Ala Lys Pro

                    165                 170                 175
Pro Leu Asn

<210>  13
<211>  540
<212>  PRT
<213>  Silvimonas terrae

<400>  13
Met Ser Ala Asn Asn Gln Ser Ala Asp Val Ile Val Ile Gly Ser Gly
1               5                   10                  15
Val Ala Gly Ala Leu Val Ala His Asp Leu Ser Ala Ala Gly Ala Ser
            20                  25                  30
Val Leu Met Leu Glu Ala Gly Pro Arg Leu Glu Arg Trp Arg Ile Val
        35                  40                  45
Glu Asn Tyr Arg Asn Gly Ala Ala Lys Asp Asp Asn Gln Arg Pro Tyr
    50                  55                  60
Pro Ala Ala Arg His Ala Pro His Pro Glu Tyr Gly Pro Asp Asn Asn
65                  70                  75                  80
Tyr Leu Ile Leu Lys Gly Pro Asp Ala Gln Ser Tyr Ala Ser Gln Tyr
                85                  90                  95
Leu Arg Val Val Gly Gly Thr Thr Trp His Trp Ala Ala Ser Cys Trp
            100                 105                 110
Arg Phe Leu Pro Asn Asp Phe His Met Lys Gln Val Tyr Gly Val Gly
        115                 120                 125
Arg Asp Trp Gly Ile Ser Tyr Ala Asp Leu Glu Pro Trp Tyr Tyr Lys
    130                 135                 140
Ala Glu Leu Glu Leu Gly Val Ser Gly Pro Asn Asp Gly Thr Asp Leu
145                 150                 155                 160
Gly Ser Pro Arg Lys Gln Pro Tyr Pro Met Asp His Leu Pro Leu Ser
                165                 170                 175
Tyr Asn Asp Gln Arg Phe His Asp Val Leu Asn Ala Asn Gly Phe Asn
            180                 185                 190
Leu Val Ala Glu Pro Val Ala Arg Asn Ser Arg Pro Tyr Asp Glu Arg
        195                 200                 205
Pro Thr Cys Cys Gly Asn Asn Asn Cys Met Pro Ile Cys Pro Ile Ala
    210                 215                 220
Ala Met Tyr Ser Gly Ile Ile His Val Leu Lys Ala Glu Arg Ala Gly
225                 230                 235                 240
Thr Arg Leu Leu Thr Asn Ala Val Val Tyr Arg Leu Glu Val Asp Ala
                245                 250                 255
His Asp Asn Ile Ile Ala Ala Leu Tyr Lys Asp Pro Asp Gly Asn Ser
            260                 265                 270
His Arg Val Thr Gly Lys Tyr Phe Val Leu Ala Ala Asn Ala Leu Glu
        275                 280                 285
Ser Ala Lys Ile Leu Leu Met Ser Thr Thr Asp Lys Phe Ala Ser Gly
    290                 295                 300
Leu Ala Asn Ser Ser Asp Gln Val Gly Arg Asn Leu Met Asp His Pro
305                 310                 315                 320
Gly Thr Gly Ile Thr Phe Leu Ala Asn Glu Pro Leu Trp Pro Gly Arg
                325                 330                 335
Gly Pro Met Glu Leu Thr Ser Ile Val Asp His Arg Asp Gly Asp Phe
            340                 345                 350
Arg Arg Glu Trp Ala Ser Lys Lys Leu His Leu Asn Asn Gln Ser Gln
        355                 360                 365
Thr Arg Lys Ala Thr Glu Lys Ala Leu Ser Met Gly Leu Val Gly Lys
    370                 375                 380
Lys Leu Asp Glu Glu Ile Arg Tyr Arg Ala Ala His Thr Val Asn Ile
385                 390                 395                 400
Asn Ser Phe His Asp Ile Leu Pro Glu Pro Asp Asn Arg Leu Val Leu
                405                 410                 415

Ser Asp Gln Lys Asp Pro Leu Gly Leu Pro Arg Met Glu Ile Thr Tyr
            420                 425                 430
Lys Ile Gly Asp Tyr Thr Lys Arg Ser Ala Glu His Thr His Glu Ser
        435                 440                 445
Tyr Ala Lys Ile Ala Gln Leu Phe Gly Gly Thr Glu Val Thr Phe Asp
    450                 455                 460
Asp Lys Phe Ala Pro Asn Asn His Leu Met Gly Ser Val Ile Met Gly
465                 470                 475                 480
Gln Asn Pro Lys Asp Ser Val Val Asp Gly Asp Cys Arg Ala His Asp
                485                 490                 495
His Ala Asn Leu Tyr Leu Ala Thr Ser Gly Val Met Ala Ser Ala Gly
            500                 505                 510
Ser Val Asn Cys Thr Leu Thr Ile Ala Ala Leu Ser Leu Arg Ile Ala
        515                 520                 525
Asp Thr Leu Lys Arg Gln Leu Gly Arg Thr Thr Ser
    530                 535                 540

<210>  14
<211>  481
<212>  PRT
<213>  Silvimonas terrae

<400>  14
Met Thr Cys Arg Leu Leu Arg Pro Phe Ala Leu Ala Ala Leu Met Leu
1               5                   10                  15
Val Ser Ala Leu Ala Gln Ala Ala Glu Val Ser Pro Glu Gln Ile Lys
            20                  25                  30
Arg Gly Glu Tyr Leu Ala Arg Ala Ala Asp Cys Ile Ala Cys His Thr
        35                  40                  45
Val Pro Arg Thr Gly Gln Pro Phe Ala Gly Gly Tyr Gly Ile Ala Ser
    50                  55                  60
Pro Leu Gly Thr Ile Tyr Ser Ser Asn Ile Thr Pro Ser Thr Ser Ala
65                  70                  75                  80
Gly Ile Gly Asn Tyr Thr Leu Gln Asp Phe Asp Asn Ala Leu Arg Arg
                85                  90                  95
Gly Ile Arg Lys Asp Gly Ala His Leu Tyr Pro Ala Met Pro Tyr Thr
            100                 105                 110
Ala Tyr Ala Lys Leu Gln Pro Glu Asp Val Glu Ala Leu Tyr Ala Tyr
        115                 120                 125
Leu Met His Gly Val Lys Pro Val Asp Ala Gln Pro Arg Arg Thr Arg
    130                 135                 140
Leu Pro Phe Pro Phe Asn Ile Arg Leu Ser Met Ala Val Trp Asn Ser
145                 150                 155                 160
Leu Phe Leu Asp Pro Arg Val Phe Gln Pro Glu Gln Gly Lys Ser Glu
                165                 170                 175
Asp Phe Asn Arg Gly Lys Tyr Leu Val Glu Ala Leu Glu His Cys Ser
            180                 185                 190
Ala Cys His Thr Pro Arg Asn Met Leu Met Ala Glu Asn Pro Asp Ala
        195                 200                 205
Pro Phe Ala Gly Gly Gln Leu Gly Ala Trp Tyr Ala Pro Asn Ile Thr
    210                 215                 220
Ala Asp Pro Val Ser Gly Val Gly Gly Trp Thr Gln Ala Glu Leu Val
225                 230                 235                 240
Gln Tyr Leu Lys Thr Gly His Val Ala Gly Lys Ala Ala Ala Ala Gly
                245                 250                 255
Pro Met Ala Glu Ala Val Ser Asp Ser Leu Ser His Leu Thr Asp Pro
            260                 265                 270
Asp Leu Gln Ala Met Ala Thr Tyr Leu Lys Thr Ile Ala Pro Ile Arg
        275                 280                 285
Asn Thr Ala Asp Ser Lys Pro Ala Trp Ala Trp Gly Ser Pro Ala Asn
    290                 295                 300
Asp Asp Asp Ser Leu Arg Gly Thr Ala Gly Val Thr Gln Ser Asn Arg

305                 310                 315                 320
Pro Thr Ser Ala Ala Glu Leu Phe Ser Ala Asn Cys Ala Ser Cys His
                325                 330                 335
Gln Ala Asn Gly Met Gly Thr Thr Asp Thr Ala Tyr Pro Ser Leu Thr
            340                 345                 350
Asn Asn Thr Ala Leu Gly His Leu Asn Pro Ser Asn Leu Ile Ala Val
        355                 360                 365
Ile Leu Asn Gly Val Asp Arg Pro Leu Pro Asp Gly His His Ala Phe
    370                 375                 380
Met Pro Gly Phe Gly Pro Gly Ser Leu Ile Asn Pro Leu Ser Asp Ala
385                 390                 395                 400
Asp Ile Ala Thr Leu Ala Asn Tyr Thr Leu Gln Arg Phe Gly Asn Thr
                405                 410                 415
Asn Val His Val Thr Pro Glu Glu Val Ala Ala Ser Arg Lys Gly Gly
            420                 425                 430
Glu Pro Pro Leu Leu Ala Lys Leu Pro Arg Val Leu Pro Val Val Leu
        435                 440                 445
Met Leu Phe Ala Leu Leu Leu Ile Leu Gly Gly Trp Arg Ser Ala Arg
    450                 455                 460
Gln Arg Lys Leu Arg His Val Ala Arg Pro Ser Pro Thr Pro Arg Thr
465                 470                 475                 480
Pro

<210>  15
<211>  3500
<212>  DNA
<213>  Zymobacter palmae

<220>
<221>  CDS
<222>  (1)..(585)

<220>
<221>  CDS
<222>  (592)..(2193)

<220>
<221>  CDS
<222>  (2199)..(3497)

<400>  15
atg agt ggg ccg gcc ata ttc ccc gct gtg ttc aat cga cga agc gag      48
Met Ser Gly Pro Ala Ile Phe Pro Ala Val Phe Asn Arg Arg Ser Glu
1               5                   10                  15
atc act gtg aaa gat gac acc ccc ctt acg gat gct ggt gcg gtg gcg      96
Ile Thr Val Lys Asp Asp Thr Pro Leu Thr Asp Ala Gly Ala Val Ala
            20                  25                  30
cct gct ttc tct cag cct gtc ggc gta tcg cgt cgc tgc cta ttg ggt      144
Pro Ala Phe Ser Gln Pro Val Gly Val Ser Arg Arg Cys Leu Leu Gly
        35                  40                  45
ggt gcc gcg ctg gga ctg gca gcg att acg cta ggt gcg ctt ccc gta      192
Gly Ala Ala Leu Gly Leu Ala Ala Ile Thr Leu Gly Ala Leu Pro Val
    50                  55                  60
gcg cag gca gca gac aac gcc caa gat agc gcg ctg gct gac ttc atc      240
Ala Gln Ala Ala Asp Asn Ala Gln Asp Ser Ala Leu Ala Asp Phe Ile
65                  70                  75                  80
cgc gta tca ggt gtg ctg aca gca cgt cag caa ctc aac ccg gtg ctg      288
Arg Val Ser Gly Val Leu Thr Ala Arg Gln Gln Leu Asn Pro Val Leu
                85                  90                  95
tca gga ctc atc ttc ggt gaa atg aag aaa gaa gat ccg gcc ttt gct      336
Ser Gly Leu Ile Phe Gly Glu Met Lys Lys Glu Asp Pro Ala Phe Ala

            100                 105                 110
gat cag ctg tct tcc tta gcg ggc gtt atc cat cag ccg cca gcg cag 384
Asp Gln Leu Ser Ser Leu Ala Gly Val Ile His Gln Pro Pro Ala Gln
        115                 120                 125
tgg agt gct tcc atg acg acg ctg gcg cgt gaa ata acc tcc gtc tgg 432
Trp Ser Ala Ser Met Thr Thr Leu Ala Arg Glu Ile Thr Ser Val Trp
    130                 135                 140
tac acc ggg ttg atc ggg agc gga gac acc atg cgt gtc gtg acg tat 480
Tyr Thr Gly Leu Ile Gly Ser Gly Asp Thr Met Arg Val Val Thr Tyr
145                 150                 155                 160
gaa ggt gcg ctc cag ttt tcg gcg acg aag gat atc ttc att gtg cgc 528
Glu Gly Ala Leu Gln Phe Ser Ala Thr Lys Asp Ile Phe Ile Val Arg
                165                 170                 175
agc tat tgc ccc aat cgt ccc ggt ttc tgg gcc agc caa cct tca gga 576
Ser Tyr Cys Pro Asn Arg Pro Gly Phe Trp Ala Ser Gln Pro Ser Gly
            180                 185                 190
tgg aga gtg tgattc atg gca gga att caa aag gct gac gtt gtc atc 624
Trp Arg Val Met Ala Gly Ile Gln Lys Ala Asp Val Val Ile
        195                 200                 205
gtt ggc aca ggg gtg gcc ggt gct ctg att gca cat cag ctg gcc aag 672
Val Gly Thr Gly Val Ala Gly Ala Leu Ile Ala His Gln Leu Ala Lys
            210                 215                 220
gcg ggc aag cgt gta ctg atg ctg gaa gcg ggg ccg cgt ctg aca cgt 720
Ala Gly Lys Arg Val Leu Met Leu Glu Ala Gly Pro Arg Leu Thr Arg
        225                 230                 235
ggt gaa att act ggc aac ttt cgt tcc agt gcg ttc aag tcg gat ttt 768
Gly Glu Ile Thr Gly Asn Phe Arg Ser Ser Ala Phe Lys Ser Asp Phe
    240                 245                 250
caa tcg cca tac ccg tgt acc gac tat gcg ccg att ccg acg atg cac 816
Gln Ser Pro Tyr Pro Cys Thr Asp Tyr Ala Pro Ile Pro Thr Met His
255                 260                 265                 270
ccc aag aac gac tac ttc atc caa agc ggt gag cac tcc tac gat gcg 864
Pro Lys Asn Asp Tyr Phe Ile Gln Ser Gly Glu His Ser Tyr Asp Ala
                275                 280                 285
cag tac ctg cgc ttg gtt ggt ggt acg act tgg cac tgg gcg gca tcg 912
Gln Tyr Leu Arg Leu Val Gly Gly Thr Thr Trp His Trp Ala Ala Ser
            290                 295                 300
gcg tgg cgc tat ctg ccg gtc gat ttc aag ctg aag agc acc tat ggt 960
Ala Trp Arg Tyr Leu Pro Val Asp Phe Lys Leu Lys Ser Thr Tyr Gly
        305                 310                 315
gtc ggg gtt gac tgg ccc atc gac tac gat gca ctg gag ccg tgg tat 1008
Val Gly Val Asp Trp Pro Ile Asp Tyr Asp Ala Leu Glu Pro Trp Tyr
    320                 325                 330
cag cgt gct gaa gaa gca ctg ggt gtg tgg ggg cct aat gat gaa gag 1056
Gln Arg Ala Glu Glu Ala Leu Gly Val Trp Gly Pro Asn Asp Glu Glu
335                 340                 345                 350
cta ggt tca ccg cgt tcc cag cct tat cca atg acg ccg ctg ccg ctg 1104
Leu Gly Ser Pro Arg Ser Gln Pro Tyr Pro Met Thr Pro Leu Pro Leu
                355                 360                 365
tcc tat aac gaa cac acg atc aag acg cgc ctc aat gcg cag ggg ttc 1152
Ser Tyr Asn Glu His Thr Ile Lys Thr Arg Leu Asn Ala Gln Gly Phe
            370                 375                 380
ggg atc gtg acg gag ccg gtg gcg cgc aat agc cgc ccc tac gat gat 1200
Gly Ile Val Thr Glu Pro Val Ala Arg Asn Ser Arg Pro Tyr Asp Asp
        385                 390                 395
cgc ccc acc tgc tgc ggt aat aac aac tgc atg ccg atc tgc cct att 1248
Arg Pro Thr Cys Cys Gly Asn Asn Asn Cys Met Pro Ile Cys Pro Ile
    400                 405                 410
ggc gcg atg tat ggc ggt atc gtc cat gtc gaa aaa gcc gaa aag gcg 1296
Gly Ala Met Tyr Gly Gly Ile Val His Val Glu Lys Ala Glu Lys Ala
415                 420                 425                 430
ggg gct aaa gtc gtt ccc gag gcg gtg gtg tac cgc atc gag acg ggc 1344
Gly Ala Lys Val Val Pro Glu Ala Val Val Tyr Arg Ile Glu Thr Gly

                435                 440                 445
gat gac aac cgt atc gtg gcg gtg cat tac aag gat ccg aaa ggc gag 1392
Asp Asp Asn Arg Ile Val Ala Val His Tyr Lys Asp Pro Lys Gly Glu
            450                 455                 460
tcg cac cgc gtt gaa ggt aaa tac ttc gtc atc gcg gca aac ggc atc 1440
Ser His Arg Val Glu Gly Lys Tyr Phe Val Ile Ala Ala Asn Gly Ile
        465                 470                 475
gaa acg ccg aag ttg ttg cag att tcg gtc gat agc cgc aat ccg aat 1488
Glu Thr Pro Lys Leu Leu Gln Ile Ser Val Asp Ser Arg Asn Pro Asn
    480                 485                 490
ggt gtg gcc aac agc tct ggc tgg gtg ggt cgc cac ctg atg gac cac 1536
Gly Val Ala Asn Ser Ser Gly Trp Val Gly Arg His Leu Met Asp His
495                 500                 505                 510
ccg ggg atc ggc gta cgc ttc tat gcc aag gaa ccg ttg tgg ccg ggc 1584
Pro Gly Ile Gly Val Arg Phe Tyr Ala Lys Glu Pro Leu Trp Pro Gly
                515                 520                 525
cgt ggg cct cag gaa atg acc tct gcg gtt gcg tgg cgc gat ggg gat 1632
Arg Gly Pro Gln Glu Met Thr Ser Ala Val Ala Trp Arg Asp Gly Asp
            530                 535                 540
ttc cga cgc acg gca gcg ggc aaa aag atc cac cta tcc aac ctg tcg 1680
Phe Arg Arg Thr Ala Ala Gly Lys Lys Ile His Leu Ser Asn Leu Ser
        545                 550                 555
cgt gtc gat cag gtc acc caa gag ctc ctt gcc ggt gat cag ctg att 1728
Arg Val Asp Gln Val Thr Gln Glu Leu Leu Ala Gly Asp Gln Leu Ile
    560                 565                 570
ttc gga aac gcg ctg gaa gag cag atc cgt gat cgc gca gca cgc ttc 1776
Phe Gly Asn Ala Leu Glu Glu Gln Ile Arg Asp Arg Ala Ala Arg Phe
575                 580                 585                 590
gtg cag ttc gac agt ttc cac gaa ata ctg ccg ctt cct gaa aac cgc 1824
Val Gln Phe Asp Ser Phe His Glu Ile Leu Pro Leu Pro Glu Asn Arg
                595                 600                 605
att gtg ccg agc aag gtg cat acg gac aaa ctg ggg att ccc aag cct 1872
Ile Val Pro Ser Lys Val His Thr Asp Lys Leu Gly Ile Pro Lys Pro
            610                 615                 620
gaa ttt ttc tac cgc atc gac gat tac gtt aag cgg ggg gca gcg cat 1920
Glu Phe Phe Tyr Arg Ile Asp Asp Tyr Val Lys Arg Gly Ala Ala His
        625                 630                 635
acc cgc gag atg tat gcg cgt gcg gca cag gtc atg ggg ggt acg gat 1968
Thr Arg Glu Met Tyr Ala Arg Ala Ala Gln Val Met Gly Gly Thr Asp
    640                 645                 650
att cag tat gcc gat gac ttt gct aat aac cag cat atc tgc ggt acg 2016
Ile Gln Tyr Ala Asp Asp Phe Ala Asn Asn Gln His Ile Cys Gly Thr
655                 660                 665                 670
gtg atg atg ggg cca acg gcc gaa act ggg gta gtg gat cag cac tgc 2064
Val Met Met Gly Pro Thr Ala Glu Thr Gly Val Val Asp Gln His Cys
                675                 680                 685
cgt acg ttc gat cac gag aac ctg ttt ttg gcc acc agt ggc gtc atg 2112
Arg Thr Phe Asp His Glu Asn Leu Phe Leu Ala Thr Ser Gly Val Met
            690                 695                 700
ccg acg gtg gga acg gtc aac tgt acg ctg act gtg gcg gca ctg tcg 2160
Pro Thr Val Gly Thr Val Asn Cys Thr Leu Thr Val Ala Ala Leu Ser
        705                 710                 715
ctg cgc atg gcc gaa gtc atc aag gcg gag gtg tgatg atg gcg agc aac 2210
Leu Arg Met Ala Glu Val Ile Lys Ala Glu Val Met Ala Ser Asn
    720                 725                 730
ttt tgg cgg gtg tcg ctg ggt gtg ggg gcg atg gcg gcc ctg tgg atg 2258
Phe Trp Arg Val Ser Leu Gly Val Gly Ala Met Ala Ala Leu Trp Met
    735                 740                 745
agc ggt gtg gcg gca gcg gaa gat gct agt acg cga cag gcc gag ctg 2306
Ser Gly Val Ala Ala Ala Glu Asp Ala Ser Thr Arg Gln Ala Glu Leu
750                 755                 760                 765
gtg aaa cga ggt gag tat gtg gcg cgg acg gcg gac tgt gtg gca tgc 2354
Val Lys Arg Gly Glu Tyr Val Ala Arg Thr Ala Asp Cys Val Ala Cys

                770                 775                 780
cat acc act gat cat gac aag ccc ttt gcg ggt ggt ttg ccg atg ggg 2402
His Thr Thr Asp His Asp Lys Pro Phe Ala Gly Gly Leu Pro Met Gly
            785                 790                 795
ttg ccg atg ggc aac att tat tcg acc aat atc acg ccg gat aag gat 2450
Leu Pro Met Gly Asn Ile Tyr Ser Thr Asn Ile Thr Pro Asp Lys Asp
        800                 805                 810
act ggg atc ggc agc tat acg ttg gag gag ttc aag cgt gtg ctg cgt 2498
Thr Gly Ile Gly Ser Tyr Thr Leu Glu Glu Phe Lys Arg Val Leu Arg
    815                 820                 825
gaa ggg gtg acg ccg gat cga ggc aac ctt tat ccg gcg atg ccg tat 2546
Glu Gly Val Thr Pro Asp Arg Gly Asn Leu Tyr Pro Ala Met Pro Tyr
830                 835                 840                 845
ccg tct tat acc aag atg acc gat gat gaa atc gag gcg ctg tat gcc 2594
Pro Ser Tyr Thr Lys Met Thr Asp Asp Glu Ile Glu Ala Leu Tyr Ala
                850                 855                 860
tat ttc atg cat ggg gtt caa ccg att cat cag gaa aac aag gcg ccg 2642
Tyr Phe Met His Gly Val Gln Pro Ile His Gln Glu Asn Lys Ala Pro
            865                 870                 875
gac ttt tac tgg ccg ctg acc att cgc tgg cct ttg aag ata tgg aac 2690
Asp Phe Tyr Trp Pro Leu Thr Ile Arg Trp Pro Leu Lys Ile Trp Asn
        880                 885                 890
tgg atg ttt ctt gat gaa ggt gcc tat cag ccc aag gcc ggg cag agc 2738
Trp Met Phe Leu Asp Glu Gly Ala Tyr Gln Pro Lys Ala Gly Gln Ser
    895                 900                 905
gat gaa tgg aac cgg ggt gcc tat ctt gtg cag ggg cct gcg cac tgt 2786
Asp Glu Trp Asn Arg Gly Ala Tyr Leu Val Gln Gly Pro Ala His Cys
910                 915                 920                 925
ggc acg tgc cat act ccg cgc ggg ctg gca atg cag gag cgc gct tat 2834
Gly Thr Cys His Thr Pro Arg Gly Leu Ala Met Gln Glu Arg Ala Tyr
                930                 935                 940
gat gag aca ggc gac gag tac ttg gcg ggc gct gat atc gcg agc tgg 2882
Asp Glu Thr Gly Asp Glu Tyr Leu Ala Gly Ala Asp Ile Ala Ser Trp
            945                 950                 955
cat gct ttc aac atc act tcg gac atc aac agt ggg atc ggc ggc tgg 2930
His Ala Phe Asn Ile Thr Ser Asp Ile Asn Ser Gly Ile Gly Gly Trp
        960                 965                 970
agt gcc gat gag ctg gtg cag tat ctg aag gat ggc aat gtg cgt ggc 2978
Ser Ala Asp Glu Leu Val Gln Tyr Leu Lys Asp Gly Asn Val Arg Gly
    975                 980                 985
aag ggg cag gcc gga ggg cag atg ggt gag gcg gtt gaa cac agc ttc 3026
Lys Gly Gln Ala Gly Gly Gln Met Gly Glu Ala Val Glu His Ser Phe
990                 995                 1000                1005
agt cat atg acc gat gca gat ctg cat gct atc gcg gtg tat atg 3071
Ser His Met Thr Asp Ala Asp Leu His Ala Ile Ala Val Tyr Met
                1010                1015                1020
aag acg gtg cct gcc gtc aac gat ggc gat acc cgt cca cgc tat 3116
Lys Thr Val Pro Ala Val Asn Asp Gly Asp Thr Arg Pro Arg Tyr
                1025                1030                1035
agc tat ggc gct cct ggc gat gac ttc ctg cgc ctt cgg gcc cag 3161
Ser Tyr Gly Ala Pro Gly Asp Asp Phe Leu Arg Leu Arg Ala Gln
                1040                1045                1050
ccc gtt gct caa gat gcc gca tcg gat cga ggg gcg cgc gtg tat 3206
Pro Val Ala Gln Asp Ala Ala Ser Asp Arg Gly Ala Arg Val Tyr
                1055                1060                1065
ctg gag aac tgc gcg gct tgc cac ggg cct tcc gga caa ggc tcg 3251
Leu Glu Asn Cys Ala Ala Cys His Gly Pro Ser Gly Gln Gly Ser
                1070                1075                1080
cct gat ggc tat tat ccg tcg atg ttc cat aac agc gtc atc ggt 3296
Pro Asp Gly Tyr Tyr Pro Ser Met Phe His Asn Ser Val Ile Gly
                1085                1090                1095
acc gat tac cac aac aac ctc atc aac gtg gta ttg gat ggc gtg 3341
Thr Asp Tyr His Asn Asn Leu Ile Asn Val Val Leu Asp Gly Val

1100                    1105                    1110
aag cgc aag acc cca  ggc aac gac gtg ctg  atg cca gcc ttc cgc          3386
Lys Arg Lys Thr Pro  Gly Asn Asp Val Leu  Met Pro Ala Phe Arg
                1115                    1120                    1125
gat gag ctg tcc gat  gag gat atc gcg gcg  ttg gct aac tac ctc          3431
Asp Glu Leu Ser Asp  Glu Asp Ile Ala Ala  Leu Ala Asn Tyr Leu
                1130                    1135                    1140
agc cag cag ttc ggg  cgt ggc gat atg aat  gtg acg ccc aaa tcc          3476
Ser Gln Gln Phe Gly  Arg Gly Asp Met Asn  Val Thr Pro Lys Ser
                1145                    1150                    1155
gtc aag gca ctg cgc  aac gat tga                                       3500
Val Lys Ala Leu Arg  Asn Asp
                1160

<210>  16
<211>  195
<212>  PRT
<213>  Zymobacter palmae

<400>  16
Met Ser Gly Pro Ala Ile Phe Pro Ala Val Phe Asn Arg Arg Ser Glu
1               5                   10                  15
Ile Thr Val Lys Asp Asp Thr Pro Leu Thr Asp Ala Gly Ala Val Ala
            20                  25                  30
Pro Ala Phe Ser Gln Pro Val Gly Val Ser Arg Arg Cys Leu Leu Gly
        35                  40                  45
Gly Ala Ala Leu Gly Leu Ala Ala Ile Thr Leu Gly Ala Leu Pro Val
    50                  55                  60
Ala Gln Ala Ala Asp Asn Ala Gln Asp Ser Ala Leu Ala Asp Phe Ile
65                  70                  75                  80
Arg Val Ser Gly Val Leu Thr Ala Arg Gln Gln Leu Asn Pro Val Leu
                85                  90                  95
Ser Gly Leu Ile Phe Gly Glu Met Lys Lys Glu Asp Pro Ala Phe Ala
            100                 105                 110
Asp Gln Leu Ser Ser Leu Ala Gly Val Ile His Gln Pro Pro Ala Gln
        115                 120                 125
Trp Ser Ala Ser Met Thr Thr Leu Ala Arg Glu Ile Thr Ser Val Trp
    130                 135                 140
Tyr Thr Gly Leu Ile Gly Ser Gly Asp Thr Met Arg Val Val Thr Tyr
145                 150                 155                 160
Glu Gly Ala Leu Gln Phe Ser Ala Thr Lys Asp Ile Phe Ile Val Arg
                165                 170                 175
Ser Tyr Cys Pro Asn Arg Pro Gly Phe Trp Ala Ser Gln Pro Ser Gly
            180                 185                 190
Trp Arg Val
        195

<210>  17
<211>  534
<212>  PRT
<213>  Zymobacter palmae

<400>  17
Met Ala Gly Ile Gln Lys Ala Asp Val Val Ile Val Gly Thr Gly Val
1               5                   10                  15
Ala Gly Ala Leu Ile Ala His Gln Leu Ala Lys Ala Gly Lys Arg Val
            20                  25                  30
Leu Met Leu Glu Ala Gly Pro Arg Leu Thr Arg Gly Glu Ile Thr Gly
        35                  40                  45
Asn Phe Arg Ser Ser Ala Phe Lys Ser Asp Phe Gln Ser Pro Tyr Pro
    50                  55                  60

Cys Thr Asp Tyr Ala Pro Ile Pro Thr Met His Pro Lys Asn Asp Tyr
65                  70                  75                  80
Phe Ile Gln Ser Gly Glu His Ser Tyr Asp Ala Gln Tyr Leu Arg Leu
                85                  90                  95
Val Gly Gly Thr Thr Trp His Trp Ala Ala Ser Ala Trp Arg Tyr Leu
            100                 105                 110
Pro Val Asp Phe Lys Leu Lys Ser Thr Tyr Gly Val Gly Val Asp Trp
        115                 120                 125
Pro Ile Asp Tyr Asp Ala Leu Glu Pro Trp Tyr Gln Arg Ala Glu Glu
    130                 135                 140
Ala Leu Gly Val Trp Gly Pro Asn Asp Glu Glu Leu Gly Ser Pro Arg
145                 150                 155                 160
Ser Gln Pro Tyr Pro Met Thr Pro Leu Pro Leu Ser Tyr Asn Glu His
                165                 170                 175
Thr Ile Lys Thr Arg Leu Asn Ala Gln Gly Phe Gly Ile Val Thr Glu
            180                 185                 190
Pro Val Ala Arg Asn Ser Arg Pro Tyr Asp Asp Arg Pro Thr Cys Cys
        195                 200                 205
Gly Asn Asn Asn Cys Met Pro Ile Cys Pro Ile Gly Ala Met Tyr Gly
    210                 215                 220
Gly Ile Val His Val Glu Lys Ala Glu Lys Ala Gly Ala Lys Val Val
225                 230                 235                 240
Pro Glu Ala Val Val Tyr Arg Ile Glu Thr Gly Asp Asp Asn Arg Ile
                245                 250                 255
Val Ala Val His Tyr Lys Asp Pro Lys Gly Glu Ser His Arg Val Glu
            260                 265                 270
Gly Lys Tyr Phe Val Ile Ala Ala Asn Gly Ile Glu Thr Pro Lys Leu
        275                 280                 285
Leu Gln Ile Ser Val Asp Ser Arg Asn Pro Asn Gly Val Ala Asn Ser
    290                 295                 300
Ser Gly Trp Val Gly Arg His Leu Met Asp His Pro Gly Ile Gly Val
305                 310                 315                 320
Arg Phe Tyr Ala Lys Glu Pro Leu Trp Pro Gly Arg Gly Pro Gln Glu
                325                 330                 335
Met Thr Ser Ala Val Ala Trp Arg Asp Gly Asp Phe Arg Arg Thr Ala
            340                 345                 350
Ala Gly Lys Lys Ile His Leu Ser Asn Leu Ser Arg Val Asp Gln Val
        355                 360                 365
Thr Gln Glu Leu Leu Ala Gly Asp Gln Leu Ile Phe Gly Asn Ala Leu
    370                 375                 380
Glu Glu Gln Ile Arg Asp Arg Ala Ala Arg Phe Val Gln Phe Asp Ser
385                 390                 395                 400
Phe His Glu Ile Leu Pro Leu Pro Glu Asn Arg Ile Val Pro Ser Lys
                405                 410                 415
Val His Thr Asp Lys Leu Gly Ile Pro Lys Pro Glu Phe Phe Tyr Arg
            420                 425                 430
Ile Asp Asp Tyr Val Lys Arg Gly Ala Ala His Thr Arg Glu Met Tyr
        435                 440                 445
Ala Arg Ala Ala Gln Val Met Gly Gly Thr Asp Ile Gln Tyr Ala Asp
    450                 455                 460
Asp Phe Ala Asn Asn Gln His Ile Cys Gly Thr Val Met Met Gly Pro
465                 470                 475                 480
Thr Ala Glu Thr Gly Val Val Asp Gln His Cys Arg Thr Phe Asp His
                485                 490                 495
Glu Asn Leu Phe Leu Ala Thr Ser Gly Val Met Pro Thr Val Gly Thr
            500                 505                 510
Val Asn Cys Thr Leu Thr Val Ala Ala Leu Ser Leu Arg Met Ala Glu
        515                 520                 525
Val Ile Lys Ala Glu Val
    530

<210>  18

<211>  433
<212>  PRT
<213>  Zymobacter palmae

<400>  18
Met Ala Ser Asn Phe Trp Arg Val Ser Leu Gly Val Gly Ala Met Ala
1               5                   10                  15
Ala Leu Trp Met Ser Gly Val Ala Ala Ala Glu Asp Ala Ser Thr Arg
            20                  25                  30
Gln Ala Glu Leu Val Lys Arg Gly Glu Tyr Val Ala Arg Thr Ala Asp
        35                  40                  45
Cys Val Ala Cys His Thr Thr Asp His Asp Lys Pro Phe Ala Gly Gly
    50                  55                  60
Leu Pro Met Gly Leu Pro Met Gly Asn Ile Tyr Ser Thr Asn Ile Thr
65                  70                  75                  80
Pro Asp Lys Asp Thr Gly Ile Gly Ser Tyr Thr Leu Glu Glu Phe Lys
                85                  90                  95
Arg Val Leu Arg Glu Gly Val Thr Pro Asp Arg Gly Asn Leu Tyr Pro
            100                 105                 110
Ala Met Pro Tyr Pro Ser Tyr Thr Lys Met Thr Asp Asp Glu Ile Glu
        115                 120                 125
Ala Leu Tyr Ala Tyr Phe Met His Gly Val Gln Pro Ile His Gln Glu
    130                 135                 140
Asn Lys Ala Pro Asp Phe Tyr Trp Pro Leu Thr Ile Arg Trp Pro Leu
145                 150                 155                 160
Lys Ile Trp Asn Trp Met Phe Leu Asp Glu Gly Ala Tyr Gln Pro Lys
                165                 170                 175
Ala Gly Gln Ser Asp Glu Trp Asn Arg Gly Ala Tyr Leu Val Gln Gly
            180                 185                 190
Pro Ala His Cys Gly Thr Cys His Thr Pro Arg Gly Leu Ala Met Gln
        195                 200                 205
Glu Arg Ala Tyr Asp Glu Thr Gly Asp Glu Tyr Leu Ala Gly Ala Asp
    210                 215                 220
Ile Ala Ser Trp His Ala Phe Asn Ile Thr Ser Asp Ile Asn Ser Gly
225                 230                 235                 240
Ile Gly Gly Trp Ser Ala Asp Glu Leu Val Gln Tyr Leu Lys Asp Gly
                245                 250                 255
Asn Val Arg Gly Lys Gly Gln Ala Gly Gly Gln Met Gly Glu Ala Val
            260                 265                 270
Glu His Ser Phe Ser His Met Thr Asp Ala Asp Leu His Ala Ile Ala
        275                 280                 285
Val Tyr Met Lys Thr Val Pro Ala Val Asn Asp Gly Asp Thr Arg Pro
    290                 295                 300
Arg Tyr Ser Tyr Gly Ala Pro Gly Asp Asp Phe Leu Arg Leu Arg Ala
305                 310                 315                 320
Gln Pro Val Ala Gln Asp Ala Ala Ser Asp Arg Gly Ala Arg Val Tyr
                325                 330                 335
Leu Glu Asn Cys Ala Ala Cys His Gly Pro Ser Gly Gln Gly Ser Pro
            340                 345                 350
Asp Gly Tyr Tyr Pro Ser Met Phe His Asn Ser Val Ile Gly Thr Asp
        355                 360                 365
Tyr His Asn Asn Leu Ile Asn Val Val Leu Asp Gly Val Lys Arg Lys
    370                 375                 380
Thr Pro Gly Asn Asp Val Leu Met Pro Ala Phe Arg Asp Glu Leu Ser
385                 390                 395                 400
Asp Glu Asp Ile Ala Ala Leu Ala Asn Tyr Leu Ser Gln Gln Phe Gly
                405                 410                 415
Arg Gly Asp Met Asn Val Thr Pro Lys Ser Val Lys Ala Leu Arg Asn
            420                 425                 430
Asp

<210>  19

<211>  3938
<212>  DNA
<213>  Cobetia sp.

<220>
<221>  CDS
<222>  (1)..(621)

<220>
<221>  CDS
<222>  (752)..(2362)

<220>
<221>  CDS
<222>  (2368)..(3933)

<400>  19
atg acc cca cgt gaa aat agt gtt tcg gat gat gtc ccc atg cgc cag      48
Met Thr Pro Arg Glu Asn Ser Val Ser Asp Asp Val Pro Met Arg Gln
1               5                   10                  15
gat ggt cat cat gaa gcg tcc caa gtc gct gag ccg acc agc ccg gca      96
Asp Gly His His Glu Ala Ser Gln Val Ala Glu Pro Thr Ser Pro Ala
            20                  25                  30
cgt cgt cgt ctg ctc aag ctg ggt gtg tcg ggg ctg gga ttg gtg gcg      144
Arg Arg Arg Leu Leu Lys Leu Gly Val Ser Gly Leu Gly Leu Val Ala
        35                  40                  45
ctt tcc agc ctg tca ctt tct gca ctg gcg gcc agt gcc acc tca tca      192
Leu Ser Ser Leu Ser Leu Ser Ala Leu Ala Ala Ser Ala Thr Ser Ser
    50                  55                  60
gag gcg tca tcc ggt gcc gcg tct gat gat gac tca gcc ttc aag gcg      240
Glu Ala Ser Ser Gly Ala Ala Ser Asp Asp Asp Ser Ala Phe Lys Ala
65                  70                  75                  80
ttc atg ctc ctg tca gca tgg tta ctg agc gac aag acg ctg gat aaa      288
Phe Met Leu Leu Ser Ala Trp Leu Leu Ser Asp Lys Thr Leu Asp Lys
                85                  90                  95
cgt ctg ggt gag cgt ttc ttt gag gca ttg gtg cgt atc cct gcc ccc      336
Arg Leu Gly Glu Arg Phe Phe Glu Ala Leu Val Arg Ile Pro Ala Pro
            100                 105                 110
aat gtg cca ggc atg ggt agt ttg ccc gcc ctg aag aac aag ttg ctg      384
Asn Val Pro Gly Met Gly Ser Leu Pro Ala Leu Lys Asn Lys Leu Leu
        115                 120                 125
gca ctt ggc gag gcg cgt gac tat ctg aag gaa gac gat ctc gac gcc      432
Ala Leu Gly Glu Ala Arg Asp Tyr Leu Lys Glu Asp Asp Leu Asp Ala
    130                 135                 140
tcc gag atg cag ttg gtg cga cgt gtc ctc caa gcc tgg atg ctg ggc      480
Ser Glu Met Gln Leu Val Arg Arg Val Leu Gln Ala Trp Met Leu Gly
145                 150                 155                 160
acg gtt ggc act gca att gat gat ccc gcg gcg gaa gtc atc gcc tat      528
Thr Val Gly Thr Ala Ile Asp Asp Pro Ala Ala Glu Val Ile Ala Tyr
                165                 170                 175
gaa tac gcg gcc atg tat gcc ggc cct cgc gat gtg cag atc atc cgc      576
Glu Tyr Ala Ala Met Tyr Ala Gly Pro Arg Asp Val Gln Ile Ile Arg
            180                 185                 190
acc tat tgc cct aac caa ccg ggc ttc tgg gca cag cgt cct gag          621
Thr Tyr Cys Pro Asn Gln Pro Gly Phe Trp Ala Gln Arg Pro Glu
        195                 200                 205
tgatcatgcc tcgtcagtcc tgaccctgat gctgtccctg atacgctttt tgaacttcct      681
gacaacttga ctcgggtact tcccgatcaa gattcacgtc taaaaaatgt tcctctgagg      741
agaatcctgc atg gct gcg gaa tcc cat tcg cat gat gcc gat gtc atc      790
           Met Ala Ala Glu Ser His Ser His Asp Ala Asp Val Ile
               210                 215                 220
atc gtc ggt tct ggt gtt gct ggc gcc ttg att gcc cac cgg ctt gcc      838
Ile Val Gly Ser Gly Val Ala Gly Ala Leu Ile Ala His Arg Leu Ala

225                 230                 235
gag gca ggt aaa tca gtc atc atg ctt gag gct ggc cct cgc atg cag          886
Glu Ala Gly Lys Ser Val Ile Met Leu Glu Ala Gly Pro Arg Met Gln
        240                 245                 250
cgc tgg cag att gtg gag cgc ttc cgt aat caa gcc gac aag atg gac          934
Arg Trp Gln Ile Val Glu Arg Phe Arg Asn Gln Ala Asp Lys Met Asp
    255                 260                 265
ttc atg ggg cct tat ccg gca agc aag gct gcg cca cac ccg atg ctg          982
Phe Met Gly Pro Tyr Pro Ala Ser Lys Ala Ala Pro His Pro Met Leu
    270                 275                 280
tat ggc gag aat gcc aat tac ctc gtg cag aag ggc gag cag gtc tat         1030
Tyr Gly Glu Asn Ala Asn Tyr Leu Val Gln Lys Gly Glu Gln Val Tyr
285                 290                 295                 300
gac gcg caa tac att cgc gcc gtc ggt ggt act act tgg cac tgg gcg         1078
Asp Ala Gln Tyr Ile Arg Ala Val Gly Gly Thr Thr Trp His Trp Ala
                305                 310                 315
gcg tct gca tgg cgc ttc ctg cct agc gat ttc cgc ttg aaa agc gaa         1126
Ala Ser Ala Trp Arg Phe Leu Pro Ser Asp Phe Arg Leu Lys Ser Glu
            320                 325                 330
tat ggc gtc gga ctg gat tgg ccg att tcc tat gat gac ctg gaa ggc         1174
Tyr Gly Val Gly Leu Asp Trp Pro Ile Ser Tyr Asp Asp Leu Glu Gly
        335                 340                 345
tac tac ttc cgt gcc gag cag gcg ctg ggg gta tgg ggc ccg aat gat         1222
Tyr Tyr Phe Arg Ala Glu Gln Ala Leu Gly Val Trp Gly Pro Asn Asp
    350                 355                 360
gtt gac ctc ggc agc ccg cgc agc gct gat tat ccg atg gca ccg ttg         1270
Val Asp Leu Gly Ser Pro Arg Ser Ala Asp Tyr Pro Met Ala Pro Leu
365                 370                 375                 380
ccg ctg tca tgg aat gag cag cgt gtc agt cag cgg atc aat ccg cat         1318
Pro Leu Ser Trp Asn Glu Gln Arg Val Ser Gln Arg Ile Asn Pro His
                385                 390                 395
ggc ttt gac atg gtc acc gaa cct gtc gcg cgc aac agt cgc ccg tac         1366
Gly Phe Asp Met Val Thr Glu Pro Val Ala Arg Asn Ser Arg Pro Tyr
            400                 405                 410
gac gat cgc ccg acc tgc tgt ggc aac aac aac tgc atg ccg atc tgt         1414
Asp Asp Arg Pro Thr Cys Cys Gly Asn Asn Asn Cys Met Pro Ile Cys
        415                 420                 425
ccg atc ggg gcg atg tac tcc ggc atc atc cat gtc gag aag gcc gaa         1462
Pro Ile Gly Ala Met Tyr Ser Gly Ile Ile His Val Glu Lys Ala Glu
    430                 435                 440
cgt gcg ggt gcc aag ttg atc gac aat gct gtg gtg cac cat ctg gaa         1510
Arg Ala Gly Ala Lys Leu Ile Asp Asn Ala Val Val His His Leu Glu
445                 450                 455                 460
agc gat gcc agc ggc aag atc cgt gaa gtg cgc tat ctc gat ccg cag         1558
Ser Asp Ala Ser Gly Lys Ile Arg Glu Val Arg Tyr Leu Asp Pro Gln
                465                 470                 475
ggc aag gaa cat cgc ctg agc gcg ccc aag gtt gtg ctg gcg gcc aac         1606
Gly Lys Glu His Arg Leu Ser Ala Pro Lys Val Val Leu Ala Ala Asn
            480                 485                 490
ggt atc gaa acg ccc aag ctg atg cag atg tcg gtg aat gca cat acg         1654
Gly Ile Glu Thr Pro Lys Leu Met Gln Met Ser Val Asn Ala His Thr
        495                 500                 505
ccc aat ggt gtc ggc aat cag aat gac atg gtg ggg cgt cac ctg atg         1702
Pro Asn Gly Val Gly Asn Gln Asn Asp Met Val Gly Arg His Leu Met
    510                 515                 520
gat cac ccc ggt acc ggc gtc agc ttc ctg gcc gat gaa gtg ctg tgg         1750
Asp His Pro Gly Thr Gly Val Ser Phe Leu Ala Asp Glu Val Leu Trp
525                 530                 535                 540
gca ggg cgt ggg cct cag gag atg acc tcg atc atc act tgg cgt gat         1798
Ala Gly Arg Gly Pro Gln Glu Met Thr Ser Ile Ile Thr Trp Arg Asp
                545                 550                 555
ggg gac ttc cgc aag gag tac gcc tcc aag aag ctg cac ctc tcc aac         1846
Gly Asp Phe Arg Lys Glu Tyr Ala Ser Lys Lys Leu His Leu Ser Asn

560                 565                 570
atg gcg cgt aca cag cag atg acg acg gag gtg ttg tcg gaa acc ccg        1894
Met Ala Arg Thr Gln Gln Met Thr Thr Glu Val Leu Ser Glu Thr Pro
        575                 580                 585
ctg cgc ctg ggc gcc gac ctg cag gcc cag att gac aat cgt gcc agc        1942
Leu Arg Leu Gly Ala Asp Leu Gln Ala Gln Ile Asp Asn Arg Ala Ser
    590                 595                 600
cgt tat gtg caa ctg gat agc ttc cat gag ttg ttg ccg gag ccg tcc        1990
Arg Tyr Val Gln Leu Asp Ser Phe His Glu Leu Leu Pro Glu Pro Ser
605                 610                 615                 620
aat cgc atc acg cca tca aaa aaa cgc gat gcg ctc ggc ctg ccg agg        2038
Asn Arg Ile Thr Pro Ser Lys Lys Arg Asp Ala Leu Gly Leu Pro Arg
                625                 630                 635
cca gaa ttc cgt tat gct atc gat gat tac gtc aag cgc agc gcc gtg        2086
Pro Glu Phe Arg Tyr Ala Ile Asp Asp Tyr Val Lys Arg Ser Ala Val
            640                 645                 650
cat acc cga gaa caa tat gca cgt atc gct cag ctg atg gga ggc acg        2134
His Thr Arg Glu Gln Tyr Ala Arg Ile Ala Gln Leu Met Gly Gly Thr
        655                 660                 665
gat atc gcg ttc cgt gac gaa ttc tcc aac aac cag cat atc tgc ggt        2182
Asp Ile Ala Phe Arg Asp Glu Phe Ser Asn Asn Gln His Ile Cys Gly
    670                 675                 680
acg acc ctg atg gga gac gac ccc aag aag tcc gtg gtg gac cgc gat        2230
Thr Thr Leu Met Gly Asp Asp Pro Lys Lys Ser Val Val Asp Arg Asp
685                 690                 695                 700
tgc cgt gtg cat ggc cat gac aac ctg ttt gtc gcc agc tcc ggc tgc        2278
Cys Arg Val His Gly His Asp Asn Leu Phe Val Ala Ser Ser Gly Cys
                705                 710                 715
atg ccg acc gtt ggg tcg gtc aat tgc acc ctg acc atc gcg gcc ttg        2326
Met Pro Thr Val Gly Ser Val Asn Cys Thr Leu Thr Ile Ala Ala Leu
            720                 725                 730
tct ctc cgt atc gcc gat cag cta gaa cag gaa ata tgagc atg ggc cag        2376
Ser Leu Arg Ile Ala Asp Gln Leu Glu Gln Glu Ile       Met Gly Gln
        735                 740                             745
cca ggt cgt aac gcc gtg aat gat gag agt att gcc aag aaa cgc ctg        2424
Pro Gly Arg Asn Ala Val Asn Asp Glu Ser Ile Ala Lys Lys Arg Leu
        750                 755                 760
atg gtc cgt cct gcg cat cgt cat ctg gga agc gcc att cgt cta gcg        2472
Met Val Arg Pro Ala His Arg His Leu Gly Ser Ala Ile Arg Leu Ala
    765                 770                 775
gca ttt gcc ggc gtc agc aca ctg ctg ttg agt ggt tgt ggc aac ggg        2520
Ala Phe Ala Gly Val Ser Thr Leu Leu Leu Ser Gly Cys Gly Asn Gly
780                 785                 790                 795
gaa gat gag cac gcc aag cag gtt gct gct gac aag gcg gcc acc agt        2568
Glu Asp Glu His Ala Lys Gln Val Ala Ala Asp Lys Ala Ala Thr Ser
                800                 805                 810
gac ccg gcc ttg gtc aag caa ggt ctt tat atg gcg cag gcc agt gac        2616
Asp Pro Ala Leu Val Lys Gln Gly Leu Tyr Met Ala Gln Ala Ser Asp
            815                 820                 825
tgt gct gcc tgc cac acg acc aag gat ggt gaa gac tac gca ggt ggc        2664
Cys Ala Ala Cys His Thr Thr Lys Asp Gly Glu Asp Tyr Ala Gly Gly
        830                 835                 840
ctc ccg ttc gag aca ccg gtc ggt gag att ttc tcc acc aac atc acg        2712
Leu Pro Phe Glu Thr Pro Val Gly Glu Ile Phe Ser Thr Asn Ile Thr
    845                 850                 855
ccg gac acc gag cat ggc atc ggc aac tat acc ctt gag gaa tac acc        2760
Pro Asp Thr Glu His Gly Ile Gly Asn Tyr Thr Leu Glu Glu Tyr Thr
860                 865                 870                 875
cgt gtg ctg cgc gaa ggg gaa gca gct gac ggc cat aac ctg tac ccg        2808
Arg Val Leu Arg Glu Gly Glu Ala Ala Asp Gly His Asn Leu Tyr Pro
                880                 885                 890
gcg atg ccg ttc ccg tcc tac gcg cgt ctg acc gat gat gac atc aag        2856
Ala Met Pro Phe Pro Ser Tyr Ala Arg Leu Thr Asp Asp Asp Ile Lys

            895                 900                 905
gcg ctg tat gcg tgg aac atg cat gat gtc acg ccg agc agc gag ccg 2904
Ala Leu Tyr Ala Trp Asn Met His Asp Val Thr Pro Ser Ser Glu Pro
        910                 915                 920
aat cgt gag agc gaa att ccg ttc ccg ctc aac atg cgt tgg ccg atg 2952
Asn Arg Glu Ser Glu Ile Pro Phe Pro Leu Asn Met Arg Trp Pro Met
    925                 930                 935
ggc ctg tgg gaa gaa atc ttc tca ccg ctt gag ccg tgg cag gat gac 3000
Gly Leu Trp Glu Glu Ile Phe Ser Pro Leu Glu Pro Trp Gln Asp Asp
940                 945                 950                 955
gag cag aaa gag gct gac tgg aat cgt ggc gcc tat ctg gtg cag ggc 3048
Glu Gln Lys Glu Ala Asp Trp Asn Arg Gly Ala Tyr Leu Val Gln Gly
                960                 965                 970
ccg ggt cac tgc ggt agc tgc cat acc gaa cgc ggc ctt gcc ttc cag 3096
Pro Gly His Cys Gly Ser Cys His Thr Glu Arg Gly Leu Ala Phe Gln
            975                 980                 985
gag aag gcg ctg acc ggc gat gaa gaa ggg tat ctc ggt ggt gcg atg 3144
Glu Lys Ala Leu Thr Gly Asp Glu Glu Gly Tyr Leu Gly Gly Ala Met
        990                 995                 1000
att gaa ggc tgg cgt gcc ttc aac ctg acg ccg gac gtc aag gat 3189
Ile Glu Gly Trp Arg Ala Phe Asn Leu Thr Pro Asp Val Lys Asp
    1005                1010                1015
ggc ttg ggt agc tgg agc gag cag gat atc gtc acc tat ctc tcg 3234
Gly Leu Gly Ser Trp Ser Glu Gln Asp Ile Val Thr Tyr Leu Ser
    1020                1025                1030
acg ggc aac ttg aaa ggc aag gca cag gcg ggt ggc ccg atg gcg 3279
Thr Gly Asn Leu Lys Gly Lys Ala Gln Ala Gly Gly Pro Met Ala
    1035                1040                1045
gat gtc atc agt cac tcc atg cgc cat atg act gac gaa gac ctc 3324
Asp Val Ile Ser His Ser Met Arg His Met Thr Asp Glu Asp Leu
    1050                1055                1060
aat gcg atg gcg gtc tat ctg aag tca ctg ccc gca ttg aat ggc 3369
Asn Ala Met Ala Val Tyr Leu Lys Ser Leu Pro Ala Leu Asn Gly
    1065                1070                1075
aag ggc gag cag ggc gag gcg gtc aat gat gat gac cgc aag gcg 3414
Lys Gly Glu Gln Gly Glu Ala Val Asn Asp Asp Asp Arg Lys Ala
    1080                1085                1090
gag gct acg gcg cca tct gag aag cag gcc gac gag aag ggc tcg 3459
Glu Ala Thr Ala Pro Ser Glu Lys Gln Ala Asp Glu Lys Gly Ser
    1095                1100                1105
caa gat gcc ccg cag gtc gcg act cgc ttc aat cag ggc gca ccg 3504
Gln Asp Ala Pro Gln Val Ala Thr Arg Phe Asn Gln Gly Ala Pro
    1110                1115                1120
gcc gat gac gtg ctg ttg ctt cgt ggg cag ccg ctg gac aag cgt 3549
Ala Asp Asp Val Leu Leu Leu Arg Gly Gln Pro Leu Asp Lys Arg
    1125                1130                1135
ccg gat gac aag gag ttg atc ggc cag ctg acg gat gca acg cct 3594
Pro Asp Asp Lys Glu Leu Ile Gly Gln Leu Thr Asp Ala Thr Pro
    1140                1145                1150
ggc gtg cgt ctg tat ctg ggc cac tgt gcc atg tgt cac ggt gct 3639
Gly Val Arg Leu Tyr Leu Gly His Cys Ala Met Cys His Gly Ala
    1155                1160                1165
gat ggt gcg ggt acg caa gac ggc tac tac ccg tca ctg ttc cac 3684
Asp Gly Ala Gly Thr Gln Asp Gly Tyr Tyr Pro Ser Leu Phe His
    1170                1175                1180
aac agt gtc acc ggc agc gtc tat ccg gat aat ctg gcg cag gcc 3729
Asn Ser Val Thr Gly Ser Val Tyr Pro Asp Asn Leu Ala Gln Ala
    1185                1190                1195
att ctc aat ggc gta cag cgt gag agc aat ggc cac agt gtc ttc 3774
Ile Leu Asn Gly Val Gln Arg Glu Ser Asn Gly His Ser Val Phe
    1200                1205                1210
atg ccg gcc ttt gac agc agc ctg aac gac aat gag ctg atc aat 3819
Met Pro Ala Phe Asp Ser Ser Leu Asn Asp Asn Glu Leu Ile Asn

1215                1220                1225
ctg atg  gac tat ctg gag aat  cgc ttc gga gaa ggc  cat gac agc        3864
Leu Met  Asp Tyr Leu Glu Asn  Arg Phe Gly Glu Gly  His Asp Ser
    1230                1235                1240
cag aca  ctg gcc gcg cca gat  gcg cgt cgt cag ctg  ctg agt gac        3909
Gln Thr  Leu Ala Ala Pro Asp  Ala Arg Arg Gln Leu  Leu Ser Asp
    1245                1250                1255
aag ctc  aag gat tgg cgt ggc  gag taagc                               3938
Lys Leu  Lys Asp Trp Arg Gly  Glu
    1260                1265

<210>  20
<211>  207
<212>  PRT
<213>  Cobetia sp.

<400>  20
Met Thr Pro Arg Glu Asn Ser Val Ser Asp Asp Val Pro Met Arg Gln
1               5                   10                  15
Asp Gly His His Glu Ala Ser Gln Val Ala Glu Pro Thr Ser Pro Ala
            20                  25                  30
Arg Arg Arg Leu Leu Lys Leu Gly Val Ser Gly Leu Gly Leu Val Ala
        35                  40                  45
Leu Ser Ser Leu Ser Leu Ser Ala Leu Ala Ala Ser Ala Thr Ser Ser
    50                  55                  60
Glu Ala Ser Ser Gly Ala Ala Ser Asp Asp Asp Ser Ala Phe Lys Ala
65                  70                  75                  80
Phe Met Leu Leu Ser Ala Trp Leu Leu Ser Asp Lys Thr Leu Asp Lys
                85                  90                  95
Arg Leu Gly Glu Arg Phe Phe Glu Ala Leu Val Arg Ile Pro Ala Pro
            100                 105                 110
Asn Val Pro Gly Met Gly Ser Leu Pro Ala Leu Lys Asn Lys Leu Leu
        115                 120                 125
Ala Leu Gly Glu Ala Arg Asp Tyr Leu Lys Glu Asp Asp Leu Asp Ala
    130                 135                 140
Ser Glu Met Gln Leu Val Arg Arg Val Leu Gln Ala Trp Met Leu Gly
145                 150                 155                 160
Thr Val Gly Thr Ala Ile Asp Asp Pro Ala Ala Glu Val Ile Ala Tyr
                165                 170                 175
Glu Tyr Ala Ala Met Tyr Ala Gly Pro Arg Asp Val Gln Ile Ile Arg
            180                 185                 190
Thr Tyr Cys Pro Asn Gln Pro Gly Phe Trp Ala Gln Arg Pro Glu
        195                 200                 205

<210>  21
<211>  537
<212>  PRT
<213>  Cobetia sp.

<400>  21
Met Ala Ala Glu Ser His Ser His Asp Ala Asp Val Ile Ile Val Gly
1               5                   10                  15
Ser Gly Val Ala Gly Ala Leu Ile Ala His Arg Leu Ala Glu Ala Gly
            20                  25                  30
Lys Ser Val Ile Met Leu Glu Ala Gly Pro Arg Met Gln Arg Trp Gln
        35                  40                  45
Ile Val Glu Arg Phe Arg Asn Gln Ala Asp Lys Met Asp Phe Met Gly
    50                  55                  60
Pro Tyr Pro Ala Ser Lys Ala Ala Pro His Pro Met Leu Tyr Gly Glu
65                  70                  75                  80
Asn Ala Asn Tyr Leu Val Gln Lys Gly Glu Gln Val Tyr Asp Ala Gln

                85                  90                  95
Tyr Ile Arg Ala Val Gly Gly Thr Thr Trp His Trp Ala Ala Ser Ala
            100                 105                 110
Trp Arg Phe Leu Pro Ser Asp Phe Arg Leu Lys Ser Glu Tyr Gly Val
        115                 120                 125
Gly Leu Asp Trp Pro Ile Ser Tyr Asp Asp Leu Glu Gly Tyr Tyr Phe
    130                 135                 140
Arg Ala Glu Gln Ala Leu Gly Val Trp Gly Pro Asn Asp Val Asp Leu
145                 150                 155                 160
Gly Ser Pro Arg Ser Ala Asp Tyr Pro Met Ala Pro Leu Pro Leu Ser
                165                 170                 175
Trp Asn Glu Gln Arg Val Ser Gln Arg Ile Asn Pro His Gly Phe Asp
            180                 185                 190
Met Val Thr Glu Pro Val Ala Arg Asn Ser Arg Pro Tyr Asp Asp Arg
        195                 200                 205
Pro Thr Cys Cys Gly Asn Asn Asn Cys Met Pro Ile Cys Pro Ile Gly
    210                 215                 220
Ala Met Tyr Ser Gly Ile Ile His Val Glu Lys Ala Glu Arg Ala Gly
225                 230                 235                 240
Ala Lys Leu Ile Asp Asn Ala Val Val His His Leu Glu Ser Asp Ala
                245                 250                 255
Ser Gly Lys Ile Arg Glu Val Arg Tyr Leu Asp Pro Gln Gly Lys Glu
            260                 265                 270
His Arg Leu Ser Ala Pro Lys Val Val Leu Ala Ala Asn Gly Ile Glu
        275                 280                 285
Thr Pro Lys Leu Met Gln Met Ser Val Asn Ala His Thr Pro Asn Gly
    290                 295                 300
Val Gly Asn Gln Asn Asp Met Val Gly Arg His Leu Met Asp His Pro
305                 310                 315                 320
Gly Thr Gly Val Ser Phe Leu Ala Asp Glu Val Leu Trp Ala Gly Arg
                325                 330                 335
Gly Pro Gln Glu Met Thr Ser Ile Ile Thr Trp Arg Asp Gly Asp Phe
            340                 345                 350
Arg Lys Glu Tyr Ala Ser Lys Lys Leu His Leu Ser Asn Met Ala Arg
        355                 360                 365
Thr Gln Gln Met Thr Thr Glu Val Leu Ser Glu Thr Pro Leu Arg Leu
    370                 375                 380
Gly Ala Asp Leu Gln Ala Gln Ile Asp Asn Arg Ala Ser Arg Tyr Val
385                 390                 395                 400
Gln Leu Asp Ser Phe His Glu Leu Leu Pro Glu Pro Ser Asn Arg Ile
                405                 410                 415
Thr Pro Ser Lys Lys Arg Asp Ala Leu Gly Leu Pro Arg Pro Glu Phe
            420                 425                 430
Arg Tyr Ala Ile Asp Asp Tyr Val Lys Arg Ser Ala Val His Thr Arg
        435                 440                 445
Glu Gln Tyr Ala Arg Ile Ala Gln Leu Met Gly Gly Thr Asp Ile Ala
    450                 455                 460
Phe Arg Asp Glu Phe Ser Asn Asn Gln His Ile Cys Gly Thr Thr Leu
465                 470                 475                 480
Met Gly Asp Asp Pro Lys Lys Ser Val Val Asp Arg Asp Cys Arg Val
                485                 490                 495
His Gly His Asp Asn Leu Phe Val Ala Ser Ser Gly Cys Met Pro Thr
            500                 505                 510
Val Gly Ser Val Asn Cys Thr Leu Thr Ile Ala Ala Leu Ser Leu Arg
        515                 520                 525
Ile Ala Asp Gln Leu Glu Gln Glu Ile
    530                 535

<210> 22
<211> 522
<212> PRT
<213> Cobetia sp.

<400>  22
Met Gly Gln Pro Gly Arg Asn Ala Val Asn Asp Glu Ser Ile Ala Lys
1               5                   10                  15
Lys Arg Leu Met Val Arg Pro Ala His Arg His Leu Gly Ser Ala Ile
            20                  25                  30
Arg Leu Ala Ala Phe Ala Gly Val Ser Thr Leu Leu Leu Ser Gly Cys
        35                  40                  45
Gly Asn Gly Glu Asp Glu His Ala Lys Gln Val Ala Ala Asp Lys Ala
    50                  55                  60
Ala Thr Ser Asp Pro Ala Leu Val Lys Gln Gly Leu Tyr Met Ala Gln
65                  70                  75                  80
Ala Ser Asp Cys Ala Ala Cys His Thr Thr Lys Asp Gly Glu Asp Tyr
                85                  90                  95
Ala Gly Gly Leu Pro Phe Glu Thr Pro Val Gly Glu Ile Phe Ser Thr
            100                 105                 110
Asn Ile Thr Pro Asp Thr Glu His Gly Ile Gly Asn Tyr Thr Leu Glu
        115                 120                 125
Glu Tyr Thr Arg Val Leu Arg Glu Gly Glu Ala Ala Asp Gly His Asn
    130                 135                 140
Leu Tyr Pro Ala Met Pro Phe Pro Ser Tyr Ala Arg Leu Thr Asp Asp
145                 150                 155                 160
Asp Ile Lys Ala Leu Tyr Ala Trp Asn Met His Asp Val Thr Pro Ser
                165                 170                 175
Ser Glu Pro Asn Arg Glu Ser Glu Ile Pro Phe Pro Leu Asn Met Arg
            180                 185                 190
Trp Pro Met Gly Leu Trp Glu Glu Ile Phe Ser Pro Leu Glu Pro Trp
        195                 200                 205
Gln Asp Asp Glu Gln Lys Glu Ala Asp Trp Asn Arg Gly Ala Tyr Leu
    210                 215                 220
Val Gln Gly Pro Gly His Cys Gly Ser Cys His Thr Glu Arg Gly Leu
225                 230                 235                 240
Ala Phe Gln Glu Lys Ala Leu Thr Gly Asp Glu Glu Gly Tyr Leu Gly
                245                 250                 255
Gly Ala Met Ile Glu Gly Trp Arg Ala Phe Asn Leu Thr Pro Asp Val
            260                 265                 270
Lys Asp Gly Leu Gly Ser Trp Ser Glu Gln Asp Ile Val Thr Tyr Leu
        275                 280                 285
Ser Thr Gly Asn Leu Lys Gly Lys Ala Gln Ala Gly Gly Pro Met Ala
    290                 295                 300
Asp Val Ile Ser His Ser Met Arg His Met Thr Asp Glu Asp Leu Asn
305                 310                 315                 320
Ala Met Ala Val Tyr Leu Lys Ser Leu Pro Ala Leu Asn Gly Lys Gly
                325                 330                 335
Glu Gln Gly Glu Ala Val Asn Asp Asp Asp Arg Lys Ala Glu Ala Thr
            340                 345                 350
Ala Pro Ser Glu Lys Gln Ala Asp Glu Lys Gly Ser Gln Asp Ala Pro
        355                 360                 365
Gln Val Ala Thr Arg Phe Asn Gln Gly Ala Pro Ala Asp Asp Val Leu
    370                 375                 380
Leu Leu Arg Gly Gln Pro Leu Asp Lys Arg Pro Asp Asp Lys Glu Leu
385                 390                 395                 400
Ile Gly Gln Leu Thr Asp Ala Thr Pro Gly Val Arg Leu Tyr Leu Gly
                405                 410                 415
His Cys Ala Met Cys His Gly Ala Asp Gly Ala Gly Thr Gln Asp Gly
            420                 425                 430
Tyr Tyr Pro Ser Leu Phe His Asn Ser Val Thr Gly Ser Val Tyr Pro
        435                 440                 445
Asp Asn Leu Ala Gln Ala Ile Leu Asn Gly Val Gln Arg Glu Ser Asn
    450                 455                 460
Gly His Ser Val Phe Met Pro Ala Phe Asp Ser Ser Leu Asn Asp Asn
465                 470                 475                 480
Glu Leu Ile Asn Leu Met Asp Tyr Leu Glu Asn Arg Phe Gly Glu Gly
                485                 490                 495

His Asp Ser Gln Thr Leu Ala Ala Pro Asp Ala Arg Arg Gln Leu Leu
            500                 505                 510
Ser Asp Lys Leu Lys Asp Trp Arg Gly Glu
        515                 520

<210>  23
<211>  8
<212>  PRT
<213>  Artificial Sequence

<220>
<223>  consensus sequence

<400>  23
Ala Arg Asn Ser Arg Pro Tyr Asp
1               5

<210>  24
<211>  8
<212>  PRT
<213>  Artificial Sequence

<220>
<223>  consensus sequence-cys introduced

<400>  24
Ala Arg Asn Ser Arg Cys Tyr Asp
1               5

<210>  25
<211>  4
<212>  PRT
<213>  Artificial Sequence

<220>
<223>  consensus sequence

<220>
<221>  MISC_FEATURE
<222>  (3)..(3)
<223>  Xaa is alanine or glycine

<400>  25
Met Pro Xaa Phe
1

<210>  26
<211>  7
<212>  PRT
<213>  Artificial Sequence

<220>
<223>  consensus sequence-cys introduced

<220>
<221>  MISC_FEATURE
<222>  (2)..(3)
<223>  Xaa is any L-amino acid

<220>
<221>  MISC_FEATURE
<222>  (6)..(6)
<223>  Xaa is alanine or glycine

<400>  26
Cys Xaa Xaa Met Pro Xaa Phe
1               5


<210>  27
<211>  4
<212>  PRT
<213>  Artificial Sequence

<220>
<223>  consensus sequence

<220>
<221>  MISC_FEATURE
<222>  (4)..(4)
<223>  Xaa is leucine or methionine

<400>  27
Tyr Pro Ser Xaa
1


<210>  28
<211>  5
<212>  PRT
<213>  Artificial Sequence

<220>
<223>  consensus sequence-cys intruduced

<220>
<221>  MISC_FEATURE
<222>  (5)..(5)
<223>  Xaa is leucine or methionine

<400>  28
Cys Tyr Pro Ser Xaa
1               5


<210>  29
<211>  8
<212>  PRT
<213>  Artificial Sequence

<220>
<223>  consensus sequence

<220>
<221>  MISC_FEATURE
<222>  (2)..(3)
<223>  Xaa is any L-amino acid

<220>
<221>  MISC_FEATURE
<222>  (5)..(7)
<223>  Xaa is any L-amino acid

<400>  29
Pro Xaa Xaa Trp Xaa Xaa Xaa Pro
1               5


<210>  30
<211>  9
<212>  PRT
<213>  Artificial Sequence

<220>
<223>  consensus sequence-cys introduced

<220>
<221>  MISC_FEATURE
<222>  (3)..(4)
<223>  Xaa is any L-amino acid

<220>
<221>  MISC_FEATURE
<222>  (6)..(8)
<223>  Xaa is any L-amino acid

<400>  30
Cys Pro Xaa Xaa Trp Xaa Xaa Xaa Pro
1               5


<210>  31
<211>  5
<212>  PRT
<213>  Artificial Sequence

<220>
<223>  heme-binding domain

<220>
<221>  MISC_FEATURE
<222>  (2)..(3)
<223>  Xaa is any L-amino acid

<400>  31
Cys Xaa Xaa Cys His
1               5